# FASCICULE DE BREVET EUROPEEN

(11) **EP 3 044 331 B1**
(45) Date de publication et mention de la délivrance du brevet: **11.03.2020**
(21) Numéro de dépôt: 14781927.0
(22) Date de dépôt: 11.09.2014
(51) Int. Cl.: C12Q 1/6886

(54) **MÉTHODE DE DIAGNOSTIC DES HÉMOPATHIES MALIGNES ET KIT ASSOCIÉ**
VERFAHREN ZUR DIAGNOSE HÄMATOLOGISCHER MALIGNOME UND ENTSPRECHENDES KIT
METHOD FOR DIAGNOSING HEMATOLOGICAL MALIGNANCIES AND ASSOCIATED KIT

(30) Priorité: 11.09.2013 FR 1358721
(43) Date de publication de la demande: 20.07.2016
(73) Titulaire: Universite de Rouen, 76130 Mont-Saint-Aignan (FR); Centre Henri Becquerel, 76000 Rouen (FR); Institut National de la Santé et de la Recherche Médicale, 75654 Paris Cedex 13 (FR)
(72) Inventeur: RUMINY, Philippe, F-76000 Rouen (FR); MARCHAND, Vinciane, F-76000 Rouen (FR); JARDIN, Fabrice, F-76000 Rouen (FR)
(74) Mandataire: Plasseraud IP
(86) Numéro de dépôt international: PCT/FR2014/052255
(87) Numéro de publication internationale: WO 2015/036705

(56) Documents cités:
- WO-A1-97/35589
- WO-A1-2006/017942
- WO-A2-03/044486
- WO-A2-2009/009431
- WO-A2-2009/062166
- ELDERING ERIC ET AL: "Expression profiling via novel multiplex assay allows rapid assessment of gene regulation in defined signalling pathways", NUCLEIC ACIDS RESEARCH, OXFORD UNIVERSITY PRESS, GB, vol. 31, no. 23, 1 décembre 2003 (2003-12-01), pages e153-1, XP002506295, ISSN: 1362-4962, DOI: 10.1093/NAR/GNG153
- JANNEKE ROTMAN ET AL: "Rapid screening of innate immune gene expression in zebrafish using reverse transcription - multiplex ligation-dependent probe amplification", BMC RESEARCH NOTES, BIOMED CENTRAL LTD, GB, vol. 4, no. 1, 15 juin 2011 (2011-06-15), page 196, XP021104291, ISSN: 1756-0500, DOI: 10.1186/1756-0500-4-196
- Mrc-Holland: "MLPA MLPA SALSA RT-MLPA probemix R009-A2 Inflammation", , 15 mai 2013 (2013-05-15), XP055091373, Extrait de l'Internet: URL:www.MLPA.com [extrait le 2013-12-03]
- "www.MLPA.com - product list - tumour characterisation", , 12 mars 2013 (2013-03-12), XP055091282, Extrait de l'Internet: URL:www.MLPA.com [extrait le 2013-12-03]
- THOMAS OHNESORG ET AL: "The Many Faces of MLPA", METHODS IN MOLECULAR BIOLOGY, HUMANA PRESS INC, NJ, US, vol. 687, 1 janvier 2011 (2011-01-01), pages 193-205, XP009174789, ISSN: 1064-3745
- MARTINEZ-GLEZ ET AL: "Multiplex ligation-dependent probe amplification (MLPA) screening in meningioma", CANCER GENETICS AND CYTOGENET, ELSEVIER SCIENCE PUBLISHING, NEW YORK, NY, US, vol. 173, no. 2, 22 February 2007 (2007-02-22), pages 170-172, XP005899960, ISSN: 0165-4608, DOI: 10.1016/J.CANCERGENCYTO.2006.09.011
- C.F. Taylor ET AL: "Genomic deletions inMSH2 orMLH1 are a frequent cause of hereditary non-polyposis colorectal cancer: Identification of novel and recurrent deletions by MLPA", HUMAN MUTATION, vol. 22, no. 6, 17 December 2003 (2003-12-17), pages 428-433, XP055385667, US ISSN: 1059-7794, DOI: 10.1002/humu.10291

### SEQUENCE LISTING

<110> Université de Rouen Centre Henri Becquerel
<120> Méthode de diagnostic des hémopathies malignes et kit associé
<130> BFF130197
<160> 799
<170> Patent In version 3.5
<210> 1
   <211> 22
   <212> DNA
   <213> Artificial Sequence
<220>
   <223> primer
<400> 1
   aagcccttca gcggccagta gc 22
<210> 2
   <211> 23
   <212> DNA
   <213> Artificial Sequence
<220>
   <223> primer
<400> 2
   gtgaaaagct ccgggtctta ggc 23
<210> 3
   <211> 22
   <212> DNA
   <213> Artificial Sequence
<220>
   <223> primer
<400> 3
   ggcgccttcc atggagacgc ag 22
<210> 4
   <211> 25
   <212> DNA
   <213> Artificial Sequence
<220>
   <223> primer
<400> 4
   attccgctga ccatcaataa ggaag 25
<210> 5
   <211> 26
   <212> DNA
   <213> Artificial Sequence
<220>
   <223> primer
<400> 5
   agccactgga tttaagcaga gttcaa 26
<210> 6
   <211> 23
   <212> DNA
   <213> Artificial Sequence
<220>
   <223> primer
<400> 6
   actgaaggca gccttcgacg tca 23
<210> 7
   <211> 24
   <212> DNA
   <213> Artificial Sequence
<220>
   <223> primer
<400> 7
   gctcttgcat cacccagggg aaag 24
<210> 8
   <211> 20
   <212> DNA
   <213> Artificial Sequence
<220>
   <223> primer
<400> 8
   cagtggcgcc ggggaggcag 20
<210> 9
   <211> 25
   <212> DNA
   <213> Artificial Sequence
<220>
   <223> primer
<400> 9
   ccattgagac ccagagcagc agttc 25
<210> 10
   <211> 24
   <212> DNA
   <213> Artificial Sequence
<220>
   <223> primer
<400> 10
   gagtttgatg aggagcgagc ccag 24
<210> 11
   <211> 25
   <212> DNA
   <213> Artificial Sequence
<220>
   <223> primer
<400> 11
   caggtctcat cgggaggaaa tggag 25
<210> 12
   <211> 26
   <212> DNA
   <213> Artificial Sequence
<220>
   <223> primer
<400> 12
   agtttcacag ctgctggcag taactg 26
<210> 13
   <211> 27
   <212> DNA
   <213> Artificial Sequence
<220>
   <223> primer
<400> 13
   gccaaggcga acctagacaa gaataag 27
<210> 14
   <211> 26
   <212> DNA
   <213> Artificial Sequence
<220>
   <223> primer
<400> 14
   aatgaagttg agagcgtcac agggat 26
<210> 15
   <211> 25
   <212> DNA
   <213> Artificial Sequence
<220>
   <223> primer
<400> 15
   gagctgcttc aagaagaaac ccggc 25
<210> 16
   <211> 25
   <212> DNA
   <213> Artificial Sequence
<220>
   <223> primer
<400> 16
   ctctccgact cgaagaagaa gctgc 25
<210> 17
   <211> 29
   <212> DNA
   <213> Artificial Sequence
<220>
   <223> primer
<400> 17
   ttgttagccg aggagaaaaa catctcttc 29
<210> 18
   <211> 25
   <212> DNA
   <213> Artificial Sequence
<220>
   <223> primer
<400> 18
   gtccatgagc tggagaagtc caagc 25
<210> 19
   <211> 28
   <212> DNA
   <213> Artificial Sequence
<220>
   <223> primer
<400> 19
   cttcacgagt atgagacgga actggaag 28
<210> 20
   <211> 25
   <212> DNA
   <213> Artificial Sequence
<220>
   <223> primer
<400> 20
   tctcggcctc ccgactccta cagtg 25
<210> 21
   <211> 26
   <212> DNA
   <213> Artificial Sequence
<220>
   <223> primer
<400> 21
   ttttgagtat ccgaggagcc caggag 26
<210> 22
   <211> 29
   <212> DNA
   <213> Artificial Sequence
<220>
   <223> primer
<400> 22
   ggtcatactg catcagaacc atgaagaag 29
<210> 23
   <211> 24
   <212> DNA
   <213> Artificial Sequence
<220>
   <223> primer
<400> 23
   ccatgcccat tgggagaata gcag 24
<210> 24
   <211> 30
   <212> DNA
   <213> Artificial Sequence
<220>
   <223> primer
<400> 24
   aatgcatact tggaatgaat ccttctagag 30
<210> 25
   <211> 23
   <212> DNA
   <213> Artificial Sequence
<220>
   <223> primer
<400> 25
   atgccagcac gagccgccgc ttc 23
<210> 26
   <211> 23
   <212> DNA
   <213> Artificial Sequence
<220>
   <223> primer
<400> 26
   acctcagctc cgcggaagtt gcg 23
<210> 27
   <211> 22
   <212> DNA
   <213> Artificial Sequence
<220>
   <223> primer
<400> 27
   atcgcccagg accacaccgc ag 22
<210> 28
   <211> 22
   <212> DNA
   <213> Artificial Sequence
<220>
   <223> primer
<400> 28
   gatgaccgag cggccgccga gc 22
<210> 29
   <211> 22
   <212> DNA
   <213> Artificial Sequence
<220>
   <223> primer
<400> 29
   gtccccacac caaagttgtg cg 22
<210> 30
   <211> 24
   <212> DNA
   <213> Artificial Sequence
<220>
   <223> primer
<400> 30
   tggatgggcc ccgagaacct cgaa 24
<210> 31
   <211> 27
   <212> DNA
   <213> Artificial Sequence
<220>
   <223> primer
<400> 31
   atcgtactga gaagcactcc acaatgc 27
<210> 32
   <211> 19
   <212> DNA
   <213> Artificial Sequence
<220>
   <223> primer
<400> 32
   gggttcccta agggttgga 19
<210> 33
   <211> 23
   <212> DNA
   <213> Artificial Sequence
<220>
   <223> primer
<400> 33
   gtgccagcaa gatccaatct aga 23
<210> 34
   <211> 19
   <212> DNA
   <213> Artificial Sequence
<220>
   <223> primer
<400> 34
   tccaaccctt agggaaccc 19
<210> 35
   <211> 41
   <212> DNA
   <213> Artificial Sequence
<220>
   <223> primer
<400> 35
   aagcccttca gcggccagta gctccaaccc ttagggaacc c 41
<210> 36
   <211> 42
   <212> DNA
   <213> Artificial Sequence
<220>
   <223> primer
<400> 36
   gtgaaaagct ccgggtctta ggctccaacc cttagggaac cc 42
<210> 37
   <211> 45
   <212> DNA
   <213> Artificial Sequence
<220>
   <223> primer
<400> 37
   gtgccagcaa gatccaatct agaggcgcct tccatggaga cgcag 45
<210> 38
   <211> 48
   <212> DNA
   <213> Artificial Sequence
<220>
   <223> primer
<400> 38
   gtgccagcaa gatccaatct agaattccgc tgaccatcaa taaggaag 48
<210> 39
   <211> 49
   <212> DNA
   <213> Artificial Sequence
<220>
   <223> primer
<400> 39
   gtgccagcaa gatccaatct agaagccact ggatttaagc agagttcaa 49
<210> 40
   <211> 46
   <212> DNA
   <213> Artificial Sequence
<220>
   <223> primer
<400> 40
   gtgccagcaa gatccaatct agaactgaag gcagccttcg acgtca 46
<210> 41
   <211> 47
   <212> DNA
   <213> Artificial Sequence
<220>
   <223> primer
<400> 41
   gtgccagcaa gatccaatct agagctcttg catcacccag gggaaag 47
<210> 42
   <211> 43
   <212> DNA
   <213> Artificial Sequence
<220>
   <223> primer
<400> 42
   gtgccagcaa gatccaatct agacagtggc gccggggagg cag 43
<210> 43
   <211> 44
   <212> DNA
   <213> Artificial Sequence
<220>
   <223> primer
<400> 43
   ccattgagac ccagagcagc agttctccaa cccttaggga accc 44
<210> 44
   <211> 47
   <212> DNA
   <213> Artificial Sequence
<220>
   <223> primer
<400> 44
   gtgccagcaa gatccaatct agagagtttg atgaggagcg agcccag 47
<210> 45
   <211> 48
   <212> DNA
   <213> Artificial Sequence
<220>
   <223> primer
<400> 45
   gtgccagcaa gatccaatct agacaggtct catcgggagg aaatggag 48
<210> 46
   <211> 49
   <212> DNA
   <213> Artificial Sequence
<220>
   <223> primer
<400> 46
   gtgccagcaa gatccaatct agaagtttca cagctgctgg cagtaactg 49
<210> 47
   <211> 46
   <212> DNA
   <213> Artificial Sequence
<220>
   <223> primer
<400> 47
   gccaaggcga acctagacaa gaataagtcc aacccttagg gaaccc 46
<210> 48
   <211> 45
   <212> DNA
   <213> Artificial Sequence
<220>
   <223> primer
<400> 48
   aatgaagttg agagcgtcac agggattcca acccttaggg aaccc 45
<210> 49
   <211> 44
   <212> DNA
   <213> Artificial Sequence
<220>
   <223> primer
<400> 49
   gagctgcttc aagaagaaac ccggctccaa cccttaggga accc 44
<210> 50
   <211> 44
   <212> DNA
   <213> Artificial Sequence
<220>
   <223> primer
<400> 50
   ctctccgact cgaagaagaa gctgctccaa cccttaggga accc 44
<210> 51
   <211> 48
   <212> DNA
   <213> Artificial Sequence
<220>
   <223> primer
<400> 51
   ttgttagccg aggagaaaaa catctcttct ccaaccctta gggaaccc 48
<210> 52
   <211> 44
   <212> DNA
   <213> Artificial Sequence
<220>
   <223> primer
<400> 52
   gtccatgagc tggagaagtc caagctccaa cccttaggga accc 44
<210> 53
   <211> 47
   <212> DNA
   <213> Artificial Sequence
<220>
   <223> primer
<400> 53
   cttcacgagt atgagacgga actggaagtc caacccttag ggaaccc 47
<210> 54
   <211> 48
   <212> DNA
   <213> Artificial Sequence
<220>
   <223> primer
<400> 54
   gtgccagcaa gatccaatct agatctcggc ctcccgactc ctacagtg 48
<210> 55
   <211> 45
   <212> DNA
   <213> Artificial Sequence
<220>
   <223> primer
<400> 55
   ttttgagtat ccgaggagcc caggagtcca acccttaggg aaccc 45
<210> 56
   <211> 52
   <212> DNA
   <213> Artificial Sequence
<220>
   <223> primer
<400> 56
   gtgccagcaa gatccaatct agaggtcata ctgcatcaga accatgaaga ag 52
<210> 57
   <211> 47
   <212> DNA
   <213> Artificial Sequence
<220>
   <223> primer
<400> 57
   gtgccagcaa gatccaatct agaccatgcc cattgggaga atagcag 47
<210> 58
   <211> 49
   <212> DNA
   <213> Artificial Sequence
<220>
   <223> primer
<400> 58
   aatgcatact tggaatgaat ccttctagag tccaaccctt agggaaccc 49
<210> 59
   <211> 42
   <212> DNA
   <213> Artificial Sequence
<220>
   <223> primer
<400> 59
   atgccagcac gagccgccgc ttctccaacc cttagggaac cc 42
<210> 60
   <211> 46
   <212> DNA
   <213> Artificial Sequence
<220>
   <223> primer
<400> 60
   gtgccagcaa gatccaatct agaacctcag ctccgcggaa gttgcg 46
<210> 61
   <211> 41
   <212> DNA
   <213> Artificial Sequence
<220>
   <223> primer
<400> 61
   atcgcccagg accacaccgc agtccaaccc ttagggaacc c 41
<210> 62
   <211> 41
   <212> DNA
   <213> Artificial Sequence
<220>
   <223> primer
<400> 62
   gatgaccgag cggccgccga gctccaaccc ttagggaacc c 41
<210> 63
   <211> 41
   <212> DNA
   <213> Artificial Sequence
<220>
   <223> primer
<400> 63
   gtccccacac caaagttgtg cgtccaaccc ttagggaacc c 41
<210> 64
   <211> 47
   <212> DNA
   <213> Artificial Sequence
<220>
   <223> primer
<400> 64
   gtgccagcaa gatccaatct agatggatgg gccccgagaa cctcgaa 47
<210> 65
   <211> 46
   <212> DNA
   <213> Artificial Sequence
<220>
   <223> primer
<400> 65
   atcgtactga gaagcactcc acaatgctcc aacccttagg gaaccc 46
<210> 66
   <211> 23
   <212> DNA
   <213> Artificial Sequence
<220>
   <223> primer
<400> 66
   gcagatggcc agtcaggcac cag 23
<210> 67
   <211> 29
   <212> DNA
   <213> Artificial Sequence
<220>
   <223> primer
<400> 67
   tcagttatca tctggtgaca aagcttcag 29
<210> 68
   <211> 27
   <212> DNA
   <213> Artificial Sequence
<220>
   <223> primer
<400> 68
   cgtcttctaa tttcactgct gcacaag 27
<210> 69
   <211> 24
   <212> DNA
   <213> Artificial Sequence
<220>
   <223> primer
<400> 69
   ggttcagctt ttgccaagct tcag 24
<210> 70
   <211> 25
   <212> DNA
   <213> Artificial Sequence
<220>
   <223> primer
<400> 70
   ggctttggat ccacagctac ctcaa 25
<210> 71
   <211> 26
   <212> DNA
   <213> Artificial Sequence
<220>
   <223> primer
<400> 71
   tgggttttcc tctccaaaca aaacag 26
<210> 72
   <211> 23
   <212> DNA
   <213> Artificial Sequence
<220>
   <223> primer
<400> 72
   tggttttgga tcaggcacag gag 23
<210> 73
   <211> 30
   <212> DNA
   <213> Artificial Sequence
<220>
   <223> primer
<400> 73
   attatgaact attaacagaa aatgacatgt 30
<210> 74
   <211> 28
   <212> DNA
   <213> Artificial Sequence
<220>
   <223> primer
<400> 74
   ttcttcagga gagaatacca tgggtacc 28
<210> 75
   <211> 30
   <212> DNA
   <213> Artificial Sequence
<220>
   <223> primer
<400> 75
   gaaattgaac ttagctcatt aagggaagct 30
<210> 76
   <211> 30
   <212> DNA
   <213> Artificial Sequence
<220>
   <223> primer
<400> 76
   cgagaaaatg tcattgaata taaacactgt 30
<210> 77
   <211> 30
   <212> DNA
   <213> Artificial Sequence
<220>
   <223> primer
<400> 77
   cctagtgaga gccttgctac tactgatgat 30
<210> 78
   <211> 30
   <212> DNA
   <213> Artificial Sequence
<220>
   <223> primer
<400> 78
   actgaatctc cagtgttagt gaatgactat 30
<210> 79
   <211> 30
   <212> DNA
   <213> Artificial Sequence
<220>
   <223> primer
<400> 79
   cagtgcatat tagtggacag cacttagtag 30
<210> 80
   <211> 28
   <212> DNA
   <213> Artificial Sequence
<220>
   <223> primer
<400> 80
   ctgagaatgc acttactggc tcattcag 28
<210> 81
   <211> 26
   <212> DNA
   <213> Artificial Sequence
<220>
   <223> primer
<400> 81
   ggagacacac aggcagaccc atactg 26
<210> 82
   <211> 28
   <212> DNA
   <213> Artificial Sequence
<220>
   <223> primer
<400> 82
   atcaccattg cttggaagtt tgattctc 28
<210> 83
   <211> 26
   <212> DNA
   <213> Artificial Sequence
<220>
   <223> primer
<400> 83
   accagttccc tgcgagtctg ctactg 26
<210> 84
   <211> 27
   <212> DNA
   <213> Artificial Sequence
<220>
   <223> primer
<400> 84
   attacctggt catgatcatt gtccgtg 27
<210> 85
   <211> 26
   <212> DNA
   <213> Artificial Sequence
<220>
   <223> primer
<400> 85
   tgctacagtt gaaactccag cagcgc 26
<210> 86
   <211> 26
   <212> DNA
   <213> Artificial Sequence
<220>
   <223> primer
<400> 86
   ctttgaaaag tccagccgca tttcat 26
<210> 87
   <211> 30
   <212> DNA
   <213> Artificial Sequence
<220>
   <223> primer
<400> 87
   gaagtcacaa tgaaacagat ttgcaaaaag 30
<210> 88
   <211> 25
   <212> DNA
   <213> Artificial Sequence
<220>
   <223> primer
<400> 88
   gaaattcggc gccttcatca gtatg 25
<210> 89
   <211> 30
   <212> DNA
   <213> Artificial Sequence
<220>
   <223> primer
<400> 89
   aagaagatga agagtcagat gatgctgatg 30
<210> 90
   <211> 30
   <212> DNA
   <213> Artificial Sequence
<220>
   <223> primer
<400> 90
   attttggatc attgtttgac ttggaaaatg 30
<210> 91
   <211> 26
   <212> DNA
   <213> Artificial Sequence
<220>
   <223> primer
<400> 91
   atgggaataa ctgggaacac aagtcc 26
<210> 92
   <211> 23
   <212> DNA
   <213> Artificial Sequence
<220>
   <223> primer
<400> 92
   gcttggtgca ggatttggaa cag 23
<210> 93
   <211> 22
   <212> DNA
   <213> Artificial Sequence
<220>
   <223> primer
<400> 93
   ggagcccccc aggccccagt ag 22
<210> 94
   <211> 28
   <212> DNA
   <213> Artificial Sequence
<220>
   <223> primer
<400> 94
   gatgtcagac cctaagaaga aggaagag 28
<210> 95
   <211> 24
   <212> DNA
   <213> Artificial Sequence
<220>
   <223> primer
<400> 95
   agccaatgga gcattcatgc ccaa 24
<210> 96
   <211> 23
   <212> DNA
   <213> Artificial Sequence
<220>
   <223> primer
<400> 96
   ctgtgcggtc agagaagaaa cgc 23
<210> 97
   <211> 27
   <212> DNA
   <213> Artificial Sequence
<220>
   <223> primer
<400> 97
   ttgatagaga aaaacaaccc agcgaag 27
<210> 98
   <211> 24
   <212> DNA
   <213> Artificial Sequence
<220>
   <223> primer
<400> 98
   ataacccagc agccaactgg cttc 24
<210> 99
   <211> 27
   <212> DNA
   <213> Artificial Sequence
<220>
   <223> primer
<400> 99
   gatggtaaat tgaaaaaacc caagaat 27
<210> 100
   <211> 27
   <212> DNA
   <213> Artificial Sequence
<220>
   <223> primer
<400> 100
   ttcttgatga agcagataga atcttgg 27
<210> 101
   <211> 23
   <212> DNA
   <213> Artificial Sequence
<220>
   <223> primer
<400> 101
   cacccctgcc actttggaac aga 23
<210> 102
   <211> 29
   <212> DNA
   <213> Artificial Sequence
<220>
   <223> primer
<400> 102
   ataatctcag tgataccttg aagaagctg 29
<210> 103
   <211> 28
   <212> DNA
   <213> Artificial Sequence
<220>
   <223> primer
<400> 103
   atacggaaac aagtgaaaaa atccaagc 28
<210> 104
   <211> 29
   <212> DNA
   <213> Artificial Sequence
<220>
   <223> primer
<400> 104
   accaagaggc tattcaagat ctctgtctg 29
<210> 105
   <211> 29
   <212> DNA
   <213> Artificial Sequence
<220>
   <223> primer
<400> 105
   accaagaggc tattcaagat ctctgcatg 29
<210> 106
   <211> 30
   <212> DNA
   <213> Artificial Sequence
<220>
   <223> primer
<400> 106
   gcagtggagg aagtctcttt aagaaaatag 30
<210> 107
   <211> 23
   <212> DNA
   <213> Artificial Sequence
<220>
   <223> primer
<400> 107
   cttctgccgc tgcttctgca cag 23
<210> 108
   <211> 23
   <212> DNA
   <213> Artificial Sequence
<220>
   <223> primer
<400> 108
   gcatggggcg gctggttctg ctg 23
<210> 109
   <211> 24
   <212> DNA
   <213> Artificial Sequence
<220>
   <223> primer
<400> 109
   ctgaggacat ctggaggaag gctg 24
<210> 110
   <211> 21
   <212> DNA
   <213> Artificial Sequence
<220>
   <223> primer
<400> 110
   cagcaggagg actccagcga g 21
<210> 111
   <211> 24
   <212> DNA
   <213> Artificial Sequence
<220>
   <223> primer
<400> 111
   gcttcctgct gaactccaag ttcc 24
<210> 112
   <211> 23
   <212> DNA
   <213> Artificial Sequence
<220>
   <223> primer
<400> 112
   gacttccagc cactgcgcta ttt 23
<210> 113
   <211> 30
   <212> DNA
   <213> Artificial Sequence
<220>
   <223> primer
<400> 113
   atccctgtaa aacaaaaacc aaaagaaaag 30
<210> 114
   <211> 28
   <212> DNA
   <213> Artificial Sequence
<220>
   <223> primer
<400> 114
   agtccacagg atcagagtgg actttaag 28
<210> 115
   <211> 27
   <212> DNA
   <213> Artificial Sequence
<220>
   <223> primer
<400> 115
   ctctgtgcca gtagtgggca tgtagag 27
<210> 116
   <211> 26
   <212> DNA
   <213> Artificial Sequence
<220>
   <223> primer
<400> 116
   gtggaaggca acatcaggct acaaag 26
<210> 117
   <211> 26
   <212> DNA
   <213> Artificial Sequence
<220>
   <223> primer
<400> 117
   cagacctact ccaatgaagt ccattg 26
<210> 118
   <211> 23
   <212> DNA
   <213> Artificial Sequence
<220>
   <223> primer
<400> 118
   gaaatgaccc attcatggcc gcc 23
<210> 119
   <211> 26
   <212> DNA
   <213> Artificial Sequence
<220>
   <223> primer
<400> 119
   gactctcagc atgtcagttc tgtaac 26
<210> 120
   <211> 26
   <212> DNA
   <213> Artificial Sequence
<220>
   <223> primer
<400> 120
   cctgagcctc caacaacaaa caaatg 26
<210> 121
   <211> 27
   <212> DNA
   <213> Artificial Sequence
<220>
   <223> primer
<400> 121
   gatgagcaat tcttaggttt tggctca 27
<210> 122
   <211> 25
   <212> DNA
   <213> Artificial Sequence
<220>
   <223> primer
<400> 122
   gaagaaccta ggaaagtccg ctttg 25
<210> 123
   <211> 27
   <212> DNA
   <213> Artificial Sequence
<220>
   <223> primer
<400> 123
   gaccctaata ggagtattca taccagc 27
<210> 124
   <211> 23
   <212> DNA
   <213> Artificial Sequence
<220>
   <223> primer
<400> 124
   tctgaacaac ccagtcctgc cag 23
<210> 125
   <211> 31
   <212> DNA
   <213> Artificial Sequence
<220>
   <223> primer
<400> 125
   attcttgaag tgaaaagtcc aataaagcaa a 31
<210> 126
   <211> 25
   <212> DNA
   <213> Artificial Sequence
<220>
   <223> primer
<400> 126
   gcatacctag atgaactggt agagc 25
<210> 127
   <211> 23
   <212> DNA
   <213> Artificial Sequence
<220>
   <223> primer
<400> 127
   tgcaccgtcc aggtgaggtt aga 23
<210> 128
   <211> 23
   <212> DNA
   <213> Artificial Sequence
<220>
   <223> primer
<400> 128
   gaggagccga ggaaggtctg ctt 23
<210> 129
   <211> 23
   <212> DNA
   <213> Artificial Sequence
<220>
   <223> primer
<400> 129
   gtgatggtaa tgcccgaagg agc 23
<210> 130
   <211> 23
   <212> DNA
   <213> Artificial Sequence
<220>
   <223> primer
<400> 130
   gacgccaaca aggagagcag caa 23
<210> 131
   <211> 23
   <212> DNA
   <213> Artificial Sequence
<220>
   <223> primer
<400> 131
   tctgcccagt caagcccgtc caa 23
<210> 132
   <211> 27
   <212> DNA
   <213> Artificial Sequence
<220>
   <223> primer
<400> 132
   agatgtgaac tttgtcccca taaggat 27
<210> 133
   <211> 29
   <212> DNA
   <213> Artificial Sequence
<220>
   <223> primer
<400> 133
   acttgctaca tttgtgatga acaaggaag 29
<210> 134
   <211> 31
   <212> DNA
   <213> Artificial Sequence
<220>
   <223> primer
<400> 134
   aaatataaag agaaggacaa acacaaacag a 31
<210> 135
   <211> 30
   <212> DNA
   <213> Artificial Sequence
<220>
   <223> primer
<400> 135
   acttatacaa gcactagcaa caactctata 30
<210> 136
   <211> 27
   <212> DNA
   <213> Artificial Sequence
<220>
   <223> primer
<400> 136
   gcaaatactc tatctggatc ttctctc 27
<210> 137
   <211> 26
   <212> DNA
   <213> Artificial Sequence
<220>
   <223> primer
<400> 137
   gatttggagt tccatggagt gatgag 26
<210> 138
   <211> 27
   <212> DNA
   <213> Artificial Sequence
<220>
   <223> primer
<400> 138
   gattctgttt cactgaggcc atctatc 27
<210> 139
   <211> 23
   <212> DNA
   <213> Artificial Sequence
<220>
   <223> primer
<400> 139
   cacatctcca tcccccagcc tga 23
<210> 140
   <211> 24
   <212> DNA
   <213> Artificial Sequence
<220>
   <223> primer
<400> 140
   gtggccaaca tgagtgctaa ggac 24
<210> 141
   <211> 31
   <212> DNA
   <213> Artificial Sequence
<220>
   <223> primer
<400> 141
   ttatcaagtg ggaatcctgt atatgaaaaa t 31
<210> 142
   <211> 27
   <212> DNA
   <213> Artificial Sequence
<220>
   <223> primer
<400> 142
   catgatacca gtagtccttt gctaatc 27
<210> 143
   <211> 27
   <212> DNA
   <213> Artificial Sequence
<220>
   <223> primer
<400> 143
   agtcgaaagg acaaagaacg ccttaag 27
<210> 144
   <211> 23
   <212> DNA
   <213> Artificial Sequence
<220>
   <223> primer
<400> 144
   gtctcctcct cggcttcctc ttc 23
<210> 145
   <211> 24
   <212> DNA
   <213> Artificial Sequence
<220>
   <223> primer
<400> 145
   gtgttttctc tggctggctc tacc 24
<210> 146
   <211> 23
   <212> DNA
   <213> Artificial Sequence
<220>
   <223> primer
<400> 146
   ggtgaaggtt gccgaactgt ccc 23
<210> 147
   <211> 23
   <212> DNA
   <213> Artificial Sequence
<220>
   <223> primer
<400> 147
   gaagctatga gggaccctgt gag 23
<210> 148
   <211> 24
   <212> DNA
   <213> Artificial Sequence
<220>
   <223> primer
<400> 148
   gcacggacac ttgctagtat gttg 24
<210> 149
   <211> 24
   <212> DNA
   <213> Artificial Sequence
<220>
   <223> primer
<400> 149
   gcatcagctt ctggtgatgt gagc 24
<210> 150
   <211> 23
   <212> DNA
   <213> Artificial Sequence
<220>
   <223> primer
<400> 150
   gagatgacgc attcatggcc tcc 23
<210> 151
   <211> 24
   <212> DNA
   <213> Artificial Sequence
<220>
   <223> primer
<400> 151
   gagtctcagc agtccaattt tggc 24
<210> 152
   <211> 26
   <212> DNA
   <213> Artificial Sequence
<220>
   <223> primer
<400> 152
   atatttaaca ccgtgcccga tatgcc 26
<210> 153
   <211> 27
   <212> DNA
   <213> Artificial Sequence
<220>
   <223> primer
<400> 153
   aagatggcag tgaacgtata ctcaacg 27
<210> 154
   <211> 30
   <212> DNA
   <213> Artificial Sequence
<220>
   <223> primer
<400> 154
   ataattcctg tggacaaatt agtaaaagga 30
<210> 155
   <211> 30
   <212> DNA
   <213> Artificial Sequence
<220>
   <223> primer
<400> 155
   gtcaacgtat tgaaacttac tgttgaagac 30
<210> 156
   <211> 24
   <212> DNA
   <213> Artificial Sequence
<220>
   <223> primer
<400> 156
   gacattgaca agcagtacgt gggc 24
<210> 157
   <211> 23
   <212> DNA
   <213> Artificial Sequence
<220>
   <223> primer
<400> 157
   gaggcacgcg gacctccagt ggc 23
<210> 158
   <211> 27
   <212> DNA
   <213> Artificial Sequence
<220>
   <223> primer
<400> 158
   ccttgaaaag atcttttgag gtcgagg 27
<210> 159
   <211> 31
   <212> DNA
   <213> Artificial Sequence
<220>
   <223> primer
<400> 159
   gaaaaaaacc ttgaagataa cttacagagt t 31
<210> 160
   <211> 26
   <212> DNA
   <213> Artificial Sequence
<220>
   <223> primer
<400> 160
   gagagtagat ctggagaaac caacag 26
<210> 161
   <211> 23
   <212> DNA
   <213> Artificial Sequence
<220>
   <223> primer
<400> 161
   gagatgacct ggcttccacc act 23
<210> 162
   <211> 23
   <212> DNA
   <213> Artificial Sequence
<220>
   <223> primer
<400> 162
   caggcagctc agagaacggc tct 23
<210> 163
   <211> 31
   <212> DNA
   <213> Artificial Sequence
<220>
   <223> primer
<400> 163
   atttttgatc accatactga agaggatata g 31
<210> 164
   <211> 23
   <212> DNA
   <213> Artificial Sequence
<220>
   <223> primer
<400> 164
   aactccatcc ggcacaacct gtc 23
<210> 165
   <211> 27
   <212> DNA
   <213> Artificial Sequence
<220>
   <223> primer
<400> 165
   ctccagggtt ccttgaaaag aaaacag 27
<210> 166
   <211> 24
   <212> DNA
   <213> Artificial Sequence
<220>
   <223> primer
<400> 166
   gatcaacact ctgtggtagg ccag 24
<210> 167
   <211> 28
   <212> DNA
   <213> Artificial Sequence
<220>
   <223> primer
<400> 167
   caagttggaa ttgacagagg tgatatac 28
<210> 168
   <211> 23
   <212> DNA
   <213> Artificial Sequence
<220>
   <223> primer
<400> 168
   gcccctagca gtcttcttga tgc 23
<210> 169
   <211> 26
   <212> DNA
   <213> Artificial Sequence
<220>
   <223> primer
<400> 169
   caacctcata atattctgca gaggcg 26
<210> 170
   <211> 23
   <212> DNA
   <213> Artificial Sequence
<220>
   <223> primer
<400> 170
   gacatgcgga agcacgtggc cat 23
<210> 171
   <211> 23
   <212> DNA
   <213> Artificial Sequence
<220>
   <223> primer
<400> 171
   ggctacatgc agccgctgaa gca 23
<210> 172
   <211> 27
   <212> DNA
   <213> Artificial Sequence
<220>
   <223> primer
<400> 172
   ttctccaagg atgtcctagt aaacatc 27
<210> 173
   <211> 24
   <212> DNA
   <213> Artificial Sequence
<220>
   <223> primer
<400> 173
   caaagcatgc gtgagaacaa ggag 24
<210> 174
   <211> 25
   <212> DNA
   <213> Artificial Sequence
<220>
   <223> primer
<400> 174
   ccagaagtca ttggatctgt gtcac 25
<210> 175
   <211> 30
   <212> DNA
   <213> Artificial Sequence
<220>
   <223> primer
<400> 175
   gtaaccatgg agcttattac agataacaaa 30
<210> 176
   <211> 24
   <212> DNA
   <213> Artificial Sequence
<220>
   <223> primer
<400> 176
   gtgattcctg tctctctgtc ttcc 24
<210> 177
   <211> 23
   <212> DNA
   <213> Artificial Sequence
<220>
   <223> primer
<400> 177
   gcagaggacc gaggaaatgg act 23
<210> 178
   <211> 24
   <212> DNA
   <213> Artificial Sequence
<220>
   <223> primer
<400> 178
   gatggagctg tagttacacc ctcc 24
<210> 179
   <211> 24
   <212> DNA
   <213> Artificial Sequence
<220>
   <223> primer
<400> 179
   agtcccaaga gtggcccaaa agag 24
<210> 180
   <211> 25
   <212> DNA
   <213> Artificial Sequence
<220>
   <223> primer
<400> 180
   gacactcaat cacttgtcgg aagtc 25
<210> 181
   <211> 23
   <212> DNA
   <213> Artificial Sequence
<220>
   <223> primer
<400> 181
   gtggaacggc cgccttctcc att 23
<210> 182
   <211> 23
   <212> DNA
   <213> Artificial Sequence
<220>
   <223> primer
<400> 182
   gtgaatgagg cctctgggga tgg 23
<210> 183
   <211> 23
   <212> DNA
   <213> Artificial Sequence
<220>
   <223> primer
<400> 183
   aagcctggaa tggtcccccc tcc 23
<210> 184
   <211> 23
   <212> DNA
   <213> Artificial Sequence
<220>
   <223> primer
<400> 184
   aactcgatcc gccacaacct gtc 23
<210> 185
   <211> 28
   <212> DNA
   <213> Artificial Sequence
<220>
   <223> primer
<400> 185
   caacagccaa ctcagtttat aaatccag 28
<210> 186
   <211> 25
   <212> DNA
   <213> Artificial Sequence
<220>
   <223> primer
<400> 186
   ctgcagaaga aagatcagca actgg 25
<210> 187
   <211> 26
   <212> DNA
   <213> Artificial Sequence
<220>
   <223> primer
<400> 187
   atgggaataa ctgggaacac aagtcc 26
<210> 188
   <211> 27
   <212> DNA
   <213> Artificial Sequence
<220>
   <223> primer
<400> 188
   tctcagatgc aaacatcagt gggaatt 27
<210> 189
   <211> 25
   <212> DNA
   <213> Artificial Sequence
<220>
   <223> primer
<400> 189
   atgatggagg aggatttgca aggag 25
<210> 190
   <211> 25
   <212> DNA
   <213> Artificial Sequence
<220>
   <223> primer
<400> 190
   gtgagtaccc agaacatgaa gatgg 25
<210> 191
   <211> 23
   <212> DNA
   <213> Artificial Sequence
<220>
   <223> primer
<400> 191
   ggcctggagc aggatgtcct cca 23
<210> 192
   <211> 27
   <212> DNA
   <213> Artificial Sequence
<220>
   <223> primer
<400> 192
   aatcagatgg gtgactcaaa tatctcc 27
<210> 193
   <211> 24
   <212> DNA
   <213> Artificial Sequence
<220>
   <223> primer
<400> 193
   tctgctccat ctggacacaa gcat 24
<210> 194
   <211> 23
   <212> DNA
   <213> Artificial Sequence
<220>
   <223> primer
<400> 194
   ctggagaatg ctggaggaga cct 23
<210> 195
   <211> 26
   <212> DNA
   <213> Artificial Sequence
<220>
   <223> primer
<400> 195
   aatactctgg agcagtgcaa tgtgtg 26
<210> 196
   <211> 26
   <212> DNA
   <213> Artificial Sequence
<220>
   <223> primer
<400> 196
   aatactctgg agcagtgcaa tgtgtg 26
<210> 197
   <211> 27
   <212> DNA
   <213> Artificial Sequence
<220>
   <223> primer
<400> 197
   agagaaatag cccggaaact tgcaaat 27
<210> 198
   <211> 29
   <212> DNA
   <213> Artificial Sequence
<220>
   <223> primer
<400> 198
   aatgaagagc ttcgaaactt gtctttgtc 29
<210> 199
   <211> 24
   <212> DNA
   <213> Artificial Sequence
<220>
   <223> primer
<400> 199
   ccacagcgtc ctgtgtttac tcat 24
<210> 200
   <211> 30
   <212> DNA
   <213> Artificial Sequence
<220>
   <223> primer
<400> 200
   ttccaaaagc tgagacaaga tcttgaaatg 30
<210> 201
   <211> 24
   <212> DNA
   <213> Artificial Sequence
<220>
   <223> primer
<400> 201
   gtgacgacgt catcaggaag caag 24
<210> 202
   <211> 23
   <212> DNA
   <213> Artificial Sequence
<220>
   <223> primer
<400> 202
   gacaacagcc ggcgtgtgga gca 23
<210> 203
   <211> 24
   <212> DNA
   <213> Artificial Sequence
<220>
   <223> primer
<400> 203
   gacttcctga cagacctgat gatg 24
<210> 204
   <211> 30
   <212> DNA
   <213> Artificial Sequence
<220>
   <223> primer
<400> 204
   gatcagtttg acaacttaga aaaacacaca 30
<210> 205
   <211> 27
   <212> DNA
   <213> Artificial Sequence
<220>
   <223> primer
<400> 205
   gctacagaca agagaaaagc tttagag 27
<210> 206
   <211> 28
   <212> DNA
   <213> Artificial Sequence
<220>
   <223> primer
<400> 206
   actgtggata ttcataagga gaaagtgg 28
<210> 207
   <211> 30
   <212> DNA
   <213> Artificial Sequence
<220>
   <223> primer
<400> 207
   gtcaagtaca aaagagattt tgaagaaagc 30
<210> 208
   <211> 31
   <212> DNA
   <213> Artificial Sequence
<220>
   <223> primer
<400> 208
   gtaaaatacc atgaagattt tgaaaaaaca a 31
<210> 209
   <211> 23
   <212> DNA
   <213> Artificial Sequence
<220>
   <223> primer
<400> 209
   aagcggtacc gcgcggtgta tga 23
<210> 210
   <211> 23
   <212> DNA
   <213> Artificial Sequence
<220>
   <223> primer
<400> 210
   ctggtcagtg agaaggtcgg agg 23
<210> 211
   <211> 27
   <212> DNA
   <213> Artificial Sequence
<220>
   <223> primer
<400> 211
   ttctccaata tccccttctt catcttc 27
<210> 212
   <211> 23
   <212> DNA
   <213> Artificial Sequence
<220>
   <223> primer
<400> 212
   ggccctcctc aggacctgtc tgt 23
<210> 213
   <211> 23
   <212> DNA
   <213> Artificial Sequence
<220>
   <223> primer
<400> 213
   ggcagcaagg agcgcttcca ctg 23
<210> 214
   <211> 24
   <212> DNA
   <213> Artificial Sequence
<220>
   <223> primer
<400> 214
   acctacatcg gctctgtgct catc 24
<210> 215
   <211> 23
   <212> DNA
   <213> Artificial Sequence
<220>
   <223> primer
<400> 215
   accttcacgg catggtgcaa ctc 23
<210> 216
   <211> 23
   <212> DNA
   <213> Artificial Sequence
<220>
   <223> primer
<400> 216
   cctatgggct atgggcctcg tat 23
<210> 217
   <211> 25
   <212> DNA
   <213> Artificial Sequence
<220>
   <223> primer
<400> 217
   gcacaaatgt ctagttcttc ctgcc 25
<210> 218
   <211> 24
   <212> DNA
   <213> Artificial Sequence
<220>
   <223> primer
<400> 218
   ctttcccagc cagctgtaag catt 24
<210> 219
   <211> 28
   <212> DNA
   <213> Artificial Sequence
<220>
   <223> primer
<400> 219
   gaaacaatga ccgataaaac agagaagg 28
<210> 220
   <211> 46
   <212> DNA
   <213> Artificial Sequence
<220>
   <223> primer
<400> 220
   gtgccagcaa gatccaatct agagcagatg gccagtcagg caccag 46
<210> 221
   <211> 52
   <212> DNA
   <213> Artificial Sequence
<220>
   <223> primer
<400> 221
   gtgccagcaa gatccaatct agatcagtta tcatctggtg acaaagcttc ag 52
<210> 222
   <211> 50
   <212> DNA
   <213> Artificial Sequence
<220>
   <223> primer
<400> 222
   gtgccagcaa gatccaatct agacgtcttc taatttcact gctgcacaag 50
<210> 223
   <211> 47
   <212> DNA
   <213> Artificial Sequence
<220>
   <223> primer
<400> 223
   gtgccagcaa gatccaatct agaggttcag cttttgccaa gcttcag 47
<210> 224
   <211> 48
   <212> DNA
   <213> Artificial Sequence
<220>
   <223> primer
<400> 224
   gtgccagcaa gatccaatct agaggctttg gatccacagc tacctcaa 48
<210> 225
   <211> 49
   <212> DNA
   <213> Artificial Sequence
<220>
   <223> primer
<400> 225
   gtgccagcaa gatccaatct agatgggttt tcctctccaa acaaaacag 49
<210> 226
   <211> 46
   <212> DNA
   <213> Artificial Sequence
<220>
   <223> primer
<400> 226
   gtgccagcaa gatccaatct agatggtttt ggatcaggca caggag 46
<210> 227
   <211> 49
   <212> DNA
   <213> Artificial Sequence
<220>
   <223> primer
<400> 227
   attatgaact attaacagaa aatgacatgt tccaaccctt agggaaccc 49
<210> 228
   <211> 47
   <212> DNA
   <213> Artificial Sequence
<220>
   <223> primer
<400> 228
   ttcttcagga gagaatacca tgggtacctc caacccttag ggaaccc 47
<210> 229
   <211> 49
   <212> DNA
   <213> Artificial Sequence
<220>
   <223> primer
<400> 229
   gaaattgaac ttagctcatt aagggaagct tccaaccctt agggaaccc 49
<210> 230
   <211> 49
   <212> DNA
   <213> Artificial Sequence
<220>
   <223> primer
<400> 230
   cgagaaaatg tcattgaata taaacactgt tccaaccctt agggaaccc 49
<210> 231
   <211> 53
   <212> DNA
   <213> Artificial Sequence
<220>
   <223> primer
<400> 231
   gtgccagcaa gatccaatct agacctagtg agagccttgc tactactgat gat 53
<210> 232
   <211> 53
   <212> DNA
   <213> Artificial Sequence
<220>
   <223> primer
<400> 232
   gtgccagcaa gatccaatct agaactgaat ctccagtgtt agtgaatgac tat 53
<210> 233
   <211> 53
   <212> DNA
   <213> Artificial Sequence
<220>
   <223> primer
<400> 233
   gtgccagcaa gatccaatct agacagtgca tattagtgga cagcacttag tag 53
<210> 234
   <211> 51
   <212> DNA
   <213> Artificial Sequence
<220>
   <223> primer
<400> 234
   gtgccagcaa gatccaatct agactgagaa tgcacttact ggctcattca g 51
<210> 235
   <211> 49
   <212> DNA
   <213> Artificial Sequence
<220>
   <223> primer
<400> 235
   gtgccagcaa gatccaatct agaggagaca cacaggcaga cccatactg 49
<210> 236
   <211> 51
   <212> DNA
   <213> Artificial Sequence
<220>
   <223> primer
<400> 236
   gtgccagcaa gatccaatct agaatcacca ttgcttggaa gtttgattct c 51
<210> 237
   <211> 49
   <212> DNA
   <213> Artificial Sequence
<220>
   <223> primer
<400> 237
   gtgccagcaa gatccaatct agaaccagtt ccctgcgagt ctgctactg 49
<210> 238
   <211> 50
   <212> DNA
   <213> Artificial Sequence
<220>
   <223> primer
<400> 238
   gtgccagcaa gatccaatct agaattacct ggtcatgatc attgtccgtg 50
<210> 239
   <211> 45
   <212> DNA
   <213> Artificial Sequence
<220>
   <223> primer
<400> 239
   tgctacagtt gaaactccag cagcgctcca acccttaggg aaccc 45
<210> 240
   <211> 45
   <212> DNA
   <213> Artificial Sequence
<220>
   <223> primer
<400> 240
   ctttgaaaag tccagccgca tttcattcca acccttaggg aaccc 45
<210> 241
   <211> 53
   <212> DNA
   <213> Artificial Sequence
<220>
   <223> primer
<400> 241
   gtgccagcaa gatccaatct agagaagtca caatgaaaca gatttgcaaa aag 53
<210> 242
   <211> 44
   <212> DNA
   <213> Artificial Sequence
<220>
   <223> primer
<400> 242
   gaaattcggc gccttcatca gtatgtccaa cccttaggga accc 44
<210> 243
   <211> 53
   <212> DNA
   <213> Artificial Sequence
<220>
   <223> primer
<400> 243
   gtgccagcaa gatccaatct agaaagaaga tgaagagtca gatgatgctg atg 53
<210> 244
   <211> 49
   <212> DNA
   <213> Artificial Sequence
<220>
   <223> primer
<400> 244
   attttggatc attgtttgac ttggaaaatg tccaaccctt agggaaccc 49
<210> 245
   <211> 45
   <212> DNA
   <213> Artificial Sequence
<220>
   <223> primer
<400> 245
   atgggaataa ctgggaacac aagtcctcca acccttaggg aaccc 45
<210> 246
   <211> 46
   <212> DNA
   <213> Artificial Sequence
<220>
   <223> primer
<400> 246
   gtgccagcaa gatccaatct agagcttggt gcaggatttg gaacag 46
<210> 247
   <211> 45
   <212> DNA
   <213> Artificial Sequence
<220>
   <223> primer
<400> 247
   gtgccagcaa gatccaatct agaggagccc cccaggcccc agtag 45
<210> 248
   <211> 51
   <212> DNA
   <213> Artificial Sequence
<220>
   <223> primer
<400> 248
   gtgccagcaa gatccaatct agagatgtca gaccctaaga agaaggaaga g 51
<210> 249
   <211> 47
   <212> DNA
   <213> Artificial Sequence
<220>
   <223> primer
<400> 249
   gtgccagcaa gatccaatct agaagccaat ggagcattca tgcccaa 47
<210> 250
   <211> 42
   <212> DNA
   <213> Artificial Sequence
<220>
   <223> primer
<400> 250
   ctgtgcggtc agagaagaaa cgctccaacc cttagggaac cc 42
<210> 251
   <211> 46
   <212> DNA
   <213> Artificial Sequence
<220>
   <223> primer
<400> 251
   ttgatagaga aaaacaaccc agcgaagtcc aacccttagg gaaccc 46
<210> 252
   <211> 43
   <212> DNA
   <213> Artificial Sequence
<220>
   <223> primer
<400> 252
   ataacccagc agccaactgg cttctccaac ccttagggaa ccc 43
<210> 253
   <211> 46
   <212> DNA
   <213> Artificial Sequence
<220>
   <223> primer
<400> 253
   gatggtaaat tgaaaaaacc caagaattcc aacccttagg gaaccc 46
<210> 254
   <211> 46
   <212> DNA
   <213> Artificial Sequence
<220>
   <223> primer
<400> 254
   ttcttgatga agcagataga atcttggtcc aacccttagg gaaccc 46
<210> 255
   <211> 42
   <212> DNA
   <213> Artificial Sequence
<220>
   <223> primer
<400> 255
   cacccctgcc actttggaac agatccaacc cttagggaac cc 42
<210> 256
   <211> 48
   <212> DNA
   <213> Artificial Sequence
<220>
   <223> primer
<400> 256
   ataatctcag tgataccttg aagaagctgt ccaaccctta gggaaccc 48
<210> 257
   <211> 47
   <212> DNA
   <213> Artificial Sequence
<220>
   <223> primer
<400> 257
   atacggaaac aagtgaaaaa atccaagctc caacccttag ggaaccc 47
<210> 258
   <211> 52
   <212> DNA
   <213> Artificial Sequence
<220>
   <223> primer
<400> 258
   gtgccagcaa gatccaatct agaaccaaga ggctattcaa gatctctgtc tg 52
<210> 259
   <211> 52
   <212> DNA
   <213> Artificial Sequence
<220>
   <223> primer
<400> 259
   gtgccagcaa gatccaatct agaaccaaga ggctattcaa gatctctgca tg 52
<210> 260
   <211> 49
   <212> DNA
   <213> Artificial Sequence
<220>
   <223> primer
<400> 260
   gcagtggagg aagtctcttt aagaaaatag tccaaccctt agggaaccc 49
<210> 261
   <211> 46
   <212> DNA
   <213> Artificial Sequence
<220>
   <223> primer
<400> 261
   gtgccagcaa gatccaatct agacttctgc cgctgcttct gcacag 46
<210> 262
   <211> 42
   <212> DNA
   <213> Artificial Sequence
<220>
   <223> primer
<400> 262
   gcatggggcg gctggttctg ctgtccaacc cttagggaac cc 42
<210> 263
   <211> 47
   <212> DNA
   <213> Artificial Sequence
<220>
   <223> primer
<400> 263
   gtgccagcaa gatccaatct agactgagga catctggagg aaggctg 47
<210> 264
   <211> 44
   <212> DNA
   <213> Artificial Sequence
<220>
   <223> primer
<400> 264
   gtgccagcaa gatccaatct agacagcagg aggactccag cgag 44
<210> 265
   <211> 43
   <212> DNA
   <213> Artificial Sequence
<220>
   <223> primer
<400> 265
   gcttcctgct gaactccaag ttcctccaac ccttagggaa ccc 43
<210> 266
   <211> 42
   <212> DNA
   <213> Artificial Sequence
<220>
   <223> primer
<400> 266
   gacttccagc cactgcgcta ttttccaacc cttagggaac cc 42
<210> 267
   <211> 53
   <212> DNA
   <213> Artificial Sequence
<220>
   <223> primer
<400> 267
   gtgccagcaa gatccaatct agaatccctg taaaacaaaa accaaaagaa aag 53
<210> 268
   <211> 51
   <212> DNA
   <213> Artificial Sequence
<220>
   <223> primer
<400> 268
   gtgccagcaa gatccaatct agaagtccac aggatcagag tggactttaa g 51
<210> 269
   <211> 50
   <212> DNA
   <213> Artificial Sequence
<220>
   <223> primer
<400> 269
   gtgccagcaa gatccaatct agactctgtg ccagtagtgg gcatgtagag 50
<210> 270
   <211> 49
   <212> DNA
   <213> Artificial Sequence
<220>
   <223> primer
<400> 270
   gtgccagcaa gatccaatct agagtggaag gcaacatcag gctacaaag 49
<210> 271
   <211> 45
   <212> DNA
   <213> Artificial Sequence
<220>
   <223> primer
<400> 271
   cagacctact ccaatgaagt ccattgtcca acccttaggg aaccc 45
<210> 272
   <211> 42
   <212> DNA
   <213> Artificial Sequence
<220>
   <223> primer
<400> 272
   gaaatgaccc attcatggcc gcctccaacc cttagggaac cc 42
<210> 273
   <211> 45
   <212> DNA
   <213> Artificial Sequence
<220>
   <223> primer
<400> 273
   gactctcagc atgtcagttc tgtaactcca acccttaggg aaccc 45
<210> 274
   <211> 45
   <212> DNA
   <213> Artificial Sequence
<220>
   <223> primer
<400> 274
   cctgagcctc caacaacaaa caaatgtcca acccttaggg aaccc 45
<210> 275
   <211> 46
   <212> DNA
   <213> Artificial Sequence
<220>
   <223> primer
<400> 275
   gatgagcaat tcttaggttt tggctcatcc aacccttagg gaaccc 46
<210> 276
   <211> 44
   <212> DNA
   <213> Artificial Sequence
<220>
   <223> primer
<400> 276
   gaagaaccta ggaaagtccg ctttgtccaa cccttaggga accc 44
<210> 277
   <211> 46
   <212> DNA
   <213> Artificial Sequence
<220>
   <223> primer
<400> 277
   gaccctaata ggagtattca taccagctcc aacccttagg gaaccc 46
<210> 278
   <211> 42
   <212> DNA
   <213> Artificial Sequence
<220>
   <223> primer
<400> 278
   tctgaacaac ccagtcctgc cagtccaacc cttagggaac cc 42
<210> 279
   <211> 50
   <212> DNA
   <213> Artificial Sequence
<220>
   <223> primer
<400> 279
   attcttgaag tgaaaagtcc aataaagcaa atccaaccct tagggaaccc 50
<210> 280
   <211> 44
   <212> DNA
   <213> Artificial Sequence
<220>
   <223> primer
<400> 280
   gcatacctag atgaactggt agagctccaa cccttaggga accc 44
<210> 281
   <211> 42
   <212> DNA
   <213> Artificial Sequence
<220>
   <223> primer
<400> 281
   tgcaccgtcc aggtgaggtt agatccaacc cttagggaac cc 42
<210> 282
   <211> 42
   <212> DNA
   <213> Artificial Sequence
<220>
   <223> primer
<400> 282
   gaggagccga ggaaggtctg ctttccaacc cttagggaac cc 42
<210> 283
   <211> 42
   <212> DNA
   <213> Artificial Sequence
<220>
   <223> primer
<400> 283
   gtgatggtaa tgcccgaagg agctccaacc cttagggaac cc 42
<210> 284
   <211> 42
   <212> DNA
   <213> Artificial Sequence
<220>
   <223> primer
<400> 284
   gacgccaaca aggagagcag caatccaacc cttagggaac cc 42
<210> 285
   <211> 42
   <212> DNA
   <213> Artificial Sequence
<220>
   <223> primer
<400> 285
   tctgcccagt caagcccgtc caatccaacc cttagggaac cc 42
<210> 286
   <211> 46
   <212> DNA
   <213> Artificial Sequence
<220>
   <223> primer
<400> 286
   agatgtgaac tttgtcccca taaggattcc aacccttagg gaaccc 46
<210> 287
   <211> 48
   <212> DNA
   <213> Artificial Sequence
<220>
   <223> primer
<400> 287
   acttgctaca tttgtgatga acaaggaagt ccaaccctta gggaaccc 48
<210> 288
   <211> 50
   <212> DNA
   <213> Artificial Sequence
<220>
   <223> primer
<400> 288
   aaatataaag agaaggacaa acacaaacag atccaaccct tagggaaccc 50
<210> 289
   <211> 49
   <212> DNA
   <213> Artificial Sequence
<220>
   <223> primer
<400> 289
   acttatacaa gcactagcaa caactctata tccaaccctt agggaaccc 49
<210> 290
   <211> 46
   <212> DNA
   <213> Artificial Sequence
<220>
   <223> primer
<400> 290
   gcaaatactc tatctggatc ttctctctcc aacccttagg gaaccc 46
<210> 291
   <211> 45
   <212> DNA
   <213> Artificial Sequence
<220>
   <223> primer
<400> 291
   gatttggagt tccatggagt gatgagtcca acccttaggg aaccc 45
<210> 292
   <211> 46
   <212> DNA
   <213> Artificial Sequence
<220>
   <223> primer
<400> 292
   gattctgttt cactgaggcc atctatctcc aacccttagg gaaccc 46
<210> 293
   <211> 42
   <212> DNA
   <213> Artificial Sequence
<220>
   <223> primer
<400> 293
   cacatctcca tcccccagcc tgatccaacc cttagggaac cc 42
<210> 294
   <211> 43
   <212> DNA
   <213> Artificial Sequence
<220>
   <223> primer
<400> 294
   gtggccaaca tgagtgctaa ggactccaac ccttagggaa ccc 43
<210> 295
   <211> 50
   <212> DNA
   <213> Artificial Sequence
<220>
   <223> primer
<400> 295
   ttatcaagtg ggaatcctgt atatgaaaaa ttccaaccct tagggaaccc 50
<210> 296
   <211> 46
   <212> DNA
   <213> Artificial Sequence
<220>
   <223> primer
<400> 296
   catgatacca gtagtccttt gctaatctcc aacccttagg gaaccc 46
<210> 297
   <211> 46
   <212> DNA
   <213> Artificial Sequence
<220>
   <223> primer
<400> 297
   agtcgaaagg acaaagaacg ccttaagtcc aacccttagg gaaccc 46
<210> 298
   <211> 42
   <212> DNA
   <213> Artificial Sequence
<220>
   <223> primer
<400> 298
   gtctcctcct cggcttcctc ttctccaacc cttagggaac cc 42
<210> 299
   <211> 43
   <212> DNA
   <213> Artificial Sequence
<220>
   <223> primer
<400> 299
   gtgttttctc tggctggctc tacctccaac ccttagggaa ccc 43
<210> 300
   <211> 42
   <212> DNA
   <213> Artificial Sequence
<220>
   <223> primer
<400> 300
   ggtgaaggtt gccgaactgt ccctccaacc cttagggaac cc 42
<210> 301
   <211> 42
   <212> DNA
   <213> Artificial Sequence
<220>
   <223> primer
<400> 301
   gaagctatga gggaccctgt gagtccaacc cttagggaac cc 42
<210> 302
   <211> 43
   <212> DNA
   <213> Artificial Sequence
<220>
   <223> primer
<400> 302
   gcacggacac ttgctagtat gttgtccaac ccttagggaa ccc 43
<210> 303
   <211> 43
   <212> DNA
   <213> Artificial Sequence
<220>
   <223> primer
<400> 303
   gcatcagctt ctggtgatgt gagctccaac ccttagggaa ccc 43
<210> 304
   <211> 42
   <212> DNA
   <213> Artificial Sequence
<220>
   <223> primer
<400> 304
   gagatgacgc attcatggcc tcctccaacc cttagggaac cc 42
<210> 305
   <211> 43
   <212> DNA
   <213> Artificial Sequence
<220>
   <223> primer
<400> 305
   gagtctcagc agtccaattt tggctccaac ccttagggaa ccc 43
<210> 306
   <211> 45
   <212> DNA
   <213> Artificial Sequence
<220>
   <223> primer
<400> 306
   atatttaaca ccgtgcccga tatgcctcca acccttaggg aaccc 45
<210> 307
   <211> 46
   <212> DNA
   <213> Artificial Sequence
<220>
   <223> primer
<400> 307
   aagatggcag tgaacgtata ctcaacgtcc aacccttagg gaaccc 46
<210> 308
   <211> 49
   <212> DNA
   <213> Artificial Sequence
<220>
   <223> primer
<400> 308
   ataattcctg tggacaaatt agtaaaagga tccaaccctt agggaaccc 49
<210> 309
   <211> 49
   <212> DNA
   <213> Artificial Sequence
<220>
   <223> primer
<400> 309
   gtcaacgtat tgaaacttac tgttgaagac tccaaccctt agggaaccc 49
<210> 310
   <211> 43
   <212> DNA
   <213> Artificial Sequence
<220>
   <223> primer
<400> 310
   gacattgaca agcagtacgt gggctccaac ccttagggaa ccc 43
<210> 311
   <211> 42
   <212> DNA
   <213> Artificial Sequence
<220>
   <223> primer
<400> 311
   gaggcacgcg gacctccagt ggctccaacc cttagggaac cc 42
<210> 312
   <211> 46
   <212> DNA
   <213> Artificial Sequence
<220>
   <223> primer
<400> 312
   ccttgaaaag atcttttgag gtcgaggtcc aacccttagg gaaccc 46
<210> 313
   <211> 50
   <212> DNA
   <213> Artificial Sequence
<220>
   <223> primer
<400> 313
   gaaaaaaacc ttgaagataa cttacagagt ttccaaccct tagggaaccc 50
<210> 314
   <211> 45
   <212> DNA
   <213> Artificial Sequence
<220>
   <223> primer
<400> 314
   gagagtagat ctggagaaac caacagtcca acccttaggg aaccc 45
<210> 315
   <211> 42
   <212> DNA
   <213> Artificial Sequence
<220>
   <223> primer
<400> 315
   gagatgacct ggcttccacc acttccaacc cttagggaac cc 42
<210> 316
   <211> 42
   <212> DNA
   <213> Artificial Sequence
<220>
   <223> primer
<400> 316
   caggcagctc agagaacggc tcttccaacc cttagggaac cc 42
<210> 317
   <211> 50
   <212> DNA
   <213> Artificial Sequence
<220>
   <223> primer
<400> 317
   atttttgatc accatactga agaggatata gtccaaccct tagggaaccc 50
<210> 318
   <211> 42
   <212> DNA
   <213> Artificial Sequence
<220>
   <223> primer
<400> 318
   aactccatcc ggcacaacct gtctccaacc cttagggaac cc 42
<210> 319
   <211> 46
   <212> DNA
   <213> Artificial Sequence
<220>
   <223> primer
<400> 319
   ctccagggtt ccttgaaaag aaaacagtcc aacccttagg gaaccc 46
<210> 320
   <211> 43
   <212> DNA
   <213> Artificial Sequence
<220>
   <223> primer
<400> 320
   gatcaacact ctgtggtagg ccagtccaac ccttagggaa ccc 43
<210> 321
   <211> 47
   <212> DNA
   <213> Artificial Sequence
<220>
   <223> primer
<400> 321
   caagttggaa ttgacagagg tgatatactc caacccttag ggaaccc 47
<210> 322
   <211> 42
   <212> DNA
   <213> Artificial Sequence
<220>
   <223> primer
<400> 322
   gcccctagca gtcttcttga tgctccaacc cttagggaac cc 42
<210> 323
   <211> 45
   <212> DNA
   <213> Artificial Sequence
<220>
   <223> primer
<400> 323
   caacctcata atattctgca gaggcgtcca acccttaggg aaccc 45
<210> 324
   <211> 42
   <212> DNA
   <213> Artificial Sequence
<220>
   <223> primer
<400> 324
   gacatgcgga agcacgtggc cattccaacc cttagggaac cc 42
<210> 325
   <211> 42
   <212> DNA
   <213> Artificial Sequence
<220>
   <223> primer
<400> 325
   ggctacatgc agccgctgaa gcatccaacc cttagggaac cc 42
<210> 326
   <211> 46
   <212> DNA
   <213> Artificial Sequence
<220>
   <223> primer
<400> 326
   ttctccaagg atgtcctagt aaacatctcc aacccttagg gaaccc 46
<210> 327
   <211> 43
   <212> DNA
   <213> Artificial Sequence
<220>
   <223> primer
<400> 327
   caaagcatgc gtgagaacaa ggagtccaac ccttagggaa ccc 43
<210> 328
   <211> 44
   <212> DNA
   <213> Artificial Sequence
<220>
   <223> primer
<400> 328
   ccagaagtca ttggatctgt gtcactccaa cccttaggga accc 44
<210> 329
   <211> 49
   <212> DNA
   <213> Artificial Sequence
<220>
   <223> primer
<400> 329
   gtaaccatgg agcttattac agataacaaa tccaaccctt agggaaccc 49
<210> 330
   <211> 43
   <212> DNA
   <213> Artificial Sequence
<220>
   <223> primer
<400> 330
   gtgattcctg tctctctgtc ttcctccaac ccttagggaa ccc 43
<210> 331
   <211> 42
   <212> DNA
   <213> Artificial Sequence
<220>
   <223> primer
<400> 331
   gcagaggacc gaggaaatgg acttccaacc cttagggaac cc 42
<210> 332
   <211> 43
   <212> DNA
   <213> Artificial Sequence
<220>
   <223> primer
<400> 332
   gatggagctg tagttacacc ctcctccaac ccttagggaa ccc 43
<210> 333
   <211> 43
   <212> DNA
   <213> Artificial Sequence
<220>
   <223> primer
<400> 333
   agtcccaaga gtggcccaaa agagtccaac ccttagggaa ccc 43
<210> 334
   <211> 44
   <212> DNA
   <213> Artificial Sequence
<220>
   <223> primer
<400> 334
   gacactcaat cacttgtcgg aagtctccaa cccttaggga accc 44
<210> 335
   <211> 42
   <212> DNA
   <213> Artificial Sequence
<220>
   <223> primer
<400> 335
   gtggaacggc cgccttctcc atttccaacc cttagggaac cc 42
<210> 336
   <211> 42
   <212> DNA
   <213> Artificial Sequence
<220>
   <223> primer
<400> 336
   gtgaatgagg cctctgggga tggtccaacc cttagggaac cc 42
<210> 337
   <211> 42
   <212> DNA
   <213> Artificial Sequence
<220>
   <223> primer
<400> 337
   aagcctggaa tggtcccccc tcctccaacc cttagggaac cc 42
<210> 338
   <211> 42
   <212> DNA
   <213> Artificial Sequence
<220>
   <223> primer
<400> 338
   aactcgatcc gccacaacct gtctccaacc cttagggaac cc 42
<210> 339
   <211> 47
   <212> DNA
   <213> Artificial Sequence
<220>
   <223> primer
<400> 339
   caacagccaa ctcagtttat aaatccagtc caacccttag ggaaccc 47
<210> 340
   <211> 44
   <212> DNA
   <213> Artificial Sequence
<220>
   <223> primer
<400> 340
   ctgcagaaga aagatcagca actggtccaa cccttaggga accc 44
<210> 341
   <211> 45
   <212> DNA
   <213> Artificial Sequence
<220>
   <223> primer
<400> 341
   atgggaataa ctgggaacac aagtcctcca acccttaggg aaccc 45
<210> 342
   <211> 46
   <212> DNA
   <213> Artificial Sequence
<220>
   <223> primer
<400> 342
   tctcagatgc aaacatcagt gggaatttcc aacccttagg gaaccc 46
<210> 343
   <211> 44
   <212> DNA
   <213> Artificial Sequence
<220>
   <223> primer
<400> 343
   atgatggagg aggatttgca aggagtccaa cccttaggga accc 44
<210> 344
   <211> 44
   <212> DNA
   <213> Artificial Sequence
<220>
   <223> primer
<400> 344
   gtgagtaccc agaacatgaa gatggtccaa cccttaggga accc 44
<210> 345
   <211> 42
   <212> DNA
   <213> Artificial Sequence
<220>
   <223> primer
<400> 345
   ggcctggagc aggatgtcct ccatccaacc cttagggaac cc 42
<210> 346
   <211> 46
   <212> DNA
   <213> Artificial Sequence
<220>
   <223> primer
<400> 346
   aatcagatgg gtgactcaaa tatctcctcc aacccttagg gaaccc 46
<210> 347
   <211> 43
   <212> DNA
   <213> Artificial Sequence
<220>
   <223> primer
<400> 347
   tctgctccat ctggacacaa gcattccaac ccttagggaa ccc 43
<210> 348
   <211> 42
   <212> DNA
   <213> Artificial Sequence
<220>
   <223> primer
<400> 348
   ctggagaatg ctggaggaga ccttccaacc cttagggaac cc 42
<210> 349
   <211> 45
   <212> DNA
   <213> Artificial Sequence
<220>
   <223> primer
<400> 349
   aatactctgg agcagtgcaa tgtgtgtcca acccttaggg aaccc 45
<210> 350
   <211> 45
   <212> DNA
   <213> Artificial Sequence
<220>
   <223> primer
<400> 350
   aatactctgg agcagtgcaa tgtgtgtcca acccttaggg aaccc 45
<210> 351
   <211> 46
   <212> DNA
   <213> Artificial Sequence
<220>
   <223> primer
<400> 351
   agagaaatag cccggaaact tgcaaattcc aacccttagg gaaccc 46
<210> 352
   <211> 48
   <212> DNA
   <213> Artificial Sequence
<220>
   <223> primer
<400> 352
   aatgaagagc ttcgaaactt gtctttgtct ccaaccctta gggaaccc 48
<210> 353
   <211> 43
   <212> DNA
   <213> Artificial Sequence
<220>
   <223> primer
<400> 353
   ccacagcgtc ctgtgtttac tcattccaac ccttagggaa ccc 43
<210> 354
   <211> 49
   <212> DNA
   <213> Artificial Sequence
<220>
   <223> primer
<400> 354
   ttccaaaagc tgagacaaga tcttgaaatg tccaaccctt agggaaccc 49
<210> 355
   <211> 43
   <212> DNA
   <213> Artificial Sequence
<220>
   <223> primer
<400> 355
   gtgacgacgt catcaggaag caagtccaac ccttagggaa ccc 43
<210> 356
   <211> 42
   <212> DNA
   <213> Artificial Sequence
<220>
   <223> primer
<400> 356
   gacaacagcc ggcgtgtgga gcatccaacc cttagggaac cc 42
<210> 357
   <211> 43
   <212> DNA
   <213> Artificial Sequence
<220>
   <223> primer
<400> 357
   gacttcctga cagacctgat gatgtccaac ccttagggaa ccc 43
<210> 358
   <211> 49
   <212> DNA
   <213> Artificial Sequence
<220>
   <223> primer
<400> 358
   gatcagtttg acaacttaga aaaacacaca tccaaccctt agggaaccc 49
<210> 359
   <211> 46
   <212> DNA
   <213> Artificial Sequence
<220>
   <223> primer
<400> 359
   gctacagaca agagaaaagc tttagagtcc aacccttagg gaaccc 46
<210> 360
   <211> 47
   <212> DNA
   <213> Artificial Sequence
<220>
   <223> primer
<400> 360
   actgtggata ttcataagga gaaagtggtc caacccttag ggaaccc 47
<210> 361
   <211> 49
   <212> DNA
   <213> Artificial Sequence
<220>
   <223> primer
<400> 361
   gtcaagtaca aaagagattt tgaagaaagc tccaaccctt agggaaccc 49
<210> 362
   <211> 50
   <212> DNA
   <213> Artificial Sequence
<220>
   <223> primer
<400> 362
   gtaaaatacc atgaagattt tgaaaaaaca atccaaccct tagggaaccc 50
<210> 363
   <211> 42
   <212> DNA
   <213> Artificial Sequence
<220>
   <223> primer
<400> 363
   aagcggtacc gcgcggtgta tgatccaacc cttagggaac cc 42
<210> 364
   <211> 42
   <212> DNA
   <213> Artificial Sequence
<220>
   <223> primer
<400> 364
   ctggtcagtg agaaggtcgg aggtccaacc cttagggaac cc 42
<210> 365
   <211> 46
   <212> DNA
   <213> Artificial Sequence
<220>
   <223> primer
<400> 365
   ttctccaata tccccttctt catcttctcc aacccttagg gaaccc 46
<210> 366
   <211> 42
   <212> DNA
   <213> Artificial Sequence
<220>
   <223> primer
<400> 366
   ggccctcctc aggacctgtc tgttccaacc cttagggaac cc 42
<210> 367
   <211> 42
   <212> DNA
   <213> Artificial Sequence
<220>
   <223> primer
<400> 367
   ggcagcaagg agcgcttcca ctgtccaacc cttagggaac cc 42
<210> 368
   <211> 43
   <212> DNA
   <213> Artificial Sequence
<220>
   <223> primer
<400> 368
   acctacatcg gctctgtgct catctccaac ccttagggaa ccc 43
<210> 369
   <211> 42
   <212> DNA
   <213> Artificial Sequence
<220>
   <223> primer
<400> 369
   accttcacgg catggtgcaa ctctccaacc cttagggaac cc 42
<210> 370
   <211> 42
   <212> DNA
   <213> Artificial Sequence
<220>
   <223> primer
<400> 370
   cctatgggct atgggcctcg tattccaacc cttagggaac cc 42
<210> 371
   <211> 44
   <212> DNA
   <213> Artificial Sequence
<220>
   <223> primer
<400> 371
   gcacaaatgt ctagttcttc ctgcctccaa cccttaggga accc 44
<210> 372
   <211> 43
   <212> DNA
   <213> Artificial Sequence
<220>
   <223> primer
<400> 372
   ctttcccagc cagctgtaag catttccaac ccttagggaa ccc 43
<210> 373
   <211> 47
   <212> DNA
   <213> Artificial Sequence
<220>
   <223> primer
<400> 373
   gaaacaatga ccgataaaac agagaaggtc caacccttag ggaaccc 47
<210> 374
   <211> 22
   <212> DNA
   <213> Artificial Sequence
<220>
   <223> primer
<400> 374
   agcagcagct acgggcagca ga 22
<210> 375
   <211> 22
   <212> DNA
   <213> Artificial Sequence
<220>
   <223> primer
<400> 375
   gaggaggacg cggtggaatg gg 22
<210> 376
   <211> 24
   <212> DNA
   <213> Artificial Sequence
<220>
   <223> primer
<400> 376
   gaggtggctt caataagcct ggtg 24
<210> 377
   <211> 27
   <212> DNA
   <213> Artificial Sequence
<220>
   <223> primer
<400> 377
   tggatgaagg accagatctt gatctag 27
<210> 378
   <211> 28
   <212> DNA
   <213> Artificial Sequence
<220>
   <223> primer
<400> 378
   gttcactgct ggcctataat acaacctc 28
<210> 379
   <211> 30
   <212> DNA
   <213> Artificial Sequence
<220>
   <223> primer
<400> 379
   acccttctta tgactcagtc agaagaggag 30
<210> 380
   <211> 23
   <212> DNA
   <213> Artificial Sequence
<220>
   <223> primer
<400> 380
   gtcctcccct tggaggggca caa 23
<210> 381
   <211> 26
   <212> DNA
   <213> Artificial Sequence
<220>
   <223> primer
<400> 381
   gttattccag gatctttgga gacccg 26
<210> 382
   <211> 28
   <212> DNA
   <213> Artificial Sequence
<220>
   <223> primer
<400> 382
   gaagccttat cagttgtgag tgaggacc 28
<210> 383
   <211> 24
   <212> DNA
   <213> Artificial Sequence
<220>
   <223> primer
<400> 383
   atttaccata tgagcccccc agga 24
<210> 384
   <211> 25
   <212> DNA
   <213> Artificial Sequence
<220>
   <223> primer
<400> 384
   ctgctcaacc atctccttcc acagt 25
<210> 385
   <211> 30
   <212> DNA
   <213> Artificial Sequence
<220>
   <223> primer
<400> 385
   atccttatca gattcttgga ccaacaagta 30
<210> 386
   <211> 22
   <212> DNA
   <213> Artificial Sequence
<220>
   <223> primer
<400> 386
   gcagtggcca gatccagctt tg 22
<210> 387
   <211> 23
   <212> DNA
   <213> Artificial Sequence
<220>
   <223> primer
<400> 387
   atcccgtcgg agacggtctc ttc 23
<210> 388
   <211> 28
   <212> DNA
   <213> Artificial Sequence
<220>
   <223> primer
<400> 388
   ctcaggtacc tgacaatgat gagcagtt 28
<210> 389
   <211> 29
   <212> DNA
   <213> Artificial Sequence
<220>
   <223> primer
<400> 389
   gagacatcaa acaagagcca ggaatgtat 29
<210> 390
   <211> 22
   <212> DNA
   <213> Artificial Sequence
<220>
   <223> primer
<400> 390
   atgtcaccgg gtgcgcatca at 22
<210> 391
   <211> 24
   <212> DNA
   <213> Artificial Sequence
<220>
   <223> primer
<400> 391
   attggcaatg gcctctcacc tcag 24
<210> 392
   <211> 30
   <212> DNA
   <213> Artificial Sequence
<220>
   <223> primer
<400> 392
   aattcaattc gtcataatct gtccctacac 30
<210> 393
   <211> 29
   <212> DNA
   <213> Artificial Sequence
<220>
   <223> primer
<400> 393
   gtgagaaacc ataccagtgt gacttcaag 29
<210> 394
   <211> 23
   <212> DNA
   <213> Artificial Sequence
<220>
   <223> primer
<400> 394
   cccaggacag cagcagggct acg 23
<210> 395
   <211> 26
   <212> DNA
   <213> Artificial Sequence
<220>
   <223> primer
<400> 395
   agcagaggcc ttatggatat gaccag 26
<210> 396
   <211> 23
   <212> DNA
   <213> Artificial Sequence
<220>
   <223> primer
<400> 396
   atcatgccca agaagccagc aga 23
<210> 397
   <211> 25
   <212> DNA
   <213> Artificial Sequence
<220>
   <223> primer
<400> 397
   gtttcaaagt caccctccca ccttt 25
<210> 398
   <211> 30
   <212> DNA
   <213> Artificial Sequence
<220>
   <223> primer
<400> 398
   tgttcaagaa ggaagtgtat cttcatacat 30
<210> 399
   <211> 27
   <212> DNA
   <213> Artificial Sequence
<220>
   <223> primer
<400> 399
   atgtgctttt ccagactgat ccaactg 27
<210> 400
   <211> 23
   <212> DNA
   <213> Artificial Sequence
<220>
   <223> primer
<400> 400
   tggaggtgga ggtggaggtg gag 23
<210> 401
   <211> 23
   <212> DNA
   <213> Artificial Sequence
<220>
   <223> primer
<400> 401
   cgtggaggca gaggtggcat ggg 23
<210> 402
   <211> 25
   <212> DNA
   <213> Artificial Sequence
<220>
   <223> primer
<400> 402
   cgtggtggct tcaataaatt tggtg 25
<210> 403
   <211> 24
   <212> DNA
   <213> Artificial Sequence
<220>
   <223> primer
<400> 403
   accaaggatc acgtcatgac tccg 24
<210> 404
   <211> 23
   <212> DNA
   <213> Artificial Sequence
<220>
   <223> primer
<400> 404
   ggctgccgtg gaatggtttg atg 23
<210> 405
   <211> 28
   <212> DNA
   <213> Artificial Sequence
<220>
   <223> primer
<400> 405
   cttctttaag cagtgtgggg ttgttaag 28
<210> 406
   <211> 45
   <212> DNA
   <213> Artificial Sequence
<220>
   <223> primer
<400> 406
   gtgccagcaa gatccaatct agaagcagca gctacgggca gcaga 45
<210> 407
   <211> 45
   <212> DNA
   <213> Artificial Sequence
<220>
   <223> primer
<400> 407
   gtgccagcaa gatccaatct agagaggagg acgcggtgga atggg 45
<210> 408
   <211> 47
   <212> DNA
   <213> Artificial Sequence
<220>
   <223> primer
<400> 408
   gtgccagcaa gatccaatct agagaggtgg cttcaataag cctggtg 47
<210> 409
   <211> 50
   <212> DNA
   <213> Artificial Sequence
<220>
   <223> primer
<400> 409
   gtgccagcaa gatccaatct agatggatga aggaccagat cttgatctag 50
<210> 410
   <211> 47
   <212> DNA
   <213> Artificial Sequence
<220>
   <223> primer
<400> 410
   gttcactgct ggcctataat acaacctctc caacccttag ggaaccc 47
<210> 411
   <211> 49
   <212> DNA
   <213> Artificial Sequence
<220>
   <223> primer
<400> 411
   acccttctta tgactcagtc agaagaggag tccaaccctt agggaaccc 49
<210> 412
   <211> 42
   <212> DNA
   <213> Artificial Sequence
<220>
   <223> primer
<400> 412
   gtcctcccct tggaggggca caatccaacc cttagggaac cc 42
<210> 413
   <211> 45
   <212> DNA
   <213> Artificial Sequence
<220>
   <223> primer
<400> 413
   gttattccag gatctttgga gacccgtcca acccttaggg aaccc 45
<210> 414
   <211> 47
   <212> DNA
   <213> Artificial Sequence
<220>
   <223> primer
<400> 414
   gaagccttat cagttgtgag tgaggacctc caacccttag ggaaccc 47
<210> 415
   <211> 43
   <212> DNA
   <213> Artificial Sequence
<220>
   <223> primer
<400> 415
   atttaccata tgagcccccc aggatccaac ccttagggaa ccc 43
<210> 416
   <211> 44
   <212> DNA
   <213> Artificial Sequence
<220>
   <223> primer
<400> 416
   ctgctcaacc atctccttcc acagttccaa cccttaggga accc 44
<210> 417
   <211> 49
   <212> DNA
   <213> Artificial Sequence
<220>
   <223> primer
<400> 417
   atccttatca gattcttgga ccaacaagta tccaaccctt agggaaccc 49
<210> 418
   <211> 41
   <212> DNA
   <213> Artificial Sequence
<220>
   <223> primer
<400> 418
   gcagtggcca gatccagctt tgtccaaccc ttagggaacc c 41
<210> 419
   <211> 42
   <212> DNA
   <213> Artificial Sequence
<220>
   <223> primer
<400> 419
   atcccgtcgg agacggtctc ttctccaacc cttagggaac cc 42
<210> 420
   <211> 47
   <212> DNA
   <213> Artificial Sequence
<220>
   <223> primer
<400> 420
   ctcaggtacc tgacaatgat gagcagtttc caacccttag ggaaccc 47
<210> 421
   <211> 48
   <212> DNA
   <213> Artificial Sequence
<220>
   <223> primer
<400> 421
   gagacatcaa acaagagcca ggaatgtatt ccaaccctta gggaaccc 48
<210> 422
   <211> 41
   <212> DNA
   <213> Artificial Sequence
<220>
   <223> primer
<400> 422
   atgtcaccgg gtgcgcatca attccaaccc ttagggaacc c 41
<210> 423
   <211> 47
   <212> DNA
   <213> Artificial Sequence
<220>
   <223> primer
<400> 423
   gtgccagcaa gatccaatct agaattggca atggcctctc acctcag 47
<210> 424
   <211> 49
   <212> DNA
   <213> Artificial Sequence
<220>
   <223> primer
<400> 424
   aattcaattc gtcataatct gtccctacac tccaaccctt agggaaccc 49
<210> 425
   <211> 48
   <212> DNA
   <213> Artificial Sequence
<220>
   <223> primer
<400> 425
   gtgagaaacc ataccagtgt gacttcaagt ccaaccctta gggaaccc 48
<210> 426
   <211> 46
   <212> DNA
   <213> Artificial Sequence
<220>
   <223> primer
<400> 426
   gtgccagcaa gatccaatct agacccagga cagcagcagg gctacg 46
<210> 427
   <211> 49
   <212> DNA
   <213> Artificial Sequence
<220>
   <223> primer
<400> 427
   gtgccagcaa gatccaatct agaagcagag gccttatgga tatgaccag 49
<210> 428
   <211> 42
   <212> DNA
   <213> Artificial Sequence
<220>
   <223> primer
<400> 428
   atcatgccca agaagccagc agatccaacc cttagggaac cc 42
<210> 429
   <211> 44
   <212> DNA
   <213> Artificial Sequence
<220>
   <223> primer
<400> 429
   gtttcaaagt caccctccca ccttttccaa cccttaggga accc 44
<210> 430
   <211> 49
   <212> DNA
   <213> Artificial Sequence
<220>
   <223> primer
<400> 430
   tgttcaagaa ggaagtgtat cttcatacat tccaaccctt agggaaccc 49
<210> 431
   <211> 46
   <212> DNA
   <213> Artificial Sequence
<220>
   <223> primer
<400> 431
   atgtgctttt ccagactgat ccaactgtcc aacccttagg gaaccc 46
<210> 432
   <211> 46
   <212> DNA
   <213> Artificial Sequence
<220>
   <223> primer
<400> 432
   gtgccagcaa gatccaatct agatggaggt ggaggtggag gtggag 46
<210> 433
   <211> 46
   <212> DNA
   <213> Artificial Sequence
<220>
   <223> primer
<400> 433
   gtgccagcaa gatccaatct agacgtggag gcagaggtgg catggg 46
<210> 434
   <211> 48
   <212> DNA
   <213> Artificial Sequence
<220>
   <223> primer
<400> 434
   gtgccagcaa gatccaatct agacgtggtg gcttcaataa atttggtg 48
<210> 435
   <211> 47
   <212> DNA
   <213> Artificial Sequence
<220>
   <223> primer
<400> 435
   gtgccagcaa gatccaatct agaaccaagg atcacgtcat gactccg 47
<210> 436
   <211> 46
   <212> DNA
   <213> Artificial Sequence
<220>
   <223> primer
<400> 436
   gtgccagcaa gatccaatct agaggctgcc gtggaatggt ttgatg 46
<210> 437
   <211> 51
   <212> DNA
   <213> Artificial Sequence
<220>
   <223> primer
<400> 437
   gtgccagcaa gatccaatct agacttcttt aagcagtgtg gggttgttaa g 51
<210> 438
   <211> 25
   <212> DNA
   <213> Artificial Sequence
<220>
   <223> primer
<400> 438
   acatttcatg gggctccact aacag 25
<210> 439
   <211> 23
   <212> DNA
   <213> Artificial Sequence
<220>
   <223> primer
<400> 439
   gctgcctgcg tcccaaagaa cag 23
<210> 440
   <211> 27
   <212> DNA
   <213> Artificial Sequence
<220>
   <223> primer
<400> 440
   ctacagagac acaacccatt gtttatg 27
<210> 441
   <211> 27
   <212> DNA
   <213> Artificial Sequence
<220>
   <223> primer
<400> 441
   gtgggaacgt gaaacatctg atacaag 27
<210> 442
   <211> 24
   <212> DNA
   <213> Artificial Sequence
<220>
   <223> primer
<400> 442
   ggaagattgc ccgagagcaa aaag 24
<210> 443
   <211> 27
   <212> DNA
   <213> Artificial Sequence
<220>
   <223> primer
<400> 443
   tgcatattag tggacagcac ttagtag 27
<210> 444
   <211> 23
   <212> DNA
   <213> Artificial Sequence
<220>
   <223> primer
<400> 444
   gcagcccaag cttcccatca cag 23
<210> 445
   <211> 27
   <212> DNA
   <213> Artificial Sequence
<220>
   <223> primer
<400> 445
   gaccttccac caatattcct gaaaatg 27
<210> 446
   <211> 27
   <212> DNA
   <213> Artificial Sequence
<220>
   <223> primer
<400> 446
   ttggcttaac agatgatcag gtttcag 27
<210> 447
   <211> 27
   <212> DNA
   <213> Artificial Sequence
<220>
   <223> primer
<400> 447
   ctcagactca agcaggtcag attgaag 27
<210> 448
   <211> 27
   <212> DNA
   <213> Artificial Sequence
<220>
   <223> primer
<400> 448
   ctcaacagta tggtattcag tattcag 27
<210> 449
   <211> 23
   <212> DNA
   <213> Artificial Sequence
<220>
   <223> primer
<400> 449
   atatgccctg cgtccaagcc caa 23
<210> 450
   <211> 23
   <212> DNA
   <213> Artificial Sequence
<220>
   <223> primer
<400> 450
   agcccactgc ggaagagggc agc 23
<210> 451
   <211> 30
   <212> DNA
   <213> Artificial Sequence
<220>
   <223> primer
<400> 451
   ctgcatcagg agatatgcaa acatatcaga 30
<210> 452
   <211> 22
   <212> DNA
   <213> Artificial Sequence
<220>
   <223> primer
<400> 452
   ttgccattgc cccaaatgga gc 22
<210> 453
   <211> 30
   <212> DNA
   <213> Artificial Sequence
<220>
   <223> primer
<400> 453
   aaaaattttg aaagacttat cttctgaaga 30
<210> 454
   <211> 30
   <212> DNA
   <213> Artificial Sequence
<220>
   <223> primer
<400> 454
   gtatcatctt tatcagaaag tgaggagtcc 30
<210> 455
   <211> 23
   <212> DNA
   <213> Artificial Sequence
<220>
   <223> primer
<400> 455
   ttgccattac ccagggagga gca 23
<210> 456
   <211> 27
   <212> DNA
   <213> Artificial Sequence
<220>
   <223> primer
<400> 456
   ctgcctctgg agacgtacaa acatacc 27
<210> 457
   <211> 28
   <212> DNA
   <213> Artificial Sequence
<220>
   <223> primer
<400> 457
   atgtgcagca cattaagagg agagacat 28
<210> 458
   <211> 23
   <212> DNA
   <213> Artificial Sequence
<220>
   <223> primer
<400> 458
   gtgcagaccc atctggagaa ccc 23
<210> 459
   <211> 29
   <212> DNA
   <213> Artificial Sequence
<220>
   <223> primer
<400> 459
   attgatgatg tcattgatga gatcatcag 29
<210> 460
   <211> 24
   <212> DNA
   <213> Artificial Sequence
<220>
   <223> primer
<400> 460
   ctgcctgtgt cagggaatct gctt 24
<210> 461
   <211> 26
   <212> DNA
   <213> Artificial Sequence
<220>
   <223> primer
<400> 461
   ggaaaactac agccaccaca cacaag 26
<210> 462
   <211> 28
   <212> DNA
   <213> Artificial Sequence
<220>
   <223> primer
<400> 462
   acacctttca tgaactcaaa tctgatgg 28
<210> 463
   <211> 29
   <212> DNA
   <213> Artificial Sequence
<220>
   <223> primer
<400> 463
   ggtcatactg catcagaacc atgaagaag 29
<210> 464
   <211> 24
   <212> DNA
   <213> Artificial Sequence
<220>
   <223> primer
<400> 464
   ccatgcccat tgggagaata gcag 24
<210> 465
   <211> 23
   <212> DNA
   <213> Artificial Sequence
<220>
   <223> primer
<400> 465
   agcaggagca ggagcgggag cgg 23
<210> 466
   <211> 23
   <212> DNA
   <213> Artificial Sequence
<220>
   <223> primer
<400> 466
   cgccggagtt gcataaggga gat 23
<210> 467
   <211> 24
   <212> DNA
   <213> Artificial Sequence
<220>
   <223> primer
<400> 467
   ggttccatga tgggaagtga catg 24
<210> 468
   <211> 28
   <212> DNA
   <213> Artificial Sequence
<220>
   <223> primer
<400> 468
   tggccaatgt gatctggaac ttattaat 28
<210> 469
   <211> 23
   <212> DNA
   <213> Artificial Sequence
<220>
   <223> primer
<400> 469
   tcaagggaac cttccctgat gcg 23
<210> 470
   <211> 27
   <212> DNA
   <213> Artificial Sequence
<220>
   <223> primer
<400> 470
   cagtgatctg gcctcagaca actactg 27
<210> 471
   <211> 29
   <212> DNA
   <213> Artificial Sequence
<220>
   <223> primer
<400> 471
   gcataagctg gaagtcacac cagtagtag 29
<210> 472
   <211> 27
   <212> DNA
   <213> Artificial Sequence
<220>
   <223> primer
<400> 472
   aggttgaaat tgggtcttca aaaccag 27
<210> 473
   <211> 23
   <212> DNA
   <213> Artificial Sequence
<220>
   <223> primer
<400> 473
   tcaggatggg aaaattgcac cag 23
<210> 474
   <211> 35
   <212> DNA
   <213> Artificial Sequence
<220>
   <223> primer
<400> 474
   gagaatatat aaaaaactgg aggccaagat acttc 35
<210> 475
   <211> 30
   <212> DNA
   <213> Artificial Sequence
<220>
   <223> primer
<400> 475
   cctagtgaga gccttgctac tactgatgat 30
<210> 476
   <211> 30
   <212> DNA
   <213> Artificial Sequence
<220>
   <223> primer
<400> 476
   actgaatctc cagtgttagt gaatgactat 30
<210> 477
   <211> 30
   <212> DNA
   <213> Artificial Sequence
<220>
   <223> primer
<400> 477
   ctgcatcagg agatatgcaa acatatcaga 30
<210> 478
   <211> 22
   <212> DNA
   <213> Artificial Sequence
<220>
   <223> primer
<400> 478
   ttgccattgc cccaaatgga gc 22
<210> 479
   <211> 30
   <212> DNA
   <213> Artificial Sequence
<220>
   <223> primer
<400> 479
   aaaaattttg aaagacttat cttctgaaga 30
<210> 480
   <211> 30
   <212> DNA
   <213> Artificial Sequence
<220>
   <223> primer
<400> 480
   gtatcatctt tatcagaaag tgaggagtcc 30
<210> 481
   <211> 48
   <212> DNA
   <213> Artificial Sequence
<220>
   <223> primer
<400> 481
   gtgccagcaa gatccaatct agaacatttc atggggctcc actaacag 48
<210> 482
   <211> 46
   <212> DNA
   <213> Artificial Sequence
<220>
   <223> primer
<400> 482
   gtgccagcaa gatccaatct agagctgcct gcgtcccaaa gaacag 46
<210> 483
   <211> 50
   <212> DNA
   <213> Artificial Sequence
<220>
   <223> primer
<400> 483
   gtgccagcaa gatccaatct agactacaga gacacaaccc attgtttatg 50
<210> 484
   <211> 50
   <212> DNA
   <213> Artificial Sequence
<220>
   <223> primer
<400> 484
   gtgccagcaa gatccaatct agagtgggaa cgtgaaacat ctgatacaag 50
<210> 485
   <211> 47
   <212> DNA
   <213> Artificial Sequence
<220>
   <223> primer
<400> 485
   gtgccagcaa gatccaatct agaggaagat tgcccgagag caaaaag 47
<210> 486
   <211> 50
   <212> DNA
   <213> Artificial Sequence
<220>
   <223> primer
<400> 486
   gtgccagcaa gatccaatct agatgcatat tagtggacag cacttagtag 50
<210> 487
   <211> 46
   <212> DNA
   <213> Artificial Sequence
<220>
   <223> primer
<400> 487
   gtgccagcaa gatccaatct agagcagccc aagcttccca tcacag 46
<210> 488
   <211> 50
   <212> DNA
   <213> Artificial Sequence
<220>
   <223> primer
<400> 488
   gtgccagcaa gatccaatct agagaccttc caccaatatt cctgaaaatg 50
<210> 489
   <211> 50
   <212> DNA
   <213> Artificial Sequence
<220>
   <223> primer
<400> 489
   gtgccagcaa gatccaatct agattggctt aacagatgat caggtttcag 50
<210> 490
   <211> 50
   <212> DNA
   <213> Artificial Sequence
<220>
   <223> primer
<400> 490
   gtgccagcaa gatccaatct agactcagac tcaagcaggt cagattgaag 50
<210> 491
   <211> 50
   <212> DNA
   <213> Artificial Sequence
<220>
   <223> primer
<400> 491
   gtgccagcaa gatccaatct agactcaaca gtatggtatt cagtattcag 50
<210> 492
   <211> 42
   <212> DNA
   <213> Artificial Sequence
<220>
   <223> primer
<400> 492
   atatgccctg cgtccaagcc caatccaacc cttagggaac cc 42
<210> 493
   <211> 42
   <212> DNA
   <213> Artificial Sequence
<220>
   <223> primer
<400> 493
   agcccactgc ggaagagggc agctccaacc cttagggaac cc 42
<210> 494
   <211> 49
   <212> DNA
   <213> Artificial Sequence
<220>
   <223> primer
<400> 494
   ctgcatcagg agatatgcaa acatatcaga tccaaccctt agggaaccc 49
<210> 495
   <211> 41
   <212> DNA
   <213> Artificial Sequence
<220>
   <223> primer
<400> 495
   ttgccattgc cccaaatgga gctccaaccc ttagggaacc c 41
<210> 496
   <211> 49
   <212> DNA
   <213> Artificial Sequence
<220>
   <223> primer
<400> 496
   aaaaattttg aaagacttat cttctgaaga tccaaccctt agggaaccc 49
<210> 497
   <211> 49
   <212> DNA
   <213> Artificial Sequence
<220>
   <223> primer
<400> 497
   gtatcatctt tatcagaaag tgaggagtcc tccaaccctt agggaaccc 49
<210> 498
   <211> 42
   <212> DNA
   <213> Artificial Sequence
<220>
   <223> primer
<400> 498
   ttgccattac ccagggagga gcatccaacc cttagggaac cc 42
<210> 499
   <211> 46
   <212> DNA
   <213> Artificial Sequence
<220>
   <223> primer
<400> 499
   ctgcctctgg agacgtacaa acatacctcc aacccttagg gaaccc 46
<210> 500
   <211> 47
   <212> DNA
   <213> Artificial Sequence
<220>
   <223> primer
<400> 500
   atgtgcagca cattaagagg agagacattc caacccttag ggaaccc 47
<210> 501
   <211> 42
   <212> DNA
   <213> Artificial Sequence
<220>
   <223> primer
<400> 501
   gtgcagaccc atctggagaa ccctccaacc cttagggaac cc 42
<210> 502
   <211> 48
   <212> DNA
   <213> Artificial Sequence
<220>
   <223> primer
<400> 502
   attgatgatg tcattgatga gatcatcagt ccaaccctta gggaaccc 48
<210> 503
   <211> 43
   <212> DNA
   <213> Artificial Sequence
<220>
   <223> primer
<400> 503
   ctgcctgtgt cagggaatct gctttccaac ccttagggaa ccc 43
<210> 504
   <211> 49
   <212> DNA
   <213> Artificial Sequence
<220>
   <223> primer
<400> 504
   gtgccagcaa gatccaatct agaggaaaac tacagccacc acacacaag 49
<210> 505
   <211> 51
   <212> DNA
   <213> Artificial Sequence
<220>
   <223> primer
<400> 505
   gtgccagcaa gatccaatct agaacacctt tcatgaactc aaatctgatg g 51
<210> 506
   <211> 52
   <212> DNA
   <213> Artificial Sequence
<220>
   <223> primer
<400> 506
   gtgccagcaa gatccaatct agaggtcata ctgcatcaga accatgaaga ag 52
<210> 507
   <211> 47
   <212> DNA
   <213> Artificial Sequence
<220>
   <223> primer
<400> 507
   gtgccagcaa gatccaatct agaccatgcc cattgggaga atagcag 47
<210> 508
   <211> 46
   <212> DNA
   <213> Artificial Sequence
<220>
   <223> primer
<400> 508
   gtgccagcaa gatccaatct agaagcagga gcaggagcgg gagcgg 46
<210> 509
   <211> 46
   <212> DNA
   <213> Artificial Sequence
<220>
   <223> primer
<400> 509
   gtgccagcaa gatccaatct agacgccgga gttgcataag ggagat 46
<210> 510
   <211> 47
   <212> DNA
   <213> Artificial Sequence
<220>
   <223> primer
<400> 510
   gtgccagcaa gatccaatct agaggttcca tgatgggaag tgacatg 47
<210> 511
   <211> 51
   <212> DNA
   <213> Artificial Sequence
<220>
   <223> primer
<400> 511
   gtgccagcaa gatccaatct agatggccaa tgtgatctgg aacttattaa t 51
<210> 512
   <211> 46
   <212> DNA
   <213> Artificial Sequence
<220>
   <223> primer
<400> 512
   gtgccagcaa gatccaatct agatcaaggg aaccttccct gatgcg 46
<210> 513
   <211> 50
   <212> DNA
   <213> Artificial Sequence
<220>
   <223> primer
<400> 513
   gtgccagcaa gatccaatct agacagtgat ctggcctcag acaactactg 50
<210> 514
   <211> 48
   <212> DNA
   <213> Artificial Sequence
<220>
   <223> primer
<400> 514
   gcataagctg gaagtcacac cagtagtagt ccaaccctta gggaaccc 48
<210> 515
   <211> 50
   <212> DNA
   <213> Artificial Sequence
<220>
   <223> primer
<400> 515
   gtgccagcaa gatccaatct agaaggttga aattgggtct tcaaaaccag 50
<210> 516
   <211> 46
   <212> DNA
   <213> Artificial Sequence
<220>
   <223> primer
<400> 516
   gtgccagcaa gatccaatct agatcaggat gggaaaattg caccag 46
<210> 517
   <211> 54
   <212> DNA
   <213> Artificial Sequence
<220>
   <223> primer
<400> 517
   gagaatatat aaaaaactgg aggccaagat acttctccaa cccttaggga accc 54
<210> 518
   <211> 53
   <212> DNA
   <213> Artificial Sequence
<220>
   <223> primer
<400> 518
   gtgccagcaa gatccaatct agacctagtg agagccttgc tactactgat gat 53
<210> 519
   <211> 53
   <212> DNA
   <213> Artificial Sequence
<220>
   <223> primer
<400> 519
   gtgccagcaa gatccaatct agaactgaat ctccagtgtt agtgaatgac tat 53
<210> 520
   <211> 49
   <212> DNA
   <213> Artificial Sequence
<220>
   <223> primer
<400> 520
   ctgcatcagg agatatgcaa acatatcaga tccaaccctt agggaaccc 49
<210> 521
   <211> 41
   <212> DNA
   <213> Artificial Sequence
<220>
   <223> primer
<400> 521
   ttgccattgc cccaaatgga gctccaaccc ttagggaacc c 41
<210> 522
   <211> 49
   <212> DNA
   <213> Artificial Sequence
<220>
   <223> primer
<400> 522
   aaaaattttg aaagacttat cttctgaaga tccaaccctt agggaaccc 49
<210> 523
   <211> 49
   <212> DNA
   <213> Artificial Sequence
<220>
   <223> primer
<400> 523
   gtatcatctt tatcagaaag tgaggagtcc tccaaccctt agggaaccc 49
<210> 524
   <211> 26
   <212> DNA
   <213> Artificial Sequence
<220>
   <223> primer
<400> 524
   gttattccag gatctttgga gacccg 26
<210> 525
   <211> 28
   <212> DNA
   <213> Artificial Sequence
<220>
   <223> primer
<400> 525
   gaagccttat cagttgtgag tgaggacc 28
<210> 526
   <211> 24
   <212> DNA
   <213> Artificial Sequence
<220>
   <223> primer
<400> 526
   atttaccata tgagcccccc agga 24
<210> 527
   <211> 25
   <212> DNA
   <213> Artificial Sequence
<220>
   <223> primer
<400> 527
   ctgctcaacc atctccttcc acagt 25
<210> 528
   <211> 30
   <212> DNA
   <213> Artificial Sequence
<220>
   <223> primer
<400> 528
   atccttatca gattcttgga ccaacaagta 30
<210> 529
   <211> 22
   <212> DNA
   <213> Artificial Sequence
<220>
   <223> primer
<400> 529
   gcagtggcca gatccagctt tg 22
<210> 530
   <211> 22
   <212> DNA
   <213> Artificial Sequence
<220>
   <223> primer
<400> 530
   agcgccgcct ggagcgcggc ag 22
<210> 531
   <211> 27
   <212> DNA
   <213> Artificial Sequence
<220>
   <223> primer
<400> 531
   gataacagca agatggcttt gaactca 27
<210> 532
   <211> 23
   <212> DNA
   <213> Artificial Sequence
<220>
   <223> primer
<400> 532
   tgtaccgccg gaagcaccag gag 23
<210> 533
   <211> 23
   <212> DNA
   <213> Artificial Sequence
<220>
   <223> primer
<400> 533
   ggctggaaac atttccgacc ctg 23
<210> 534
   <211> 27
   <212> DNA
   <213> Artificial Sequence
<220>
   <223> primer
<400> 534
   tggaaaagac aattgatgac ctggaag 27
<210> 535
   <211> 27
   <212> DNA
   <213> Artificial Sequence
<220>
   <223> primer
<400> 535
   cagtgaaaaa atcagtctca agtaaag 27
<210> 536
   <211> 27
   <212> DNA
   <213> Artificial Sequence
<220>
   <223> primer
<400> 536
   agcataaaga tgtcatcatc aaccaag 27
<210> 537
   <211> 25
   <212> DNA
   <213> Artificial Sequence
<220>
   <223> primer
<400> 537
   cccacacctg ggaaaggacc taaag 25
<210> 538
   <211> 27
   <212> DNA
   <213> Artificial Sequence
<220>
   <223> primer
<400> 538
   gatctgaatc ctgaaagaga aatagag 27
<210> 539
   <211> 23
   <212> DNA
   <213> Artificial Sequence
<220>
   <223> primer
<400> 539
   gccataggaa cgcactcagg cag 23
<210> 540
   <211> 27
   <212> DNA
   <213> Artificial Sequence
<220>
   <223> primer
<400> 540
   agctctctgt gatgcgctac tcaatag 27
<210> 541
   <211> 27
   <212> DNA
   <213> Artificial Sequence
<220>
   <223> primer
<400> 541
   actcgggaga ctatgaaata ttgtact 27
<210> 542
   <211> 25
   <212> DNA
   <213> Artificial Sequence
<220>
   <223> primer
<400> 542
   ctggagtccc aaataaacca ggcat 25
<210> 543
   <211> 30
   <212> DNA
   <213> Artificial Sequence
<220>
   <223> primer
<400> 543
   atgatttttg gataccagaa acaagtttca 30
<210> 544
   <211> 30
   <212> DNA
   <213> Artificial Sequence
<220>
   <223> primer
<400> 544
   tctggcatag aagattaaag aatcaaaaaa 30
<210> 545
   <211> 24
   <212> DNA
   <213> Artificial Sequence
<220>
   <223> primer
<400> 545
   agctgtctgg ctctggagat ctgg 24
<210> 546
   <211> 23
   <212> DNA
   <213> Artificial Sequence
<220>
   <223> primer
<400> 546
   tgagagaacg gaggtcctgg cag 23
<210> 547
   <211> 27
   <212> DNA
   <213> Artificial Sequence
<220>
   <223> primer
<400> 547
   acataaccat tagcagagag gctcagg 27
<210> 548
   <211> 23
   <212> DNA
   <213> Artificial Sequence
<220>
   <223> primer
<400> 548
   gcccactgac gctccaccga aag 23
<210> 549
   <211> 26
   <212> DNA
   <213> Artificial Sequence
<220>
   <223> primer
<400> 549
   ggagaagaca aagaaggcag agagag 26
<210> 550
   <211> 27
   <212> DNA
   <213> Artificial Sequence
<220>
   <223> primer
<400> 550
   ttttcttacc acaacatgac agtagtg 27
<210> 551
   <211> 23
   <212> DNA
   <213> Artificial Sequence
<220>
   <223> primer
<400> 551
   atccactgtg cgacgagctg tgc 23
<210> 552
   <211> 24
   <212> DNA
   <213> Artificial Sequence
<220>
   <223> primer
<400> 552
   gaggatccaa agtgggaatt ccct 24
<210> 553
   <211> 27
   <212> DNA
   <213> Artificial Sequence
<220>
   <223> primer
<400> 553
   attgctgtgg gaaataatga tgtaaag 27
<210> 554
   <211> 27
   <212> DNA
   <213> Artificial Sequence
<220>
   <223> primer
<400> 554
   gcagcatgtc agcttcgtat ctctcaa 27
<210> 555
   <211> 27
   <212> DNA
   <213> Artificial Sequence
<220>
   <223> primer
<400> 555
   aagaactagt ccagcttcga gcacaag 27
<210> 556
   <211> 27
   <212> DNA
   <213> Artificial Sequence
<220>
   <223> primer
<400> 556
   caggacctgg ctacaagagt taaaaag 27
<210> 557
   <211> 27
   <212> DNA
   <213> Artificial Sequence
<220>
   <223> primer
<400> 557
   gaacagctca ctaaagtgca caaacag 27
<210> 558
   <211> 25
   <212> DNA
   <213> Artificial Sequence
<220>
   <223> primer
<400> 558
   agaagagggc attctgcaca gattg 25
<210> 559
   <211> 23
   <212> DNA
   <213> Artificial Sequence
<220>
   <223> primer
<400> 559
   tgcgcaaagc cagcgtgacc atc 23
<210> 560
   <211> 45
   <212> DNA
   <213> Artificial Sequence
<220>
   <223> primer
<400> 560
   gttattccag gatctttgga gacccgtcca acccttaggg aaccc 45
<210> 561
   <211> 47
   <212> DNA
   <213> Artificial Sequence
<220>
   <223> primer
<400> 561
   gaagccttat cagttgtgag tgaggacctc caacccttag ggaaccc 47
<210> 562
   <211> 43
   <212> DNA
   <213> Artificial Sequence
<220>
   <223> primer
<400> 562
   atttaccata tgagcccccc aggatccaac ccttagggaa ccc 43
<210> 563
   <211> 44
   <212> DNA
   <213> Artificial Sequence
<220>
   <223> primer
<400> 563
   ctgctcaacc atctccttcc acagttccaa cccttaggga accc 44
<210> 564
   <211> 49
   <212> DNA
   <213> Artificial Sequence
<220>
   <223> primer
<400> 564
   atccttatca gattcttgga ccaacaagta tccaaccctt agggaaccc 49
<210> 565
   <211> 41
   <212> DNA
   <213> Artificial Sequence
<220>
   <223> primer
<400> 565
   gcagtggcca gatccagctt tgtccaaccc ttagggaacc c 41
<210> 566
   <211> 45
   <212> DNA
   <213> Artificial Sequence
<220>
   <223> primer
<400> 566
   gtgccagcaa gatccaatct agaagcgccg cctggagcgc ggcag 45
<210> 567
   <211> 50
   <212> DNA
   <213> Artificial Sequence
<220>
   <223> primer
<400> 567
   gtgccagcaa gatccaatct agagataaca gcaagatggc tttgaactca 50
<210> 568
   <211> 42
   <212> DNA
   <213> Artificial Sequence
<220>
   <223> primer
<400> 568
   tgtaccgccg gaagcaccag gagtccaacc cttagggaac cc 42
<210> 569
   <211> 46
   <212> DNA
   <213> Artificial Sequence
<220>
   <223> primer
<400> 569
   gtgccagcaa gatccaatct agaggctgga aacatttccg accctg 46
<210> 570
   <211> 50
   <212> DNA
   <213> Artificial Sequence
<220>
   <223> primer
<400> 570
   gtgccagcaa gatccaatct agatggaaaa gacaattgat gacctggaag 50
<210> 571
   <211> 50
   <212> DNA
   <213> Artificial Sequence
<220>
   <223> primer
<400> 571
   gtgccagcaa gatccaatct agacagtgaa aaaatcagtc tcaagtaaag 50
<210> 572
   <211> 50
   <212> DNA
   <213> Artificial Sequence
<220>
   <223> primer
<400> 572
   gtgccagcaa gatccaatct agaagcataa agatgtcatc atcaaccaag 50
<210> 573
   <211> 48
   <212> DNA
   <213> Artificial Sequence
<220>
   <223> primer
<400> 573
   gtgccagcaa gatccaatct agacccacac ctgggaaagg acctaaag 48
<210> 574
   <211> 50
   <212> DNA
   <213> Artificial Sequence
<220>
   <223> primer
<400> 574
   gtgccagcaa gatccaatct agagatctga atcctgaaag agaaatagag 50
<210> 575
   <211> 46
   <212> DNA
   <213> Artificial Sequence
<220>
   <223> primer
<400> 575
   gtgccagcaa gatccaatct agagccatag gaacgcactc aggcag 46
<210> 576
   <211> 50
   <212> DNA
   <213> Artificial Sequence
<220>
   <223> primer
<400> 576
   gtgccagcaa gatccaatct agaagctctc tgtgatgcgc tactcaatag 50
<210> 577
   <211> 50
   <212> DNA
   <213> Artificial Sequence
<220>
   <223> primer
<400> 577
   gtgccagcaa gatccaatct agaactcggg agactatgaa atattgtact 50
<210> 578
   <211> 44
   <212> DNA
   <213> Artificial Sequence
<220>
   <223> primer
<400> 578
   ctggagtccc aaataaacca ggcattccaa cccttaggga accc 44
<210> 579
   <211> 49
   <212> DNA
   <213> Artificial Sequence
<220>
   <223> primer
<400> 579
   atgatttttg gataccagaa acaagtttca tccaaccctt agggaaccc 49
<210> 580
   <211> 49
   <212> DNA
   <213> Artificial Sequence
<220>
   <223> primer
<400> 580
   tctggcatag aagattaaag aatcaaaaaa tccaaccctt agggaaccc 49
<210> 581
   <211> 46
   <212> DNA
   <213> Artificial Sequence
<220>
   <223> primer
<400> 581
   gtgccagcaa gatccaatct agaagctgtc tggctctgga gatctg 46
<210> 582
   <211> 46
   <212> DNA
   <213> Artificial Sequence
<220>
   <223> primer
<400> 582
   gtgccagcaa gatccaatct agatgagaga acggaggtcc tggcag 46
<210> 583
   <211> 50
   <212> DNA
   <213> Artificial Sequence
<220>
   <223> primer
<400> 583
   gtgccagcaa gatccaatct agaacataac cattagcaga gaggctcagg 50
<210> 584
   <211> 46
   <212> DNA
   <213> Artificial Sequence
<220>
   <223> primer
<400> 584
   gtgccagcaa gatccaatct agagcccact gacgctccac cgaaag 46
<210> 585
   <211> 49
   <212> DNA
   <213> Artificial Sequence
<220>
   <223> primer
<400> 585
   gtgccagcaa gatccaatct agaggagaag acaaagaagg cagagagag 49
<210> 586
   <211> 50
   <212> DNA
   <213> Artificial Sequence
<220>
   <223> primer
<400> 586
   gtgccagcaa gatccaatct agattttctt accacaacat gacagtagtg 50
<210> 587
   <211> 42
   <212> DNA
   <213> Artificial Sequence
<220>
   <223> primer
<400> 587
   atccactgtg cgacgagctg tgctccaacc cttagggaac cc 42
<210> 588
   <211> 43
   <212> DNA
   <213> Artificial Sequence
<220>
   <223> primer
<400> 588
   gaggatccaa agtgggaatt cccttccaac ccttagggaa ccc 43
<210> 589
   <211> 50
   <212> DNA
   <213> Artificial Sequence
<220>
   <223> primer
<400> 589
   gtgccagcaa gatccaatct agaattgctg tgggaaataa tgatgtaaag 50
<210> 590
   <211> 50
   <212> DNA
   <213> Artificial Sequence
<220>
   <223> primer
<400> 590
   gtgccagcaa gatccaatct agagcagcat gtcagcttcg tatctctcaa 50
<210> 591
   <211> 50
   <212> DNA
   <213> Artificial Sequence
<220>
   <223> primer
<400> 591
   gtgccagcaa gatccaatct agaaagaact agtccagctt cgagcacaag 50
<210> 592
   <211> 50
   <212> DNA
   <213> Artificial Sequence
<220>
   <223> primer
<400> 592
   gtgccagcaa gatccaatct agacaggacc tggctacaag agttaaaaag 50
<210> 593
   <211> 50
   <212> DNA
   <213> Artificial Sequence
<220>
   <223> primer
<400> 593
   gtgccagcaa gatccaatct agagaacagc tcactaaagt gcacaaacag 50
<210> 594
   <211> 48
   <212> DNA
   <213> Artificial Sequence
<220>
   <223> primer
<400> 594
   gtgccagcaa gatccaatct agaagaagag ggcattctgc acagattg 48
<210> 595
   <211> 46
   <212> DNA
   <213> Artificial Sequence
<220>
   <223> primer
<400> 595
   gtgccagcaa gatccaatct agatgcgcaa agccagcgtg accatc 46
<210> 596
   <211> 21
   <212> DNA
   <213> Artificial Sequence
<220>
   <223> primer forward
<400> 596
   cgcaaatggg cggtaggcgt g 21
<210> 597
   <211> 19
   <212> DNA
   <213> Artificial Sequence
<220>
   <223> primer reverse
<400> 597
   cgccatccac gctgttttg 19
<210> 598
   <211> 21
   <212> DNA
   <213> Artificial Sequence
<220>
   <223> primer forward
<400> 598
   ggctaactag agaacccact g 21
<210> 599
   <211> 20
   <212> DNA
   <213> Artificial Sequence
<220>
   <223> primer reverse
<400> 599
   ggcaactaga aggcacagtc 20
<210> 600
   <211> 20
   <212> DNA
   <213> Artificial Sequence
<220>
   <223> primer forward
<400> 600
   agagctcgtt tagtgaaccg 20
<210> 601
   <211> 20
   <212> DNA
   <213> Artificial Sequence
<220>
   <223> primer reverse
<400> 601
   gtggtttgtc caaactcatc 20
<210> 602
   <211> 19
   <212> DNA
   <213> Artificial Sequence
<220>
   <223> primer forward
<400> 602
   gatcactctc ggcatggac 19
<210> 603
   <211> 23
   <212> DNA
   <213> Artificial Sequence
<220>
   <223> primer reverse
<400> 603
   cattttatgt ttcaggttca ggg 23
<210> 604
   <211> 18
   <212> DNA
   <213> Artificial Sequence
<220>
   <223> primer forward
<400> 604
   gtcgtaacaa ctccgccc 18
<210> 605
   <211> 19
   <212> DNA
   <213> Artificial Sequence
<220>
   <223> primer reverse
<400> 605
   gtccagctcg accaggatg 19
<210> 606
   <211> 19
   <212> DNA
   <213> Artificial Sequence
<220>
   <223> primer forward
<400> 606
   atagcatggc ctttgcagg 19
<210> 607
   <211> 19
   <212> DNA
   <213> Artificial Sequence
<220>
   <223> primer reverse
<400> 607
   gagctgcatg tgtcagagg 19
<210> 608
   <211> 22
   <212> DNA
   <213> Artificial Sequence
<220>
   <223> primer forward
<400> 608
   gtatcttatg gtactgtaac tg 22
<210> 609
   <211> 22
   <212> DNA
   <213> Artificial Sequence
<220>
   <223> primer reverse
<400> 609
   ctttatgttt ttggcgtctt cc 22
<210> 610
   <211> 20
   <212> DNA
   <213> Artificial Sequence
<220>
   <223> primer forward
<400> 610
   ggtctatata agcagagctg 20
<210> 611
   <211> 23
   <212> DNA
   <213> Artificial Sequence
<220>
   <223> primer reverse
<400> 611
   gtggtatggc tgattatgat cag 23
<210> 612
   <211> 26
   <212> DNA
   <213> Artificial Sequence
<220>
   <223> AF6 exon 2
<400> 612
   ctcatcactc catggaactc caaatc 26
<210> 613
   <211> 24
   <212> DNA
   <213> Artificial Sequence
<220>
   <223> MLL exon 9
<400> 613
   cttttctttt ggtttttgtt ttac 24
<210> 614
   <211> 23
   <212> DNA
   <213> Artificial Sequence
<220>
   <223> AF1Q exon 2
<400> 614
   ctcacagggt ccctcatagc ttc 23
<210> 615
   <211> 13
   <212> DNA
   <213> Artificial Sequence
<220>
   <223> MLL exon 10
<400> 615
   cttaaagtcc act 13
<210> 616
   <211> 23
   <212> DNA
   <213> Artificial Sequence
<220>
   <223> primer
<400> 616
   ctctacgtct cctccgagag ccg 23
<210> 617
   <211> 23
   <212> DNA
   <213> Artificial Sequence
<220>
   <223> primer
<400> 617
   gtctcctcct cggcttcctc ttc 23
<210> 618
   <211> 29
   <212> DNA
   <213> Artificial Sequence
<220>
   <223> primer
<400> 618
   ataatctcag tgataccttg aagaagctg 29
<210> 619
   <211> 28
   <212> DNA
   <213> Artificial Sequence
<220>
   <223> primer
<400> 619
   aaatgacatc agatgtacca tcactggg 28
<210> 620
   <211> 29
   <212> DNA
   <213> Artificial Sequence
<220>
   <223> primer
<400> 620
   tcgcttcatg gagatataag tagcctgaa 29
<210> 621
   <211> 27
   <212> DNA
   <213> Artificial Sequence
<220>
   <223> primer
<400> 621
   gaaattcggc gccttcatca gtatgtg 27
<210> 622
   <211> 30
   <212> DNA
   <213> Artificial Sequence
<220>
   <223> primer
<400> 622
   atcgtactga gaagcactcc acaatgccag 30
<210> 623
   <211> 33
   <212> DNA
   <213> Artificial Sequence
<220>
   <223> primer
<400> 623
   caggaacgaa tttcatatac acctccagag agc 33
<210> 624
   <211> 26
   <212> DNA
   <213> Artificial Sequence
<220>
   <223> primer
<400> 624
   gcttgcagcc aatttactgg agcagg 26
<210> 625
   <211> 33
   <212> DNA
   <213> Artificial Sequence
<220>
   <223> primer
<400> 625
   atcttagacg aattttacaa tgtgaagttc tgc 33
<210> 626
   <211> 24
   <212> DNA
   <213> Artificial Sequence
<220>
   <223> primer
<400> 626
   gtgtctgctg actccagtgc atcc 24
<210> 627
   <211> 28
   <212> DNA
   <213> Artificial Sequence
<220>
   <223> primer
<400> 627
   attgtcagta aacgggcagg tactcagt 28
<210> 628
   <211> 27
   <212> DNA
   <213> Artificial Sequence
<220>
   <223> primer
<400> 628
   gatgacagca tggaagagaa accacta 27
<210> 629
   <211> 23
   <212> DNA
   <213> Artificial Sequence
<220>
   <223> primer
<400> 629
   tgagtccatt cttgcacacc gag 23
<210> 630
   <211> 27
   <212> DNA
   <213> Artificial Sequence
<220>
   <223> primer
<400> 630
   attttggctc tctatttgac ttggagc 27
<210> 631
   <211> 24
   <212> DNA
   <213> Artificial Sequence
<220>
   <223> primer
<400> 631
   atgggaatga tgaacaaccc caat 24
<210> 632
   <211> 23
   <212> DNA
   <213> Artificial Sequence
<220>
   <223> primer
<400> 632
   ccttgccctt taaggtggtg gtg 23
<210> 633
   <211> 28
   <212> DNA
   <213> Artificial Sequence
<220>
   <223> primer
<400> 633
   ctgccttatg actcaagatg ggagtttc 28
<210> 634
   <211> 22
   <212> DNA
   <213> Artificial Sequence
<220>
   <223> primer
<400> 634
   gggccccgag aacctcgaaa tc 22
<210> 635
   <211> 22
   <212> DNA
   <213> Artificial Sequence
<220>
   <223> primer
<400> 635
   gggcgacctc ttccagaagc tg 22
<210> 636
   <211> 27
   <212> DNA
   <213> Artificial Sequence
<220>
   <223> primer
<400> 636
   atccaacgac caagaactct ctggaaa 27
<210> 637
   <211> 28
   <212> DNA
   <213> Artificial Sequence
<220>
   <223> primer
<400> 637
   gccagttatt tgcctcagta aaagacag 28
<210> 638
   <211> 30
   <212> DNA
   <213> Artificial Sequence
<220>
   <223> primer
<400> 638
   cagtatgagt acacggagct caagaaacag 30
<210> 639
   <211> 28
   <212> DNA
   <213> Artificial Sequence
<220>
   <223> primer
<400> 639
   cacaatgaaa cagatttgca aaaaggaa 28
<210> 640
   <211> 20
   <212> DNA
   <213> Artificial Sequence
<220>
   <223> primer
<400> 640
   ccgcctgcct gcgcacctgc 20
<210> 641
   <211> 22
   <212> DNA
   <213> Artificial Sequence
<220>
   <223> primer
<400> 641
   cgggcagaag gctggtgaca ag 22
<210> 642
   <211> 20
   <212> DNA
   <213> Artificial Sequence
<220>
   <223> primer
<400> 642
   aagggccaac cactggggaa 20
<210> 643
   <211> 30
   <212> DNA
   <213> Artificial Sequence
<220>
   <223> primer
<400> 643
   aaaacaagtg cacagacaac accaagtaag 30
<210> 644
   <211> 26
   <212> DNA
   <213> Artificial Sequence
<220>
   <223> primer
<400> 644
   gggcaaatga gaacagcaac atacag 26
<210> 645
   <211> 26
   <212> DNA
   <213> Artificial Sequence
<220>
   <223> primer
<400> 645
   gcacacctct caatgcagct ttacag 26
<210> 646
   <211> 29
   <212> DNA
   <213> Artificial Sequence
<220>
   <223> primer
<400> 646
   tgaggacaag ttctacagcc acaagaaaa 29
<210> 647
   <211> 24
   <212> DNA
   <213> Artificial Sequence
<220>
   <223> primer
<400> 647
   agaaccccaa cagcaaagaa ggct 24
<210> 648
   <211> 25
   <212> DNA
   <213> Artificial Sequence
<220>
   <223> primer
<400> 648
   cctaagatgc ccgacttcaa ctgct 25
<210> 649
   <211> 24
   <212> DNA
   <213> Artificial Sequence
<220>
   <223> primer
<400> 649
   aatgctggac ttccgtagtg acca 24
<210> 650
   <211> 30
   <212> DNA
   <213> Artificial Sequence
<220>
   <223> primer
<400> 650
   cagtgccagg aaagagaatt agagatcagt 30
<210> 651
   <211> 23
   <212> DNA
   <213> Artificial Sequence
<220>
   <223> primer
<400> 651
   caaagcccac gctgaagcga aag 23
<210> 652
   <211> 26
   <212> DNA
   <213> Artificial Sequence
<220>
   <223> primer
<400> 652
   agctaaaagg acagcaggtg ctacca 26
<210> 653
   <211> 28
   <212> DNA
   <213> Artificial Sequence
<220>
   <223> primer
<400> 653
   gttcagagac tgaaggatga agccagag 28
<210> 654
   <211> 29
   <212> DNA
   <213> Artificial Sequence
<220>
   <223> primer
<400> 654
   gatgatgatg aagaggatga tgatgaaga 29
<210> 655
   <211> 24
   <212> DNA
   <213> Artificial Sequence
<220>
   <223> primer
<400> 655
   cagcttccag tcacagcata ggct 24
<210> 656
   <211> 28
   <212> DNA
   <213> Artificial Sequence
<220>
   <223> primer
<400> 656
   gatgtcagca tacccagatc cacattag 28
<210> 657
   <211> 24
   <212> DNA
   <213> Artificial Sequence
<220>
   <223> primer
<400> 657
   accgcttgga atgctgcaac aatg 24
<210> 658
   <211> 27
   <212> DNA
   <213> Artificial Sequence
<220>
   <223> primer
<400> 658
   ccaacaggca tgataggata tggaatt 27
<210> 659
   <211> 25
   <212> DNA
   <213> Artificial Sequence
<220>
   <223> primer
<400> 659
   ctttggccct gtatcaggag cacag 25
<210> 660
   <211> 21
   <212> DNA
   <213> Artificial Sequence
<220>
   <223> primer
<400> 660
   ctgggccaga atggtgagga g 21
<210> 661
   <211> 23
   <212> DNA
   <213> Artificial Sequence
<220>
   <223> primer
<400> 661
   ggctggcgca cctgggcgtg cag 23
<210> 662
   <211> 25
   <212> DNA
   <213> Artificial Sequence
<220>
   <223> primer
<400> 662
   tgatgaaggg gaggaaggag aggaa 25
<210> 663
   <211> 28
   <212> DNA
   <213> Artificial Sequence
<220>
   <223> primer
<400> 663
   cgtgatcttt gatagatcca gggaagag 28
<210> 664
   <211> 27
   <212> DNA
   <213> Artificial Sequence
<220>
   <223> primer
<400> 664
   ttcttggctc aacaagccat aaaacag 27
<210> 665
   <211> 29
   <212> DNA
   <213> Artificial Sequence
<220>
   <223> primer
<400> 665
   ggtcttataa tcttccctct cttccggat 29
<210> 666
   <211> 25
   <212> DNA
   <213> Artificial Sequence
<220>
   <223> primer
<400> 666
   ccagcaacct tccatctctc atcag 25
<210> 667
   <211> 24
   <212> DNA
   <213> Artificial Sequence
<220>
   <223> primer
<400> 667
   ccgcagagca ctgtatcacg aaaa 24
<210> 668
   <211> 30
   <212> DNA
   <213> Artificial Sequence
<220>
   <223> primer
<400> 668
   attcctgttc ctactacagt tcctgttcct 30
<210> 669
   <211> 29
   <212> DNA
   <213> Artificial Sequence
<220>
   <223> primer
<400> 669
   accaagaggc tattcaagat ctctgcctg 29
<210> 670
   <211> 29
   <212> DNA
   <213> Artificial Sequence
<220>
   <223> primer
<400> 670
   accaagaggc tattcaagat ctctgtatg 29
<210> 671
   <211> 30
   <212> DNA
   <213> Artificial Sequence
<220>
   <223> primer
<400> 671
   agcatttggt tttaaattat ggagtatgtt 30
<210> 672
   <211> 26
   <212> DNA
   <213> Artificial Sequence
<220>
   <223> primer
<400> 672
   tctgtggaga cgagaatatt ctggtt 26
<210> 673
   <211> 27
   <212> DNA
   <213> Artificial Sequence
<220>
   <223> primer
<400> 673
   gatttgtgat tttggtctag ccagagt 27
<210> 674
   <211> 31
   <212> DNA
   <213> Artificial Sequence
<220>
   <223> primer
<400> 674
   catcaagaat gattctaatt atgtggttaa a 31
<210> 675
   <211> 42
   <212> DNA
   <213> Artificial Sequence
<220>
   <223> primer
<400> 675
   ctctacgtct cctccgagag ccgtccaacc cttagggaac cc 42
<210> 676
   <211> 42
   <212> DNA
   <213> Artificial Sequence
<220>
   <223> primer
<400> 676
   gtctcctcct cggcttcctc ttctccaacc cttagggaac cc 42
<210> 677
   <211> 48
   <212> DNA
   <213> Artificial Sequence
<220>
   <223> primer
<400> 677
   ataatctcag tgataccttg aagaagctgt ccaaccctta gggaaccc 48
<210> 678
   <211> 47
   <212> DNA
   <213> Artificial Sequence
<220>
   <223> primer
<400> 678
   aaatgacatc agatgtacca tcactgggtc caacccttag ggaaccc 47
<210> 679
   <211> 48
   <212> DNA
   <213> Artificial Sequence
<220>
   <223> primer
<400> 679
   tcgcttcatg gagatataag tagcctgaat ccaaccctta gggaaccc 48
<210> 680
   <211> 46
   <212> DNA
   <213> Artificial Sequence
<220>
   <223> primer
<400> 680
   gaaattcggc gccttcatca gtatgtgtcc aacccttagg gaaccc 46
<210> 681
   <211> 49
   <212> DNA
   <213> Artificial Sequence
<220>
   <223> primer
<400> 681
   atcgtactga gaagcactcc acaatgccag tccaaccctt agggaaccc 49
<210> 682
   <211> 52
   <212> DNA
   <213> Artificial Sequence
<220>
   <223> primer
<400> 682
   caggaacgaa tttcatatac acctccagag agctccaacc cttagggaac cc 52
<210> 683
   <211> 45
   <212> DNA
   <213> Artificial Sequence
<220>
   <223> primer
<400> 683
   gcttgcagcc aatttactgg agcaggtcca acccttaggg aaccc 45
<210> 684
   <211> 52
   <212> DNA
   <213> Artificial Sequence
<220>
   <223> primer
<400> 684
   atcttagacg aattttacaa tgtgaagttc tgctccaacc cttagggaac cc 52
<210> 685
   <211> 43
   <212> DNA
   <213> Artificial Sequence
<220>
   <223> primer
<400> 685
   gtgtctgctg actccagtgc atcctccaac ccttagggaa ccc 43
<210> 686
   <211> 47
   <212> DNA
   <213> Artificial Sequence
<220>
   <223> primer
<400> 686
   attgtcagta aacgggcagg tactcagttc caacccttag ggaaccc 47
<210> 687
   <211> 46
   <212> DNA
   <213> Artificial Sequence
<220>
   <223> primer
<400> 687
   gatgacagca tggaagagaa accactatcc aacccttagg gaaccc 46
<210> 688
   <211> 42
   <212> DNA
   <213> Artificial Sequence
<220>
   <223> primer
<400> 688
   tgagtccatt cttgcacacc gagtccaacc cttagggaac cc 42
<210> 689
   <211> 46
   <212> DNA
   <213> Artificial Sequence
<220>
   <223> primer
<400> 689
   attttggctc tctatttgac ttggagctcc aacccttagg gaaccc 46
<210> 690
   <211> 43
   <212> DNA
   <213> Artificial Sequence
<220>
   <223> primer
<400> 690
   atgggaatga tgaacaaccc caattccaac ccttagggaa ccc 43
<210> 691
   <211> 42
   <212> DNA
   <213> Artificial Sequence
<220>
   <223> primer
<400> 691
   ccttgccctt taaggtggtg gtgtccaacc cttagggaac cc 42
<210> 692
   <211> 47
   <212> DNA
   <213> Artificial Sequence
<220>
   <223> primer
<400> 692
   ctgccttatg actcaagatg ggagtttctc caacccttag ggaaccc 47
<210> 693
   <211> 42
   <212> DNA
   <213> Artificial Sequence
<220>
   <223> primer
<400> 693
   gtgccagcaa gatccaatct agagggcccc gagaacctcg aa 42
<210> 694
   <211> 45
   <212> DNA
   <213> Artificial Sequence
<220>
   <223> primer
<400> 694
   gtgccagcaa gatccaatct agagggcgac ctcttccaga agctg 45
<210> 695
   <211> 50
   <212> DNA
   <213> Artificial Sequence
<220>
   <223> primer
<400> 695
   gtgccagcaa gatccaatct agaatccaac gaccaagaac tctctggaaa 50
<210> 696
   <211> 48
   <212> DNA
   <213> Artificial Sequence
<220>
   <223> primer
<400> 696
   gtgccagcaa gatccaatct agagccagtt atttgcctca gtaaaaga 48
<210> 697
   <211> 53
   <212> DNA
   <213> Artificial Sequence
<220>
   <223> primer
<400> 697
   gtgccagcaa gatccaatct agacagtatg agtacacgga gctcaagaaa cag 53
<210> 698
   <211> 48
   <212> DNA
   <213> Artificial Sequence
<220>
   <223> primer
<400> 698
   gtgccagcaa gatccaatct agacacaatg aaacagattt gcaaaaag 48
<210> 699
   <211> 43
   <212> DNA
   <213> Artificial Sequence
<220>
   <223> primer
<400> 699
   gtgccagcaa gatccaatct agaccgcctg cctgcgcacc tgc 43
<210> 700
   <211> 45
   <212> DNA
   <213> Artificial Sequence
<220>
   <223> primer
<400> 700
   gtgccagcaa gatccaatct agacgggcag aaggctggtg acaag 45
<210> 701
   <211> 43
   <212> DNA
   <213> Artificial Sequence
<220>
   <223> primer
<400> 701
   gtgccagcaa gatccaatct agaaagggcc aaccactggg gaa 43
<210> 702
   <211> 53
   <212> DNA
   <213> Artificial Sequence
<220>
   <223> primer
<400> 702
   gtgccagcaa gatccaatct agaaaaacaa gtgcacagac aacaccaagt aag 53
<210> 703
   <211> 49
   <212> DNA
   <213> Artificial Sequence
<220>
   <223> primer
<400> 703
   gtgccagcaa gatccaatct agagggcaaa tgagaacagc aacatacag 49
<210> 704
   <211> 49
   <212> DNA
   <213> Artificial Sequence
<220>
   <223> primer
<400> 704
   gtgccagcaa gatccaatct agagcacacc tctcaatgca gctttacag 49
<210> 705
   <211> 52
   <212> DNA
   <213> Artificial Sequence
<220>
   <223> primer
<400> 705
   gtgccagcaa gatccaatct agatgaggac aagttctaca gccacaagaa aa 52
<210> 706
   <211> 44
   <212> DNA
   <213> Artificial Sequence
<220>
   <223> primer
<400> 706
   gtgccagcaa gatccaatct agaagaaccc caacagcaaa gaag 44
<210> 707
   <211> 45
   <212> DNA
   <213> Artificial Sequence
<220>
   <223> primer
<400> 707
   gtgccagcaa gatccaatct agacctaaga tgcccgactt caact 45
<210> 708
   <211> 47
   <212> DNA
   <213> Artificial Sequence
<220>
   <223> primer
<400> 708
   gtgccagcaa gatccaatct agaaatgctg gacttccgta gtgacca 47
<210> 709
   <211> 53
   <212> DNA
   <213> Artificial Sequence
<220>
   <223> primer
<400> 709
   gtgccagcaa gatccaatct agacagtgcc aggaaagaga attagagatc agt 53
<210> 710
   <211> 46
   <212> DNA
   <213> Artificial Sequence
<220>
   <223> primer
<400> 710
   gtgccagcaa gatccaatct agacaaagcc cacgctgaag cgaaag 46
<210> 711
   <211> 49
   <212> DNA
   <213> Artificial Sequence
<220>
   <223> primer
<400> 711
   gtgccagcaa gatccaatct agaagctaaa aggacagcag gtgctacca 49
<210> 712
   <211> 51
   <212> DNA
   <213> Artificial Sequence
<220>
   <223> primer
<400> 712
   gtgccagcaa gatccaatct agagttcaga gactgaagga tgaagccaga g 51
<210> 713
   <211> 52
   <212> DNA
   <213> Artificial Sequence
<220>
   <223> primer
<400> 713
   gtgccagcaa gatccaatct agagatgatg atgaagagga tgatgatgaa ga 52
<210> 714
   <211> 44
   <212> DNA
   <213> Artificial Sequence
<220>
   <223> primer
<400> 714
   gtgccagcaa gatccaatct agacagcttc cagtcacagc atag 44
<210> 715
   <211> 51
   <212> DNA
   <213> Artificial Sequence
<220>
   <223> primer
<400> 715
   gtgccagcaa gatccaatct agagatgtca gcatacccag atccacatta g 51
<210> 716
   <211> 47
   <212> DNA
   <213> Artificial Sequence
<220>
   <223> primer
<400> 716
   gtgccagcaa gatccaatct agaaccgctt ggaatgctgc aacaatg 47
<210> 717
   <211> 50
   <212> DNA
   <213> Artificial Sequence
<220>
   <223> primer
<400> 717
   gtgccagcaa gatccaatct agaccaacag gcatgatagg atatggaatt 50
<210> 718
   <211> 48
   <212> DNA
   <213> Artificial Sequence
<220>
   <223> primer
<400> 718
   gtgccagcaa gatccaatct agactttggc cctgtatcag gagcacag 48
<210> 719
   <211> 44
   <212> DNA
   <213> Artificial Sequence
<220>
   <223> primer
<400> 719
   gtgccagcaa gatccaatct agactgggcc agaatggtga ggag 44
<210> 720
   <211> 46
   <212> DNA
   <213> Artificial Sequence
<220>
   <223> primer
<400> 720
   gtgccagcaa gatccaatct agaggctggc gcacctgggc gtgcag 46
<210> 721
   <211> 45
   <212> DNA
   <213> Artificial Sequence
<220>
   <223> primer
<400> 721
   gtgccagcaa gatccaatct agatgatgaa ggggaggaag gagag 45
<210> 722
   <211> 51
   <212> DNA
   <213> Artificial Sequence
<220>
   <223> primer
<400> 722
   gtgccagcaa gatccaatct agacgtgatc tttgatagat ccagggaaga g 51
<210> 723
   <211> 50
   <212> DNA
   <213> Artificial Sequence
<220>
   <223> primer
<400> 723
   gtgccagcaa gatccaatct agattcttgg ctcaacaagc cataaaacag 50
<210> 724
   <211> 52
   <212> DNA
   <213> Artificial Sequence
<220>
   <223> primer
<400> 724
   gtgccagcaa gatccaatct agaggtctta taatcttccc tctcttccgg at 52
<210> 725
   <211> 48
   <212> DNA
   <213> Artificial Sequence
<220>
   <223> primer
<400> 725
   gtgccagcaa gatccaatct agaccagcaa ccttccatct ctcatcag 48
<210> 726
   <211> 47
   <212> DNA
   <213> Artificial Sequence
<220>
   <223> primer
<400> 726
   gtgccagcaa gatccaatct agaccgcaga gcactgtatc acgaaaa 47
<210> 727
   <211> 53
   <212> DNA
   <213> Artificial Sequence
<220>
   <223> primer
<400> 727
   gtgccagcaa gatccaatct agaattcctg ttcctactac agttcctgtt cct 53
<210> 728
   <211> 52
   <212> DNA
   <213> Artificial Sequence
<220>
   <223> primer
<400> 728
   gtgccagcaa gatccaatct agaaccaaga ggctattcaa gatctctgcc tg 52
<210> 729
   <211> 52
   <212> DNA
   <213> Artificial Sequence
<220>
   <223> primer
<400> 729
   gtgccagcaa gatccaatct agaaccaaga ggctattcaa gatctctgta tg 52
<210> 730
   <211> 53
   <212> DNA
   <213> Artificial Sequence
<220>
   <223> primer
<400> 730
   gtgccagcaa gatccaatct agaagcattt ggttttaaat tatggagtat gtt 53
<210> 731
   <211> 45
   <212> DNA
   <213> Artificial Sequence
<220>
   <223> primer
<400> 731
   tctgtggaga cgagaatatt ctggtttcca acccttaggg aaccc 45
<210> 732
   <211> 50
   <212> DNA
   <213> Artificial Sequence
<220>
   <223> primer
<400> 732
   gtgccagcaa gatccaatct agagatttgt gattttggtc tagccagagt 50
<210> 733
   <211> 50
   <212> DNA
   <213> Artificial Sequence
<220>
   <223> primer
<400> 733
   catcaagaat gattctaatt atgtggttaa atccaaccct tagggaaccc 50
<210> 734
   <211> 30
   <212> DNA
   <213> Artificial Sequence
<220>
   <223> primer
<400> 734
   ggagcggaca tggactacga ctcgtaccag 30
<210> 735
   <211> 30
   <212> DNA
   <213> Artificial Sequence
<220>
   <223> primer
<400> 735
   agaatgaaga aattgatgtt gtgacagtag 30
<210> 736
   <211> 26
   <212> DNA
   <213> Artificial Sequence
<220>
   <223> primer
<400> 736
   aggaaggcat caaccacgag tgtaag 26
<210> 737
   <211> 28
   <212> DNA
   <213> Artificial Sequence
<220>
   <223> primer
<400> 737
   cagctctctg taatgcgata ctcaccag 28
<210> 738
   <211> 30
   <212> DNA
   <213> Artificial Sequence
<220>
   <223> primer
<400> 738
   aagagaggct gtatctccat gccagagcag 30
<210> 739
   <211> 30
   <212> DNA
   <213> Artificial Sequence
<220>
   <223> primer
<400> 739
   acccaaaagc agaccttgga gaacagtcag 30
<210> 740
   <211> 30
   <212> DNA
   <213> Artificial Sequence
<220>
   <223> primer
<400> 740
   tctttgaact actgccggag cttctgccag 30
<210> 741
   <211> 24
   <212> DNA
   <213> Artificial Sequence
<220>
   <223> primer
<400> 741
   caccgaggac cagctcaatg acag 24
<210> 742
   <211> 49
   <212> DNA
   <213> Artificial Sequence
<220>
   <223> primer
<400> 742
   ggagcggaca tggactacga ctcgtaccag tccaaccctt agggaaccc 49
<210> 743
   <211> 49
   <212> DNA
   <213> Artificial Sequence
<220>
   <223> primer
<400> 743
   agaatgaaga aattgatgtt gtgacagtag tccaaccctt agggaaccc 49
<210> 744
   <211> 45
   <212> DNA
   <213> Artificial Sequence
<220>
   <223> primer
<400> 744
   aggaaggcat caaccacgag tgtaagtcca acccttaggg aaccc 45
<210> 745
   <211> 51
   <212> DNA
   <213> Artificial Sequence
<220>
   <223> primer
<400> 745
   gtgccagcaa gatccaatct agacagctct ctgtaatgcg atactcacca g 51
<210> 746
   <211> 53
   <212> DNA
   <213> Artificial Sequence
<220>
   <223> primer
<400> 746
   gtgccagcaa gatccaatct agaaagagag gctgtatctc catgccagag cag 53
<210> 747
   <211> 53
   <212> DNA
   <213> Artificial Sequence
<220>
   <223> primer
<400> 747
   gtgccagcaa gatccaatct agaacccaaa agcagacctt ggagaacagt cag 53
<210> 748
   <211> 53
   <212> DNA
   <213> Artificial Sequence
<220>
   <223> primer
<400> 748
   gtgccagcaa gatccaatct agatctttga actactgccg gagcttctgc cag 53
<210> 749
   <211> 47
   <212> DNA
   <213> Artificial Sequence
<220>
   <223> primer
<400> 749
   gtgccagcaa gatccaatct agacaccgag gaccagctca atgacag 47
<210> 750
   <211> 22
   <212> DNA
   <213> Artificial Sequence
<220>
   <223> primer
<400> 750
   catcccagtg actgcatccc tc 22
<210> 751
   <211> 20
   <212> DNA
   <213> Artificial Sequence
<220>
   <223> primer
<400> 751
   ggggacccca ttcccgagga 20
<210> 752
   <211> 27
   <212> DNA
   <213> Artificial Sequence
<220>
   <223> primer
<400> 752
   gctctccaca gatagagaac atccagc 27
<210> 753
   <211> 25
   <212> DNA
   <213> Artificial Sequence
<220>
   <223> primer
<400> 753
   ctgaacagat gggtaaggat ggcag 25
<210> 754
   <211> 23
   <212> DNA
   <213> Artificial Sequence
<220>
   <223> primer
<400> 754
   ggaccaacca cttcctaccc cag 23
<210> 755
   <211> 20
   <212> DNA
   <213> Artificial Sequence
<220>
   <223> primer
<400> 755
   gccccaggtg tacccaccac 20
<210> 756
   <211> 20
   <212> DNA
   <213> Artificial Sequence
<220>
   <223> primer
<400> 756
   gcctcacctg cagatgcccc 20
<210> 757
   <211> 25
   <212> DNA
   <213> Artificial Sequence
<220>
   <223> primer
<400> 757
   gcaacctcca agtcccagat catgt 25
<210> 758
   <211> 20
   <212> DNA
   <213> Artificial Sequence
<220>
   <223> primer
<400> 758
   ggagttcctg gtcggctccg 20
<210> 759
   <211> 27
   <212> DNA
   <213> Artificial Sequence
<220>
   <223> primer
<400> 759
   cgagttcaag caggcctata tcacctg 27
<210> 760
   <211> 20
   <212> DNA
   <213> Artificial Sequence
<220>
   <223> primer
<400> 760
   gtgggcctcc tgggcctcag 20
<210> 761
   <211> 23
   <212> DNA
   <213> Artificial Sequence
<220>
   <223> primer
<400> 761
   tccctggaat gaagggacac aga 23
<210> 762
   <211> 20
   <212> DNA
   <213> Artificial Sequence
<220>
   <223> primer
<400> 762
   atggcaaaac tggcccccct 20
<210> 763
   <211> 23
   <212> DNA
   <213> Artificial Sequence
<220>
   <223> primer
<400> 763
   tccctggacc taaaggtgct gct 23
<210> 764
   <211> 23
   <212> DNA
   <213> Artificial Sequence
<220>
   <223> primer
<400> 764
   aagcaggcaa acctggtgaa cag 23
<210> 765
   <211> 22
   <212> DNA
   <213> Artificial Sequence
<220>
   <223> primer
<400> 765
   tccagggcct aagggtgaca ga 22
<210> 766
   <211> 20
   <212> DNA
   <213> Artificial Sequence
<220>
   <223> primer
<400> 766
   ctggtgcccc tggtgacaag 20
<210> 767
   <211> 20
   <212> DNA
   <213> Artificial Sequence
<220>
   <223> primer
<400> 767
   ctggaccccc tggccccatt 20
<210> 768
   <211> 20
   <212> DNA
   <213> Artificial Sequence
<220>
   <223> primer
<400> 768
   agggtccccc tggccctcct 20
<210> 769
   <211> 20
   <212> DNA
   <213> Artificial Sequence
<220>
   <223> primer
<400> 769
   ctggtcctgc tggtccccga 20
<210> 770
   <211> 20
   <212> DNA
   <213> Artificial Sequence
<220>
   <223> primer
<400> 770
   ctggcgagcc tggagcttca 20
<210> 771
   <211> 22
   <212> DNA
   <213> Artificial Sequence
<220>
   <223> primer
<400> 771
   tgtcctcctt gaagggctcc ag 22
<210> 772
   <211> 27
   <212> DNA
   <213> Artificial Sequence
<220>
   <223> primer
<400> 772
   cctccactga agaagctgaa acaagag 27
<210> 773
   <211> 25
   <212> DNA
   <213> Artificial Sequence
<220>
   <223> primer
<400> 773
   gagagtctgg atggacattt gcagg 25
<210> 774
   <211> 20
   <212> DNA
   <213> Artificial Sequence
<220>
   <223> primer
<400> 774
   tgcgaagcca cctctcgcag 20
<210> 775
   <211> 41
   <212> DNA
   <213> Artificial Sequence
<220>
   <223> primer
<400> 775
   catcccagtg actgcatccc tctccaaccc ttagggaacc c 41
<210> 776
   <211> 39
   <212> DNA
   <213> Artificial Sequence
<220>
   <223> primer
<400> 776
   ggggacccca ttcccgagga tccaaccctt agggaaccc 39
<210> 777
   <211> 46
   <212> DNA
   <213> Artificial Sequence
<220>
   <223> primer
<400> 777
   gctctccaca gatagagaac atccagctcc aacccttagg gaaccc 46
<210> 778
   <211> 44
   <212> DNA
   <213> Artificial Sequence
<220>
   <223> primer
<400> 778
   ctgaacagat gggtaaggat ggcagtccaa cccttaggga accc 44
<210> 779
   <211> 42
   <212> DNA
   <213> Artificial Sequence
<220>
   <223> primer
<400> 779
   ggaccaacca cttcctaccc cagtccaacc cttagggaac cc 42
<210> 780
   <211> 39
   <212> DNA
   <213> Artificial Sequence
<220>
   <223> primer
<400> 780
   gccccaggtg tacccaccac tccaaccctt agggaaccc 39
<210> 781
   <211> 39
   <212> DNA
   <213> Artificial Sequence
<220>
   <223> primer
<400> 781
   gcctcacctg cagatgcccc tccaaccctt agggaaccc 39
<210> 782
   <211> 44
   <212> DNA
   <213> Artificial Sequence
<220>
   <223> primer
<400> 782
   gcaacctcca agtcccagat catgttccaa cccttaggga accc 44
<210> 783
   <211> 39
   <212> DNA
   <213> Artificial Sequence
<220>
   <223> primer
<400> 783
   ggagttcctg gtcggctccg tccaaccctt agggaaccc 39
<210> 784
   <211> 50
   <212> DNA
   <213> Artificial Sequence
<220>
   <223> primer
<400> 784
   gtgccagcaa gatccaatct agacgagttc aagcaggcct atatcacctg 50
<210> 785
   <211> 43
   <212> DNA
   <213> Artificial Sequence
<220>
   <223> primer
<400> 785
   gtgccagcaa gatccaatct agagtgggcc tcctgggcct cag 43
<210> 786
   <211> 46
   <212> DNA
   <213> Artificial Sequence
<220>
   <223> primer
<400> 786
   gtgccagcaa gatccaatct agatccctgg aatgaaggga cacaga 46
<210> 787
   <211> 43
   <212> DNA
   <213> Artificial Sequence
<220>
   <223> primer
<400> 787
   gtgccagcaa gatccaatct agaatggcaa aactggcccc cct 43
<210> 788
   <211> 46
   <212> DNA
   <213> Artificial Sequence
<220>
   <223> primer
<400> 788
   gtgccagcaa gatccaatct agatccctgg acctaaaggt gctgct 46
<210> 789
   <211> 46
   <212> DNA
   <213> Artificial Sequence
<220>
   <223> primer
<400> 789
   gtgccagcaa gatccaatct agaaagcagg caaacctggt gaacag 46
<210> 790
   <211> 45
   <212> DNA
   <213> Artificial Sequence
<220>
   <223> primer
<400> 790
   gtgccagcaa gatccaatct agatccaggg cctaagggtg acaga 45
<210> 791
   <211> 43
   <212> DNA
   <213> Artificial Sequence
<220>
   <223> primer
<400> 791
   gtgccagcaa gatccaatct agactggtgc ccctggtgac aag 43
<210> 792
   <211> 43
   <212> DNA
   <213> Artificial Sequence
<220>
   <223> primer
<400> 792
   gtgccagcaa gatccaatct agactggacc ccctggcccc att 43
<210> 793
   <211> 43
   <212> DNA
   <213> Artificial Sequence
<220>
   <223> primer
<400> 793
   gtgccagcaa gatccaatct agaagggtcc ccctggccct cct 43
<210> 794
   <211> 43
   <212> DNA
   <213> Artificial Sequence
<220>
   <223> primer
<400> 794
   gtgccagcaa gatccaatct agactggtcc tgctggtccc cga 43
<210> 795
   <211> 43
   <212> DNA
   <213> Artificial Sequence
<220>
   <223> primer
<400> 795
   gtgccagcaa gatccaatct agactggcga gcctggagct tca 43
<210> 796
   <211> 45
   <212> DNA
   <213> Artificial Sequence
<220>
   <223> primer
<400> 796
   gtgccagcaa gatccaatct agatgtcctc cttgaagggc tccag 45
<210> 797
   <211> 50
   <212> DNA
   <213> Artificial Sequence
<220>
   <223> primer
<400> 797
   gtgccagcaa gatccaatct agacctccac tgaagaagct gaaacaagag 50
<210> 798
   <211> 48
   <212> DNA
   <213> Artificial Sequence
<220>
   <223> primer
<400> 798
   gtgccagcaa gatccaatct agagagagtc tggatggaca tttgcagg 48
<210> 799
   <211> 43
   <212> DNA
   <213> Artificial Sequence
<220>
   <223> primer
<400> 799
   gtgccagcaa gatccaatct agatgcgaag ccacctctcg cag 43

## Description

La présente invention concerne une méthode de diagnostic d'un cancer associé à la formation de gènes de fusion chez un sujet, choisi parmi une leucémie, un sarcome ou un carcinome, ladite méthode comprenant une étape de RT-MLPA sur un échantillon biologique obtenu à partir dudit sujet à l'aide au moins des couples de sondes choisi parmi :
- les sondes SEQ ID NO : 1 à 25, 30, 31 et 113 à 120 pour le diagnostic d'une leucémie, et/ou,
- les sondes SEQ ID NO : 374 à 405 pour le diagnostic d'un sarcome, et/ou
- les sondes SEQ ID NO : 524 à 559 pour le diagnostic d'un carcinome,
chacune des sondes étant fusionnée, à au moins une extrémité, avec une séquence d'amorçage ; ledit couple de sondes étant constitué de deux sondes s'hybridant côte à côte pendant l'étape de RT-MLPA.

De préférence, la présente invention concerne une méthode de diagnostic d'un cancer chez un sujet, comprenant une étape de RT-MLPA réalisée sur un échantillon biologique obtenu à partir dudit sujet, notamment dans le but de rechercher des transcrits de fusion, à l'aide des sondes SEQ ID NO : 1 à 25, 30, 31 et 113 à 120, et/ou à l'aide des sondes SEQ ID NO : 374 à 405, et/ou à l'aide d'au moins les sondes SEQ ID NO : 524 à 559, chacune des sondes étant fusionnée, à au moins une extrémité, avec une séquence d'amorçage. La présente invention concerne également un kit comprenant au moins les sondes SEQ ID NO : 1 à 25, 30, 31 et 113 à 120, et/ou au moins les sondes SEQ ID NO : 374 à 405, et/ou au moins les sondes SEQ ID NO : 524 à 559, chacune des sondes étant fusionnée, à au moins une extrémité, avec une séquence d'amorçage.

Les cancers sont dus à une accumulation d'anomalies génétiques par les cellules tumorales. Parmi ces anomalies figurent de nombreux réarrangements chromosomiques (translocations, délétions et inversions) qui entraînent la formation de gènes de fusion. Ces gènes de fusions sont souvent associés à des formes particulières de tumeur, et leur mise en évidence peut contribuer de façon significative à poser le diagnostic (The impact of translocations and gene fusions on cancer causation. Mitelman F, Johansson B, Mertens F., Nat Rev Cancer. 2007 Apr;7(4):233-45.). Ils sont aussi souvent utilisés comme marqueurs moléculaires pour contrôler l'efficacité des traitements et suivre l'évolution de la maladie, comme par exemple dans les leucémies aiguës (Standardized RT-PCR analysis of fusion gene transcripts from chromosome aberrations in acute leukemia for detection of minimal residual disease. Report of the BIOMED-1 Concerted Action: investigation of minimal residual disease in acute leukemia. van Dongen JJ, Macintyre EA, Gabert JA, Delabesse E, Rossi V, Saglio G, Gottardi E, Rambaldi A, Dotti G, Griesinger F, Parreira A, Gameiro P, Diáz MG, Malec M, Langerak AW, San Miguel JF, Biondi A. Leukemia. 1999 Dec;13(12):1901-28.).

Aujourd'hui, les deux principales techniques qui permettent de rechercher ces gènes de fusion sont la cytogénétique et la RT-PCR.

La cytogénétique consiste à établir le caryotype des cellules cancéreuses pour rechercher d'éventuelles anomalies de nombre et/ou de structure des chromosomes. Elle possède l'avantage de fournir une vision globale de l'ensemble du génome. Elle est cependant relativement peu sensible, son efficacité dépendant fortement du pourcentage de cellules tumorales dans l'échantillon à analyser et de la possibilité d'obtenir des cultures cellulaires viables. Un autre de ses inconvénients est sa faible résolution qui ne permet pas de détecter certains réarrangements (en particulier des inversions et délétions de petite taille). Enfin, certaines tumeurs sont associées à une instabilité génomique majeure qui masque les anomalies génétiques pathognomoniques. L'analyse des caryotypes est donc souvent difficile et ne peut être réalisée que par du personnel possédant une excellente expertise.

La seconde technique, la RT-PCR, est elle réalisée à partir de l'ARN extrait des cellules tumorales. Elle possède une excellente sensibilité, bien supérieure à la cytogénétique. Cette sensibilité en fait la technique de référence pour analyser des échantillons biologiques où le pourcentage de cellules tumorales est faible, ce qui permet de contrôler l'efficacité des traitements ou d'anticiper très précocement d'éventuelles rechutes. En revanche, sa limite principale est liée au fait qu'il est extrêmement difficile de multiplexer ce type d'analyses. Chaque translocation devant en général être recherchée par un test spécifique, seuls quelques fusions récurrentes parmi les très nombreuses qui sont aujourd'hui connues sont recherchées dans les laboratoires d'analyse de routine.

La demande internationale WO03/044486 décrit également une méthode de diagnostic d'un cancer comprenant la détection d'une translocation chromosomique, et nécessitant l'amplification préalable de la translocation à détecter.

Il existe donc aujourd'hui un besoin pour un test spécifique et sensible, qui soit également simple et rapide à mettre en œuvre, permettant de diagnostiquer avec précision un cancer, notamment une tumeur solide (sarcome ou carcinome) ou une leucémie.

De façon surprenante, les inventeurs ont réussi à mettre en œuvre un test permettant de diagnostiquer un cancer spécifique, notamment une tumeur solide (par exemple sarcome ou carcinome) ou une leucémie. Ce test permet de rechercher simultanément un très grand nombre de réarrangements chromosomiques dans de nombreuses formes de cancer, et ce de façon économique et rapide (le test peut être réalisé en une journée). En outre, sa simplicité de mise en œuvre permet à une personne maîtrisant les techniques classiques de biologie moléculaire de réaliser l'ensemble des étapes sans formation spécifique, en utilisant des équipements déjà disponibles dans la plupart des laboratoires de diagnostic de routine.

L'invention se rapporte donc à un test moléculaire comprenant notamment une étape de RT-MLPA (Reverse Transcriptase Ligation-Dependent Probe Amplification), réalisée en mode multiplex. Le mode multiplex permet un gain de temps, car il est plus rapide que plusieurs monoplex, et est économiquement avantageux. Il permet également de rechercher simultanément un nombre beaucoup plus élevé d'anomalies que les techniques actuellement disponibles.

La présente invention se rapporte donc à une méthode de diagnostic d'un cancer associé à la formation de gènes de fusion chez un sujet, choisi parmi une leucémie, un sarcome ou un carcinome, ladite méthode comprenant une étape de RT-MLPA sur un échantillon biologique obtenu à partir dudit sujet, à l'aide au moins des couples de sondes choisi parmi :
- les sondes SEQ ID NO : 1 à 25, 30, 31 et 113 à 120 pour le diagnostic d'une leucémie, et/ou,
- les sondes SEQ ID NO : 374 à 405 pour le diagnostic d'un sarcome, et/ou
- les sondes SEQ ID NO : 524 à 559 pour le diagnostic d'un carcinome,
chacune des sondes étant fusionnée, à au moins une extrémité, avec une séquence d'amorçage. Ledit couple de sondes est constitué de deux sondes s'hybridant côte à côte pendant l'étape de RT-MLPA.

La présente invention se rapporte à une méthode de diagnostic d'un cancer associé à la formation de gènes de fusion chez un sujet, comprenant une étape de RT-MLPA réalisée sur un échantillon biologique obtenu à partir dudit sujet à l'aide des sondes SEQ ID NO : 1 à 25, 30, 31 et 113 à 120, et/ou à l'aide des sondes SEQ ID NO : 374 à 405, et/ou au moins les sondes SEQ ID NO : 524 à 559, chacune des sondes étant fusionnée, à au moins une extrémité, avec une séquence d'amorçage.

La présente invention se rapporte également à un kit comprenant au moins les sondes SEQ ID NO : 1 à 25, 30, 31 et 113 à 120, et/ou les sondes SEQ ID NO : 374 à 405, et/ou au moins les sondes SEQ ID NO : 524 à 559, de préférence comprenant en outre les sondes SEQ ID NO : 26 à 29, 66 à 112 et 121 à 219, et/ou les sondes SEQ ID NO : 438 à 480, et/ou les sondes SEQ ID NO : 616 à 674, et/ou les sondes SEQ ID NO : 734 à 741, et/ou les sondes SEQ ID NO : 750 à 774, chacune des sondes étant fusionnée, à au moins une extrémité, avec une séquence d'amorçage. De préférence, le kit comprend au moins les sondes SEQ ID NO : 35 à 59, 64, 65 et 267 à 274, et/ou les sondes SEQ ID NO : 406 à 437, et/ou les sondes SEQ ID NO : 560 à 595, de préférence il comprend en outre les sondes SEQ ID NO : 60 à 63, 220 à 266 et 275 à 373, et/ou les sondes SEQ ID NO :675 à 733, et/ou les sondes SEQ ID NO : 775 à 799, et/ou les sondes SEQ ID NO : 742 à 749.

Par "**MLPA**", on entend Multiplex Ligation-Dependent Probe Amplification, qui permet l'amplification simultanée de plusieurs cibles d'intérêt contiguës l'une de l'autre, en utilisant une ou plusieurs sondes spécifiques. Cette technique est très avantageuse, dans le cadre de la présente invention, pour déterminer la présence de translocations, qui sont fréquentes dans les tumeurs malignes.

Par "**RT-MLPA**", on entend Multiplex Ligation-Dependent Probe Amplification précédée d'une Transcription Inverse (Reverse transcription, RT), qui permet, dans le cadre de la présente invention, de partir de l'ARN d'un sujet pour amplifier et caractériser les gènes de fusion d'intérêt.

Par **"sujet",** on entend un individu sain ou susceptible d'être atteint d'un cancer ou en quête de dépistage, de diagnostic ou de suivi.

Par "**échantillon biologique",** on entend un échantillon contenant de la matière biologique. Plus préférentiellement, on entend tout échantillon contenant de l'ARN. Cet échantillon peut provenir d'un prélèvement biologique effectué chez un être vivant (patient humain, animal, végétal). Préférentiellement, les échantillons biologiques selon l'invention sont choisis parmi le sang total, la moelle osseuse et une biopsie obtenus à partir d'un sujet, notamment humain.

Par **"sensibilité",** on entend la proportion de tests positifs chez les sujets atteints de cancers et porteurs des anomalies recherchées.

Par **"spécificité",** on entend la proportion de tests négatifs chez les sujets non atteints de cancers et non porteurs des anomalies recherchées.

Par « **cancer** », on entend une maladie caractérisée par une prolifération cellulaire anormalement importante au sein d'un tissu normal de l'organisme, de telle manière que la survie de ce dernier est menacée. Selon l'invention, le cancer est de préférence choisi parmi les leucémies, les sarcomes et les carcinomes. Les leucémies sont des cancers des cellules de la moelle osseuse, des systèmes lymphoïdes et myéloïdes. Les sarcomes sont des tumeurs malignes développées aux dépens du tissu conjonctif commun extra-squelettique comme le tissu adipeux, le tissu musculaire, les vaisseaux et le système nerveux périphérique. Les carcinomes sont des tumeurs malignes développées aux dépens d'un tissu épithélial.

Par « **sonde** », on entend une séquence d'acide nucléique de longueur comprise entre 15 et 40 nucléotides, de préférence comprise entre 20 et 25 nucléotides, et complémentaire d'une séquence d'ADNc issue d'un ARN du sujet (endogène). Elle est donc capable de s'hybrider avec ladite séquence d'ADNc issue d'un ARN du sujet.

Par « **séquence d'amorçage** », on entend une séquence d'acide nucléique de longueur comprise entre 15 et 30 nucléotides, de préférence comprise entre 20 et 25 nucléotides, et non complémentaire des séquences d'ADNc issues d'ARN du sujet. Elle n'est donc pas complémentaire de l'ADNc correspondant à l'ARN endogène. Elle ne peut donc pas s'hybrider avec lesdites séquences d'ADNc. De préférence, la séquence d'amorçage est choisie parmi les séquences SEQ ID NO : 33 et SEQ ID NO :34.

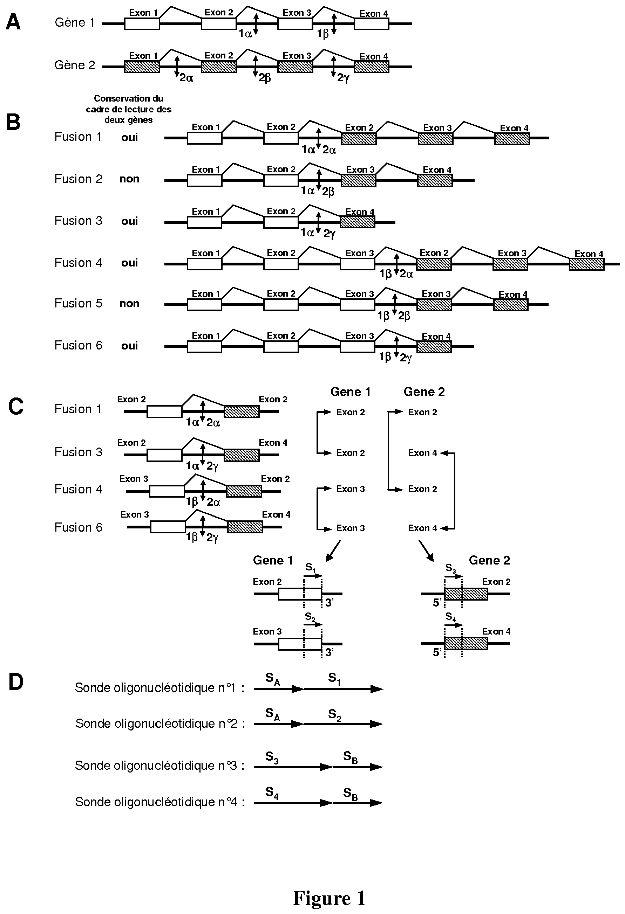

Les inventeurs ont identifié des sondes spécifiques pour chaque type de translocation observé dans certains cancers. Cette identification repose sur l'analyse de la structure intron-exon des gènes impliqués dans les translocations, comme cela est montré en figure 1. En particulier, les points de cassures susceptibles de conduire à l'expression de protéines chimériques fonctionnelles sont recherchés (figure 1B). A partir de ces résultats, des séquences d'ADN de 25 à 40 paires de bases sont définies, correspondant précisément aux extrémités 5' et 3' des exons des deux gènes juxtaposés après épissage des transcrits hybrides (figure 1C). Un ensemble de sondes est ensuite défini de la façon suivante : une séquence d'amorçage (S_{A} sur la figure 1) d'une vingtaine de paire de bases, est ajoutée en 5' de toutes les sondes complémentaires des exons des gènes formant la partie 5' des transcrits de fusion. Une deuxième séquence d'amorçage (S_{B} sur la figure 1), également d'une vingtaine de paire de bases mais différente de S_{A}, est ajoutée aux extrémités 3' de toutes les sondes complémentaires des exons des gènes formant la partie 3' des transcrits de fusion (figure 1D). Ces sondes sont ensuite regroupées dans un mélange, et contiennent tous les éléments nécessaires à la détection d'un ou de plusieurs transcrits de fusion, produits par une ou plusieurs translocations.

Les sondes utilisées dans l'invention sont donc capables de s'hybrider soit avec les derniers nucléotides du dernier exon en 5' de la translocation, soit avec les premiers nucléotides du premier exon en 3' de la translocation. De préférence, les sondes utilisées dans l'invention, capables de s'hybrider avec les premiers nucléotides du premier exon en 3' de la translocation, sont phosphorylées en 5' avant leur utilisation.

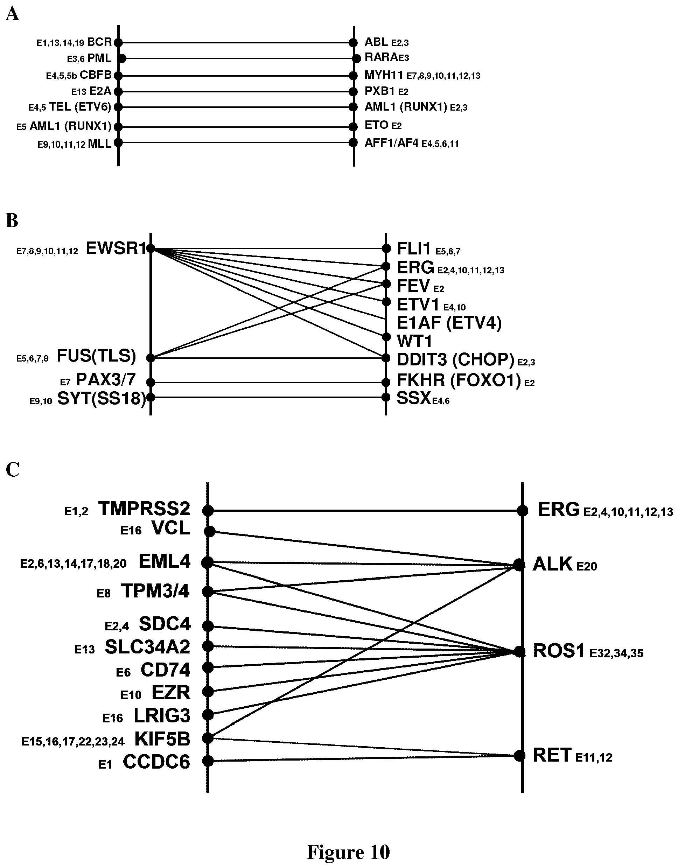

Les différentes translocations identifiées selon la présente invention sont illustrées dans les figures 10 à 12.

Les sondes selon l'invention sont les séquences SEQ ID NO : 1 à 25, 30, 31 et 113 à 120, éventuellement combinées avec les sondes SEQ ID NO : 26 à 29, 66 à 112 et 121 à 219 et/ou avec les sondes SEQ ID NO : 616 à 674.

Les sondes selon l'invention sont les séquences SEQ ID NO: 374 à 405, éventuellement combinées avec les sondes SEQ ID NO : 438 à 480 et/ou au moins avec les sondes SEQ ID NO : 750 à 774.

Les sondes selon l'invention sont les séquences SEQ ID NO: 524 à 559, éventuellement combinées avec les sondes SEQ ID NO : 438 à 480 et/ou au moins avec les sondes SEQ ID NO : 374 à 405 et/ou avec les sondes SEQ ID NO : 734 à 741.

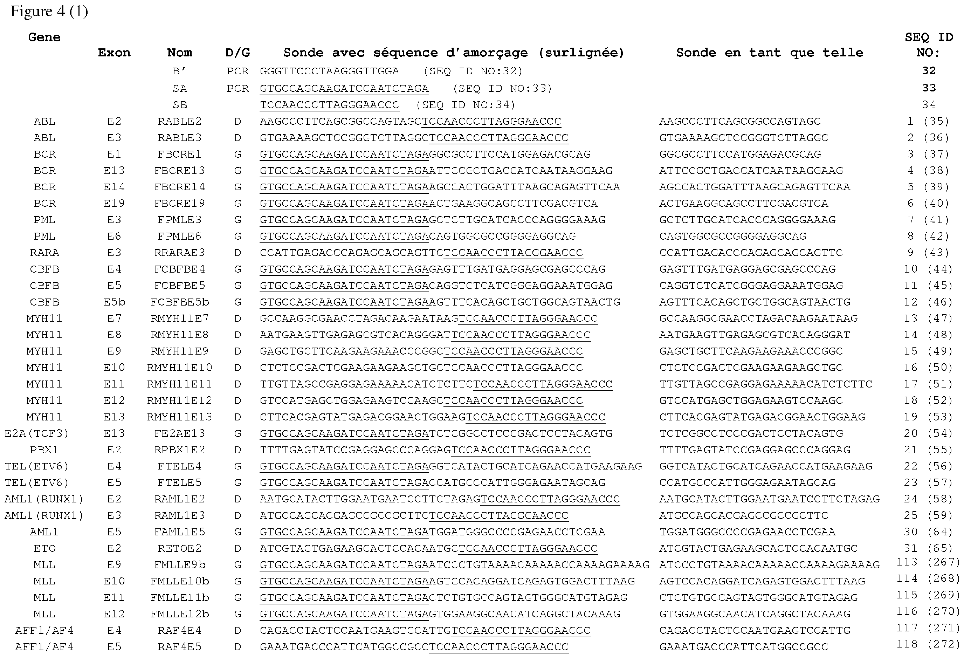

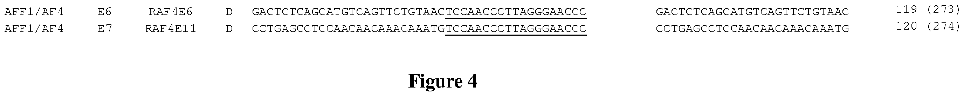

Les figures 4, 5, et 7 à 9 détaillent le nom de chaque sonde, leur position (i.e. si elles sont en 5' (G) ou en 3' (D) de la jonction anormale), leur structure (présence ou non d'une séquence d'amorçage, et caractéristiques de cette séquence), ainsi que le gène et l'exon auxquels chaque sonde s'hybride.

De préférence, chacune des sondes SEQ ID NO : 3 à SEQ ID NO : 8 ; SEQ ID NO : 10 à SEQ ID NO : 12 ; SEQ ID NO : 20, SEQ ID NO : 22, SEQ ID NO : 23, SEQ ID NO : 26 et SEQ ID NO : 30 est fusionnée en 5' avec une séquence d'amorçage, et
chacune des sondes SEQ ID NO : 1, SEQ ID NO : 2, SEQ ID NO : 9, SEQ ID NO : 13 à SEQ ID NO : 19 ; SEQ ID NO : 21, SEQ ID NO : 24, SEQ ID NO : 25, SEQ ID NO : 27 à SEQ ID NO : 29 ; et SEQ ID NO : 31 est fusionnée en 3' avec une séquence d'amorçage différente.

De préférence, les sondes fusionnées aux séquences d'amorçage utilisables selon l'invention sont les séquences SEQ ID NO : 35 à 59, 64, 65 et 267 à 274, éventuellement combinées aux sondes SEQ ID NO : 60 à 63, 220 à 266 et 275 à 373 et/ou aux sondes SEQ ID NO : 675 à 733.

De préférence, les sondes fusionnées aux séquences d'amorçage utilisables selon l'invention sont les séquences SEQ ID NO : 406 à 437, éventuellement combinées aux sondes SEQ ID NO : 481 à 523 et/ou aux sondes SEQ ID NO : 775 à 799.

De préférence, les sondes fusionnées aux séquences d'amorçage utilisables selon l'invention sont les séquences SEQ ID NO : 560 à 595, éventuellement combinées aux sondes SEQ ID NO : 481 à 523 et/ou aux sondes SEQ ID NO : 742 à 749.

La méthode de diagnostic selon l'invention s'effectue avec au moins les couples de sondes choisi parmi :
- les sondes SEQ ID NO : 1 à 25, 30, 31 et 113 à 120 pour le diagnostic d'une leucémie,
- les sondes SEQ ID NO : 374 à 405 pour le diagnostic d'un sarcome, et
- les sondes SEQ ID NO : 524 à 559 pour le diagnostic d'un carcinome,
chacune des sondes étant fusionnée, à au moins une extrémité, avec une séquence d'amorçage.

Le couple de sondes peut être choisi parmi les sondes de séquence spécifique décrites ci-dessus, et comme cela est explicité en figures 4, 7 et 9. Par « couple de sondes », on entend un ensemble de deux sondes, l'une étant située en 5' (« G » sur les figures 4-5, et 7 à 9) de la translocation ou mutation génique, l'autre étant située en 3' (« D » sur les figures 4-5, et 7 à 9) de la translocation ou mutation génique.

La méthode de diagnostic selon l'invention concerne le diagnostic de leucémies, et comprend une étape de RT-MLPA réalisée sur un échantillon biologique obtenu à partir dudit sujet à l'aide des sondes SEQ ID NO : 1 à 25, 30, 31 et 113 à 120, chacune des sondes étant fusionnée, à au moins une extrémité, avec une séquence d'amorçage.

Les leucémies sont de préférence choisies parmi les leucémies aiguës lymphoblastiques B, les leucémies aiguës lymphoblastiques T, les leucémies aiguës myéloblastiques, les leucémies myéloïdes chroniques, les lymphomes et les myélomes.

De préférence, dans ce cas, la méthode de diagnostic selon l'invention utilise également les sondes SEQ ID NO : 26 à 29, 66 à 112 et 121 à 219, et/ou les sondes SEQ ID NO : 616 à 674, pour l'étape de RT-MLPA, chacune des sondes étant fusionnée, à au moins une extrémité, avec une séquence d'amorçage. Ainsi, dans ce cas, un mélange des sondes SEQ ID NO : 1 à 25, 30, 31 et 113 à 120, et SEQ ID NO 26 à 29, 66 à 112 et 121 à 219 et/ou SEQ ID NO : 616 à 674 est utilisé, chacune des sondes étant fusionnée, à au moins une extrémité, avec une séquence d'amorçage.

De préférence, l'invention se rapporte à un kit de diagnostic des leucémies comprenant les sondes SEQ ID NO : 1 à 25, 30, 31 et 113 à 120, et 26 à 29, 66 à 112 et 121 à 219, et/ou SEQ ID NO : 616 à 674, chacune des sondes étant fusionnée, à au moins une extrémité, avec une séquence d'amorçage. De préférence, ledit kit comprend les sondes SEQ ID NO : 35 à 59, 64, 65 et 267 à 274, et de préférence également les sondes SEQ ID NO : 60 à 63, 220 à 266 et 275 à 373, et/ou de préférence également les sondes SEQ ID NO : 675 à 733.

La méthode de diagnostic selon l'invention concerne le diagnostic des sarcomes, et comprend une étape de RT-MLPA dans un échantillon biologique obtenu à partir dudit sujet à l'aide des sondes SEQ ID NO : 374 à 405, chacune des sondes étant fusionnée, à au moins une extrémité, avec une séquence d'amorçage.

Les sarcomes sont de préférence choisis parmi les sarcomes d'Ewing, les rhabdomyosarcomes, les tumeurs desmoplastiques à cellules rondes, les synovialosarcomes et les liposarcomes mixoïdes.

De préférence, dans ce cas, la méthode de diagnostic selon l'invention utilise également les sondes SEQ ID NO : 438 à 480 et/ou les sondes SEQ ID NO : 750 à 774, pour l'étape de RT-MLPA, chacune des sondes étant fusionnée, à au moins une extrémité, avec une séquence d'amorçage. Ainsi, dans ce cas, un mélange des sondes SEQ ID NO : 374 à 405, et 438 à 480 et/ou 750 à 774, est utilisé, chacune des sondes étant fusionnée, à au moins une extrémité, avec une séquence d'amorçage.

De préférence, l'invention se rapporte à un kit de diagnostic des sarcomes comprenant les sondes SEQ ID NO : 374 à 405, chacune des sondes étant fusionnée, à au moins une extrémité, avec une séquence d'amorçage. De préférence, ledit kit comprend les sondes SEQ ID NO : 406 à 437, et de préférence également les sondes SEQ ID NO : 481 à 523, et/ou de préférence également les sondes SEQ ID NO : 775 à 799.

La méthode de diagnostic selon l'invention concerne le diagnostic des carcinomes, et comprend une étape de RT-MLPA dans un échantillon biologique obtenu à partir dudit sujet à l'aide des sondes SEQ ID NO : 524 à 559, chacune des sondes étant fusionnée, à au moins une extrémité, avec une séquence d'amorçage.

Les carcinomes sont de préférence choisis parmi les carcinomes bronchopulmonaires non à petites cellules, les adénocarcinomes prostatiques, les carcinomes du rein, de la thyroïde et du sein.

De préférence, dans ce cas, la méthode de diagnostic selon l'invention utilise également les sondes SEQ ID NO : 438 à 480 et/ou les sondes SEQ ID NO : 734 à 741, pour l'étape de RT-MLPA, chacune des sondes étant fusionnée, à au moins une extrémité, avec une séquence d'amorçage. Ainsi, dans ce cas, un mélange des sondes SEQ ID NO : 524 à 559, et 438 à 480 et/ou 734 à 741, est utilisé, chacune des sondes étant fusionnée, à au moins une extrémité, avec une séquence d'amorçage.

De préférence, l'invention se rapporte à un kit de diagnostic des carcinomes comprenant les sondes SEQ ID NO : 524 à 559, chacune des sondes étant fusionnée, à au moins une extrémité, avec une séquence d'amorçage. De préférence, ledit kit comprend les sondes SEQ ID NO : 560 à 595, et de préférence également les sondes SEQ ID NO : 481 à 523, et/ou de préférence également les sondes SEQ ID NO : 742 à 749.

Préférentiellement la méthode de l'invention utilise un échantillon biologique choisi parmi le sang total, la moelle osseuse et une biopsie obtenus à partir du sujet.

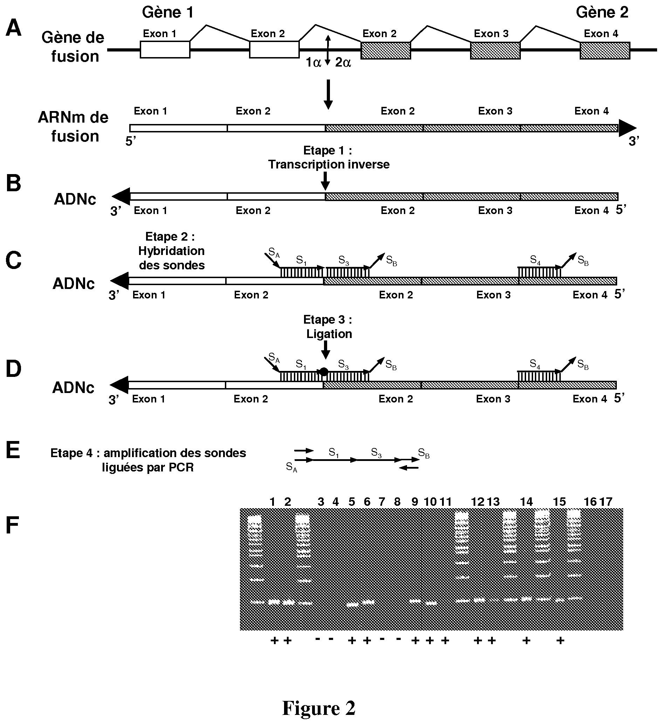

L'étape de RT-MLPA est dérivée de la MLPA (Multiplex Ligation-Dépendent Probe Amplification), décrite notamment dans le brevet US 6,955,901. Elle permet la détection et le dosage simultané d'un grand nombre de séquences oligonucléotidiques différentes. Le principe est le suivant (voir figure 2) : l'ARN extrait du tissu tumoral est d'abord converti en ADN complémentaire (ADNc) par transcription inverse. Cet ADNc est ensuite incubé avec le mélange de sondes adéquates, chacune pouvant alors s'hybrider sur les séquences des exons auxquels elles correspondent. Si un des transcrits de fusion recherché est présent dans l'échantillon, deux sondes viennent se fixer côte à côte sur l'ADNc correspondant. Une réaction de ligation est alors réalisée à l'aide d'une enzyme à activité ADN ligase, qui établit une liaison covalente entre les deux sondes contiguës. Une réaction de PCR (Polymérase Chain Reaction) est ensuite réalisée, en utilisant des amorces correspondant aux séquences d'amorçage (S_{A} et S_{B} sur la figure 2), qui permet d'amplifier spécifiquement les deux sondes liguées. L'obtention d'un produit d'amplification après l'étape de RT-MLPA indique que l'une des translocations recherchée est présente dans l'échantillon analysé.

De préférence, l'étape de RT-MLPA utilisée dans la méthode selon l'invention comprend au moins les étapes suivantes :
a) extraction de l'ARN de l'échantillon biologique du sujet ;
b) conversion de l'ARN extrait en a) en ADNc par transcription inverse ;
c) incubation de l'ADNc obtenu en b) avec au moins un couple de sondes choisi parmi :
   - les sondes SEQ ID NO : 1 à 25, 30, 31 et 113 à 120 pour le diagnostic d'une leucémie, et/ou
   - les sondes SEQ ID NO : 374 à 405 pour le diagnostic d'un sarcome, et/ou
   - les sondes SEQ ID NO : 524 à 559 pour le diagnostic d'un carcinome,
   chacune des sondes étant fusionnée, à au moins une extrémité, avec une séquence d'amorçage. De préférence, dans cette étape c), des mélanges de sondes tels que décrits précédemment sont utilisés.
   De préférence, dans cette étape c), l'ADNc obtenu en b) est incubé avec au moins les sondes SEQ ID NO : 1 à 25, 30, 31 et 113 à 120, et/ou avec au moins les sondes SEQ ID NO : 374 à 405, et/ou avec au moins les sondes SEQ ID NO : 524 à 559, chacune des sondes étant fusionnée, à au moins une extrémité, avec une séquence d'amorçage ;
d) addition d'une ADN ligase dans le mélange obtenu en c), afin d'établir une liaison covalente entre deux sondes contiguës ;
e) amplification par PCR des sondes contiguës liées de manière covalente obtenues en d).

Typiquement, l'extraction d'ARN de l'échantillon biologique selon l'étape a) s'effectue selon les techniques classiques, bien connues de l'homme du métier. Par exemple, cette extraction peut être effectuée par lyse cellulaire des cellules issues de l'échantillon biologique. Cette lyse peut être de nature chimique, physique ou thermique. Cette lyse cellulaire est généralement suivie d'une étape de purification permettant de séparer et concentrer les acides nucléiques d'autres débris cellulaires. Pour la mise en œuvre de l'étape a), les kits commerciaux de type QIAGEN et Zymo Research, ou encore ceux commercialisés par Invitrogen, peuvent être utilisés. Bien entendu, les techniques pertinentes diffèrent en fonction de la nature de l'échantillon biologique testé. Les connaissances de l'homme du métier lui permettent aisément d'adapter ces étapes de lyse et de purification audit échantillon biologique testé.

De préférence, l'ARN extrait à l'étape a) est alors converti par transcription inverse en ADNc ; c'est l'étape b) (voir figure 2B). Cette étape b) peut être effectuée à l'aide de toute technique de transcription inverse connue de l'art antérieur. Elle peut notamment se faire à l'aide de transcriptase inverse commercialisée par Qiagen, Promega ou Ambion, selon les conditions classiques d'utilisation, ou encore à l'aide de M-MLV Reverse Transcriptase de chez Invitrogen.

De préférence, l'ADNc obtenu à l'étape b) est ensuite incubé avec au moins les sondes SEQ ID NO : 1 à 25, 30, 31 et 113 à 120, et/ou avec au moins les sondes SEQ ID NO : 374 à 405, et/ou avec au moins les sondes SEQ ID NO : 524 à 559, chacune des sondes étant fusionnée, à au moins une extrémité, avec une séquence d'amorçage. C'est l'étape c) d'hybridation des sondes (voir figure 2C). En effet, les sondes, qui sont complémentaires d'une portion d'ADNc, vont venir s'hybrider avec cette portion si celle-ci est présente dans l'ADNc. Comme cela est montré dans la figure 2C, en raison de leur séquence, les sondes vont donc s'hybrider :
- soit avec la portion d'ADNc correspondant aux derniers nucléotides du dernier exon en 5' de la translocation. Il s'agit alors de sondes « F » ou « Forward », appelées aussi « G » ou « Gauche » ;
- soit avec la portion d'ADNc correspondant aux premiers nucléotides du premier exon en 3' de la translocation. Il s'agit alors de sondes « R » ou « Reverse », appelées aussi « D » ou « Droite ».
A la fin de l'étape c), les sondes hybridées à l'ADNc sont contiguës, si et seulement si la translocation a eu lieu.
Cette étape c) est typiquement réalisée en incubant l'ADNc et le mélange de sondes à une température comprise entre 90°C et 100°C, pendant une durée de 1 à 5 minutes, puis en laissant incuber pendant une durée d'au moins 1h, de préférence 16h, à une température d'environ 60°C. Elle peut être réalisée à l'aide du kit commercial, vendu par la société MRC-Holland (SALSA MLPA Buffer).

A la fin de l'étape c), une ADN ligase est typiquement ajoutée pour lier de manière covalente uniquement les sondes contiguës ; c'est l'étape d) (voir figure 2D). L'ADN ligase est notamment la ligase 65, vendue par MRC-Holland, Amsterdam, Netherlands (SALSA Ligase-65). Cette étape d) est typiquement réalisée à l'aide des kits Lig-5a, Lig-10 ou Lig-50 de MRC-Holland, Amsterdam, Netherlands. Elle est typiquement réalisée en incubant l'ADN ligase et le mélange obtenu à l'étape c) à une température comprise entre 50°C et 60°C, pendant une durée de 10 à 20 minutes, puis pendant une durée de 2 à 10 minutes à une température comprise entre 95°C et 100°C. A la fin de l'étape d), chaque couple de sondes contiguës G et D est lié de manière covalente, et la séquence d'amorçage de chaque sonde est toujours présente en 5' et en 3'.

De préférence, la méthode comprend également une étape e) d'amplification par PCR des sondes contiguës liées de manière covalente obtenues en d) (voir figure 2E). Cette étape de PCR se fait à l'aide d'un couple d'amorces, l'une des amorces étant identique à la séquence d'amorçage en 5', l'autre amorce étant complémentaire de la séquence d'amorçage en 3'. De préférence, l'amplification par PCR de l'étape e) se fait à l'aide des amorces SEQ ID NO :32 et 33, de préférence l'une étant marquée à son extrémité 5' par une biotine, afin de permettre l'étape (f). La PCR est typiquement réalisée à l'aide de kits commerciaux, tels que les kits prêts à l'emploi vendus par Eurogentec (Red'y'Star Mix). Typiquement, la PCR se déroule en une première phase de dénaturation initiale à une température comprise entre 90°C et 100°C, typiquement d'environ 94°C, pendant un temps de 5 à 8 minutes ; puis une seconde phase d'amplification comprenant plusieurs cycles, typiquement 35 cycles, chaque cycle comprenant 30 secondes à 94°C, puis 30 secondes à 58°C, puis 30 secondes à 72°C ; et une dernière phase de retour à 72°C pendant 4 minutes environ. A la fin de la PCR, les amplicons sont conservés de préférence à 4°C.

Typiquement, les amorces utilisables dans l'étape e) de PCR sont les suivantes :

| **SEQ ID NO :** | **Amorce** |
|---|---|
| 596 | CMV Forward : CGC AAA TGG GCG GTA GGC GTG |
| 597 | CMV Reverse : CGC CAT CCA CGC TGT TTT G |
| 598 | pcDNA3 Forward : GGC TAA CTA GAG AAC CCA CTG |
| 599 | pcDNA3 Reverse : GGC AAC TAG AAG GCA CAG TC |
| 600 | pCEP Forward : AGA GCT CGT TTA GTG AAC CG |
| 601 | pCEP Reverse : GTG GTT TGT CCA AAC TCA TC |
| 602 | pEGFPC 1 Forward: GAT CAC TCT CGG CAT GGA C |
| 603 | pEGFPC 1 Reverse : CAT TTT ATG TTT CAG GTT CAG GG |
| 604 | pEGFPN 1 Forward : GTC GTA ACA ACT CCG CCC |
| 605 | pEGFPN 1 Reverse : GTC CAG CTC GAC CAG GAT G |
| 606 | pGex Forward : ATA GCA TGG CCT TTG CAG G |
| 607 | pGex Reverse : GAG CTG CAT GTG TCA GAG G |
| 608 | pGL Forward: GTA TCT TAT GGT ACT GTA ACT G |
| 609 | pGL Reverse : CTT TAT GTT TTT GGC GTC TTC C |
| 610 | pShuttleCMV Forward : GGT CTA TAT AAG CAG AGC TG |
| 611 | pShuttleCMV Reverse : GTG GTA TGG CTG ATT ATG ATC AG |
| 32 | Reverse : GGGTTCCCTAAGGGTTGGA (complémentaire de la séquence d'amorçage SEQ ID NO : 34) |
| 33 | Forward : GTGCCAGCAAGATCCAATCTAGA |

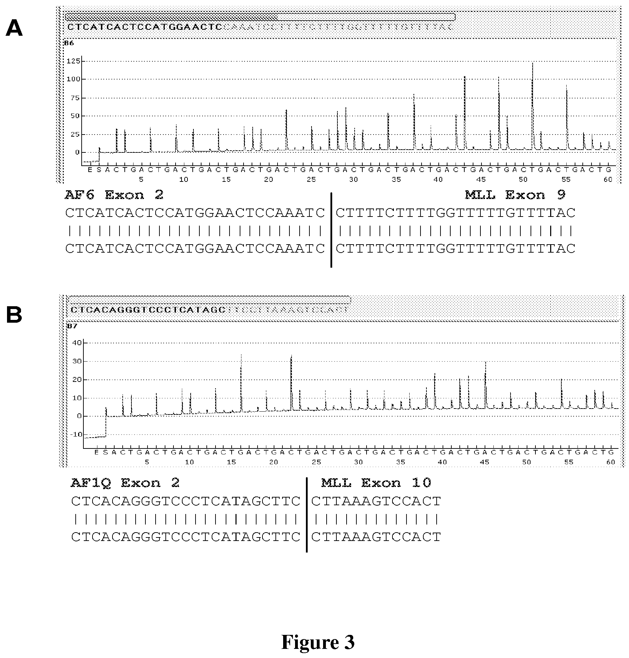

De préférence, la méthode selon l'invention comprend une étape f) d'analyse des résultats de la PCR de l'étape e), de préférence par pyroséquençage. A cette fin, on peut utiliser un pyroséquenceur, tel que le pyroséquenceur PyroMark Q24 vendu par la société Qiagen, en utilisant le kit commercial Pyromark Gold Q24 Reagent et l'un des oligonucléotides d'amorçage.
Cette étape d'analyse permet une lecture immédiate du résultat, et indique directement si l'échantillon du sujet est porteur d'une translocation spécifique identifiée ou non. Cela est notamment démontré dans la figure 3.
En effet, dans l'étape f), si, pour un échantillon biologique d'un sujet, une amplification par PCR est obtenue à l'étape e) suite à l'hybridation avec un couple de sondes, alors le sujet est porteur du cancer lié au réarrangement chromosomique correspondant au couple de sondes identifiées.

Les différentes séquences citées dans la présente invention sont résumées dans le tableau ci-dessous :

| **SEQ ID NO :** | **Identification** |
|---|---|
| 1-25, 30, 31 et 113 à 120 | Sondes en tant que telles, pour le diagnostic des leucémies notamment |
| 32 | Amorce reverse PCR |
| 33 | Amorce forward PCR et séquence d'amorçage en 5' |
| 34 | Séquence d'amorçage en 3' |
| 35-59, 64, 65 et 267-274 | Sondes SEQ ID NO : 1-25, 30, 31 et 113 à 120 fusionnées à la séquence d'amorçage |
| 26-29, 66-112 et 121-219 | Sondes en tant que telles, pour le diagnostic des leucémies notamment |
| 60-63, 220-266 et 275-373 | Sondes SEQ ID NO : 26-29, 66-112 et 121-219 fusionnées à la séquence d'amorçage |
| 374-405 | Sondes en tant que telles, pour le diagnostic des sarcomes notamment |
| 406-437 | Sondes SEQ ID NO : 374-405 fusionnées à la séquence d'amorçage |
| 438-480 | Sondes en tant que telles, notamment pour le diagnostic des tumeurs solides |
| 481-523 | Sondes SEQ ID NO : 438-480 fusionnées à la séquence d'amorçage |
| 524-559 | Sondes en tant que telles, pour le diagnostic des carcinomes notamment |
| 560-595 | Sondes SEQ ID NO : 524-559 fusionnées à la séquence d'amorçage |
| 596-611 | Amorces de PCR |
| 612-615 | Séquences de la figure 3A et B |
| 616-674 | Sondes en tant que telles, pour le diagnostic des leucémies notamment |
| 675-733 | Sondes SEQ ID NO : 616-674 fusionnées à la séquence d'amorçage |
| 734-741 | Sondes en tant que telles, pour le diagnostic des carcinomes notamment |
| 742-749 | Sondes SEQ ID NO : 734-741 fusionnées à la séquence d'amorçage |
| 750-774 | Sondes en tant que telles, pour le diagnostic des sarcomes notamment |
| 775-799 | Sondes SEQ ID NO : 750-774 fusionnées à la séquence d'amorçage |

**Figure 1** **: Schéma de définition des sondes.**
**Figure 2** **: Schéma de la RT-MLPA.**
   A) Exemple de translocation dans l'intron situé entre l'exon 2 du gène 1 et l'exon 2 du gène 2, aboutissant à un ARNm de fusion.
   B) Etape 1 : transcription inverse de cet ARNm de fusion, pour obtenir un ADNc.
   C) Etape 2 : incubation avec les sondes et hybridation de celles-ci avec les portions complémentaires d'ADNc. La sonde S1 est constituée d'une séquence complémentaire des derniers nucléotides de l'exon 2 du gène 1 d'ADNc, et la sonde S2 est constituée d'une séquence complémentaire des premiers nucléotides de l'exon 2 du gène 2 d'ADNc. La sonde S1 est fusionnée en 5' avec une séquence d'amorçage SA.
      La sonde S2 est fusionnée en 3' avec une séquence d'amorçage SB.
      De par la contiguïté entre les exons 2 du gène 1 et du gène 2, les sondes S1 et S2 se retrouvent côte à côte.
   D) Etape 3 : ligation par une ADN ligase. Les sondes côte à côte se retrouvent alors liées. S1 et S2 forment ainsi une séquence continue, avec SA et SB.
   E) Etape 4 : PCR : à l'aide d'amorces adéquates, les sondes liées sont amplifiées. En l'occurrence, les amorces utilisées sont la séquence SA, et la séquence complémentaire de SB (appelée B').
   F : analyse des résultats obtenus. La figure montre un exemple de résultats obtenus avec les sondes se liant aux translocations impliquant le gène MLL (caractéristique des leucémies) sur des échantillons provenant de 15 patients (pistes 1 à 15). Les pistes 16 et 17 sont des témoins négatifs.
      Il apparaît que 11 tumeurs sur les 15 sont porteuses d'un réarrangement du gène MLL.
**Figure 3** **: Identification des translocations dans les cas de leucémies.** Identification des partenaires du gène *MLL* des échantillons n°14 (A) et 15 (B) de la figure 2. Les produits d'amplifications de PCR ont été analysés par pyroséquençage.
   La séquence A correspond à une jonction entre le 9ème exon du gène *MLL* et le 2ème exon du gène *AF6*, indiquant que la tumeur portait une translocation t(6;11)(q27;q23).
   La séquence B correspond à une jonction entre le 10ème exon du gène *MLL* et le 2ème exon du gène *AF1Q*, indiquant que la tumeur portait une translocation t(1;11)(q21;q23).
   Ces deux translocations confirment le diagnostic de leucémie aigue, et sont associées à des formes de très mauvais pronostic.
**Figure 4** **: Tableau des sondes SEQ ID NO** : **1 à 25, 30, 31 et 113 à 120 (correspondant aux sondes fusionnées à la séquence d'amorçage SEQ ID NO** : **35 à 59, 64, 65 et 267 à 274).**
   Le tableau indique le gène et l'exon auxquels la sonde s'hybride. La colonne « SEQ ID NO : » indique le numéro de séquence de la sonde en tant que telle, et entre parenthèses le numéro de séquence de la sonde fusionnée à la séquence d'amorçage.
   Ces sondes sont utilisables pour le diagnostic des leucémies notamment.

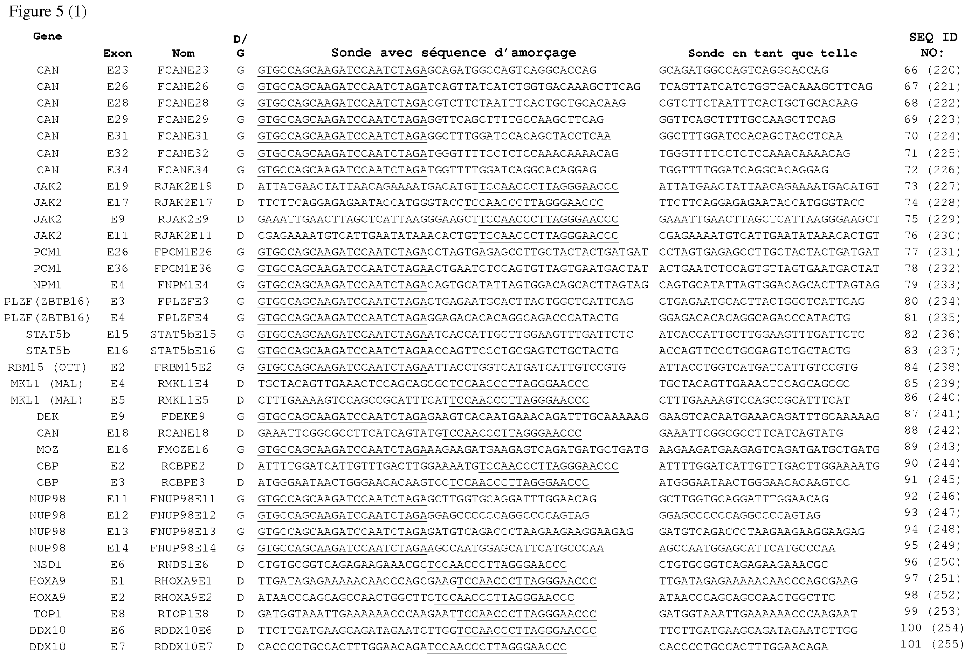

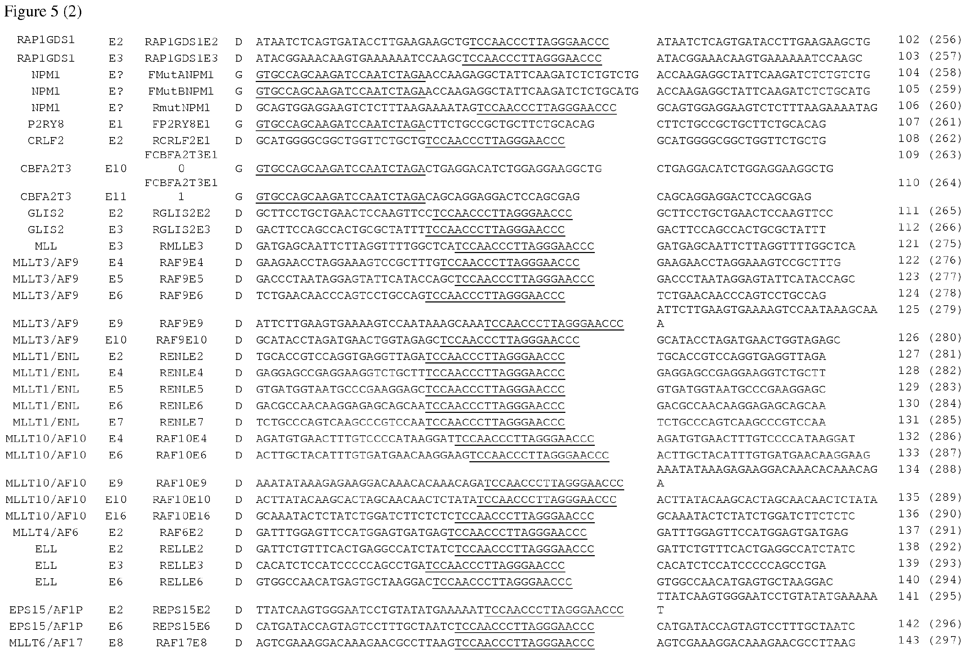

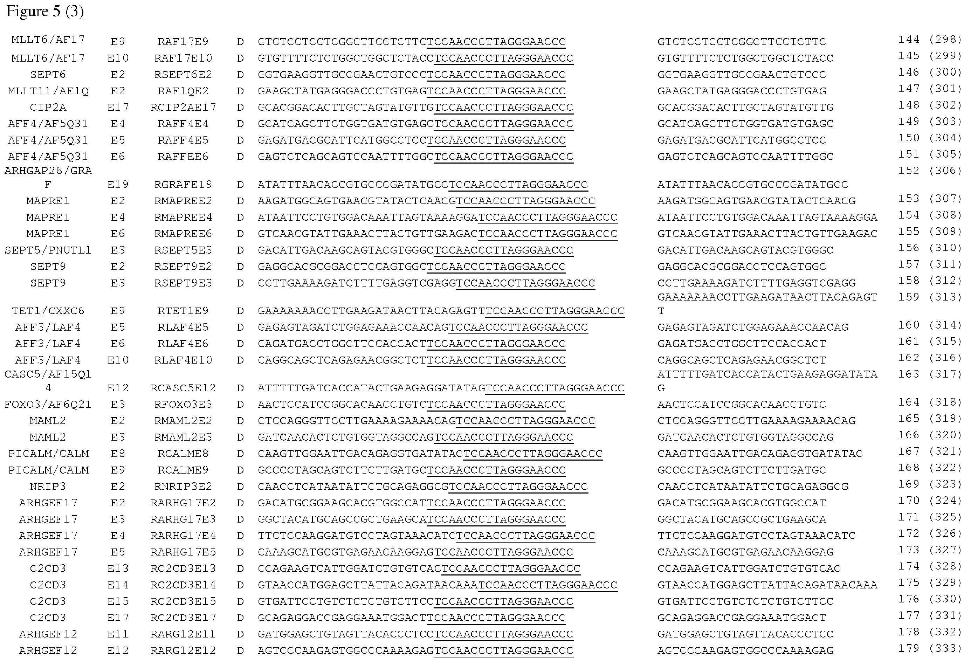

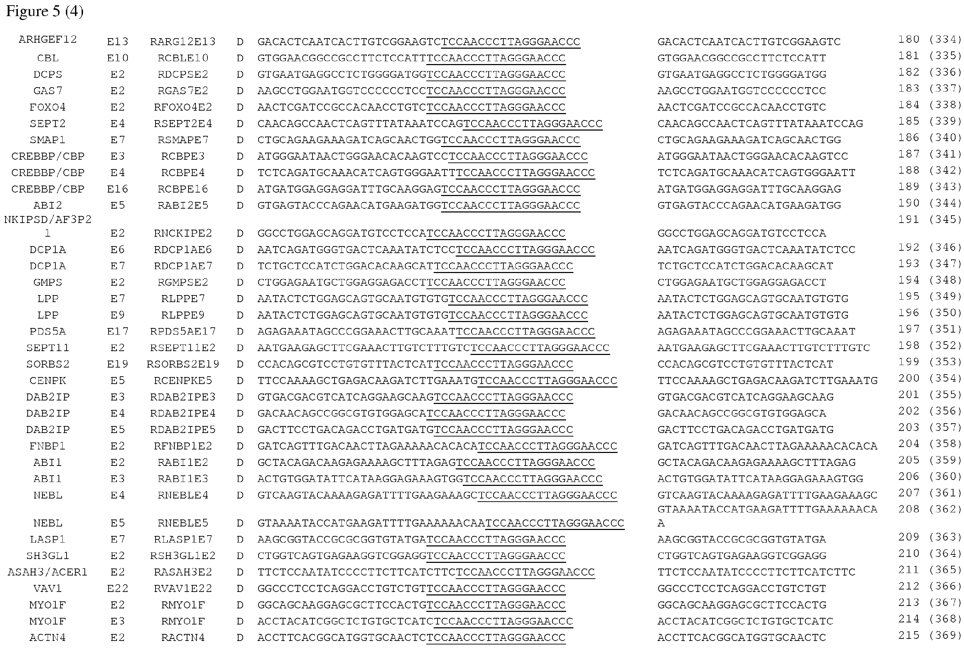

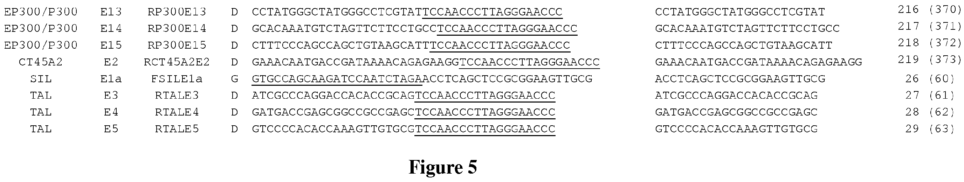

**Figure 5** **: Tableau des sondes SEQ ID NO : 26 à 29, 66 à 112 et 121 à 219 (correspondant aux sondes fusionnées à la séquence d'amorçage SEQ ID NO** : **60 à 63, 220 à 266 et 275 à 373).**
   Le tableau indique le gène et l'exon auxquels la sonde s'hybride. La colonne « SEQ ID NO : » indique le numéro de séquence de la sonde en tant que telle, et entre parenthèses le numéro de séquence de la sonde fusionnée à la séquence d'amorçage.
   Ces sondes sont utilisables pour le diagnostic des leucémies notamment.

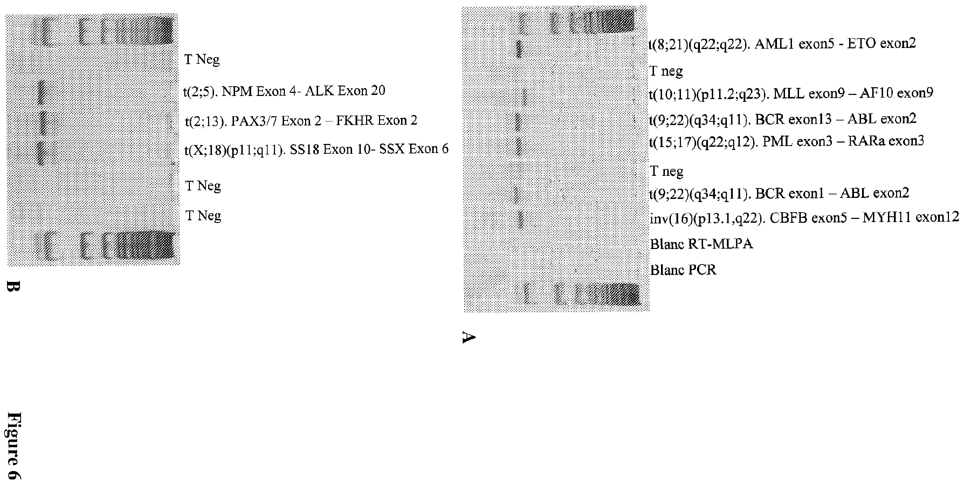

**Figure 6** **: Identification des translocations dans les cas de leucémies (A) et de tumeurs solides (B).**
   Les produits d'amplifications de PCR ont été analysés par pyroséquençage.
   A) Les translocations identifiées correspondent à :
      « AML1 exon5 - ETO exon2 » : LAM2 (Leucémie aiguë myléoblastique de type 2)
      « MLL exon9 - AF10 exon9 » : LAL (Leucémie aiguë lymphoïde)
      « BCR exonl3 - ABL exon2 » : leucémie myéloïde chronique
      « PML exon3 - RARa exon3 » : LAM3 (Leucémie aiguë myléoblastique de type 3)
      « BCR exon1 - ABL exon2 » : LAL (Leucémie aiguë lymphoïde) ; et
      « CBFB exon5 -MYH11 exon12 » : LAM4 (Leucémie aiguë myléoblastique de type 4).
   B) Les translocations identifiées correspondent à un synovialosarcome (SYT-SSX), à un Rhabdomyosarcome (PAX-FKHR), et à un lymphome anaplasique (NPM-ALK).
   Le terme « Tneg » signifie un échantillon contrôle, provenant d'une tumeur connue ne présentant pas les réarrangements génétiques testés.

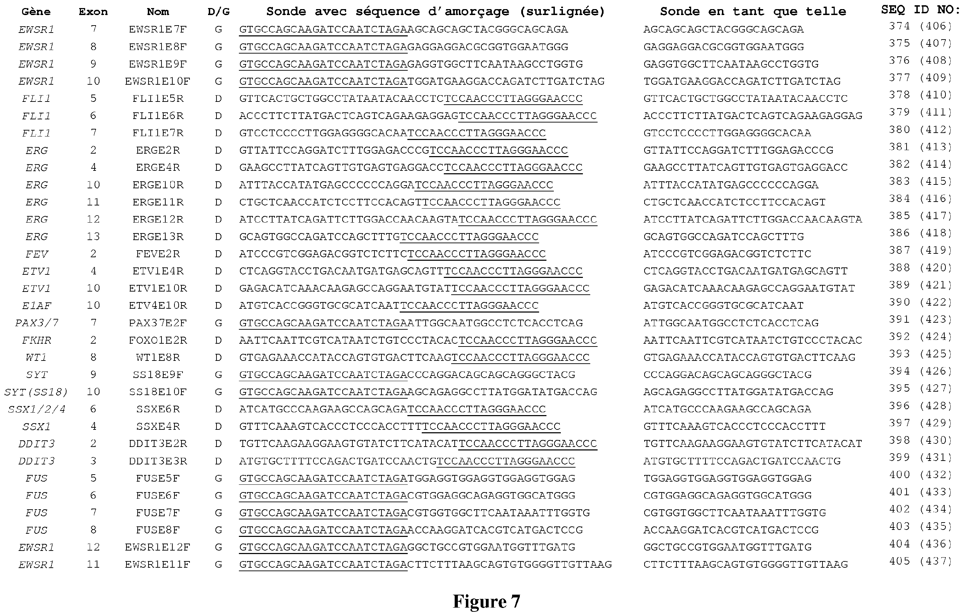

**Figure 7** **: Tableau des sondes SEQ ID NO** : **374 à 405 (correspondant aux sondes fusionnées à la séquence d'amorçage SEQ ID NO** : **406 à 437).**
   Le tableau indique le gène et l'exon auxquels la sonde s'hybride. La colonne « SEQ ID NO : » indique le numéro de séquence de la sonde en tant que telle, et entre parenthèses le numéro de séquence de la sonde fusionnée à la séquence d'amorçage.
   Ces sondes sont utilisables pour le diagnostic des sarcomes notamment.

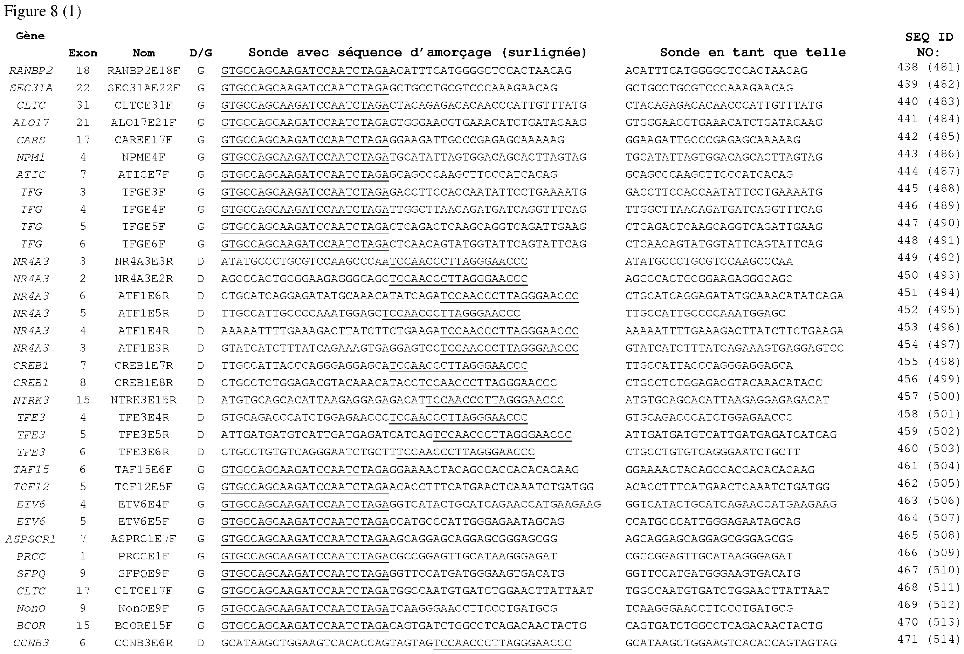

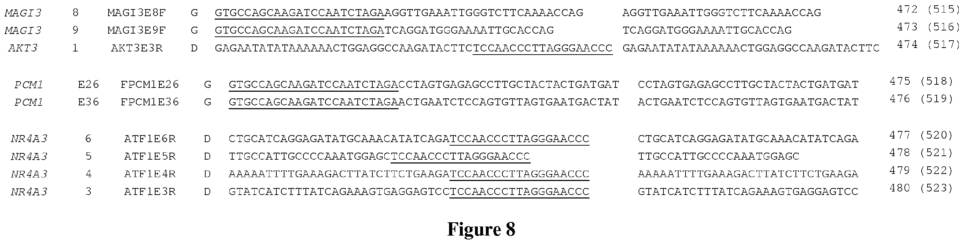

**Figure 8** **: Tableau des sondes SEQ ID NO** : **438 à 480 (correspondant aux sondes fusionnées à la séquence d'amorçage SEQ ID NO** : **481 à 523).**
   Le tableau indique le gène et l'exon auxquels la sonde s'hybride. La colonne « SEQ ID NO : » indique le numéro de séquence de la sonde en tant que telle, et entre parenthèses le numéro de séquence de la sonde fusionnée à la séquence d'amorçage.
   Ces sondes sont utilisables pour le diagnostic des sarcomes notamment.

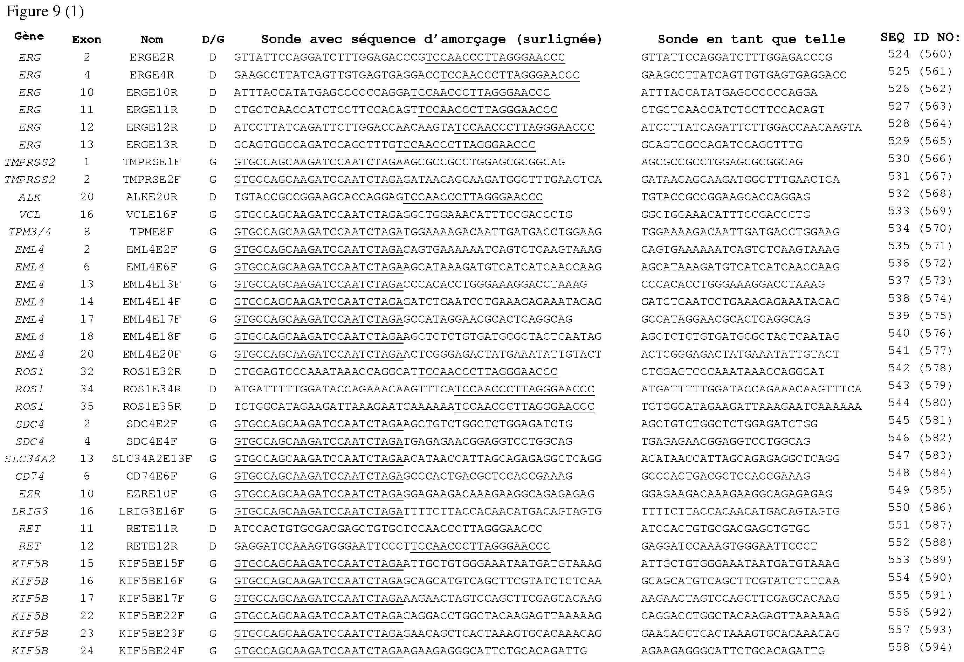

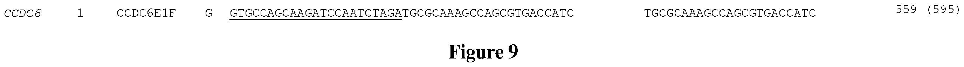

**Figure 9** **: Tableau des sondes SEQ ID NO** : **524 à 559 (correspondant aux sondes fusionnées à la séquence d'amorçage SEQ ID NO : 560 à 595).**
   Le tableau indique le gène et l'exon auxquels la sonde s'hybride. La colonne « SEQ ID NO : » indique le numéro de séquence de la sonde en tant que telle, et entre parenthèses le numéro de séquence de la sonde fusionnée à la séquence d'amorçage.
   Ces sondes sont utilisables pour le diagnostic des carcinomes notamment.
**Figure 10** : **Identification des gènes de fusion.**
   A) Gènes de fusion dans les cas de leucémies. Les transcrits de fusion s'hybrident avec les sondes SEQ ID NO : 1 à 25, 30, 31 et 113 à 120.
   B) Gènes de fusion dans les cas de sarcomes. Les transcrits de fusion s'hybrident avec les sondes SEQ ID NO : 374 à 405.
   C) Gènes de fusion dans les cas de carcinomes. Les transcrits de fusion s'hybrident avec les sondes SEQ ID NO : 524 à 559.

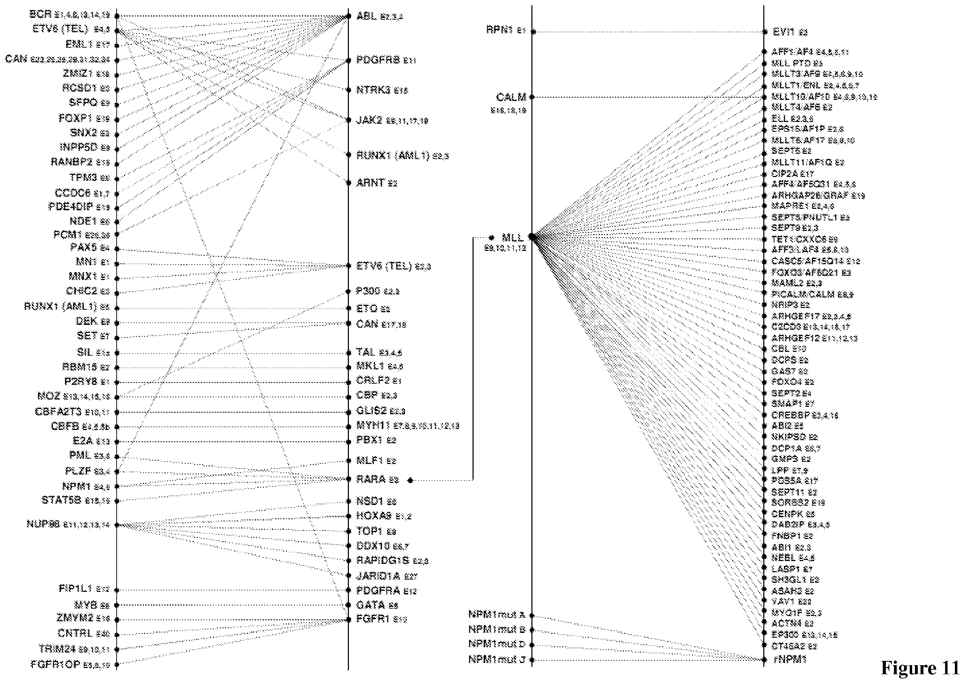

**Figure 11** **: Identification des gènes de fusion dans le cas de leucémies.**
   L'utilisation, dans un kit unique, des sondes SEQ ID NO : 1 à 31, 66 à 219, 438, 467, 534, 559, 616 à 639, 641 à 670, 737 et 759, permettrait de rechercher simultanément plus de 110 réarrangements géniques différents dans les leucémies.

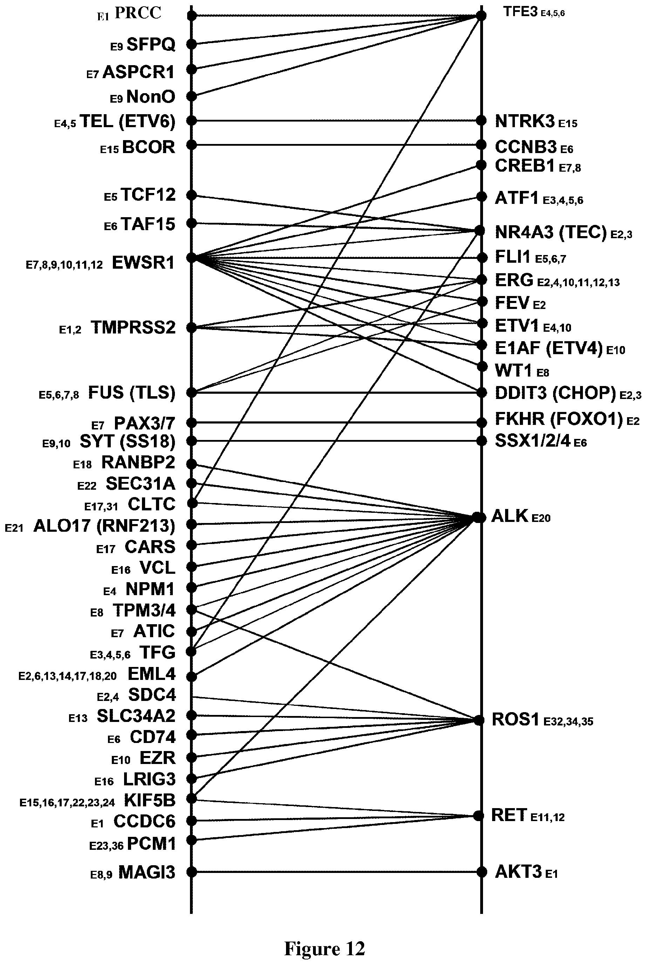

**Figure 12** : **Identification des gènes de fusion dans le cas de sarcomes et carcinomes.** Les transcrits de fusion s'hybrident avec les sondes SEQ ID NO : 374 à 405, 524 à 559 et 438 à 480.

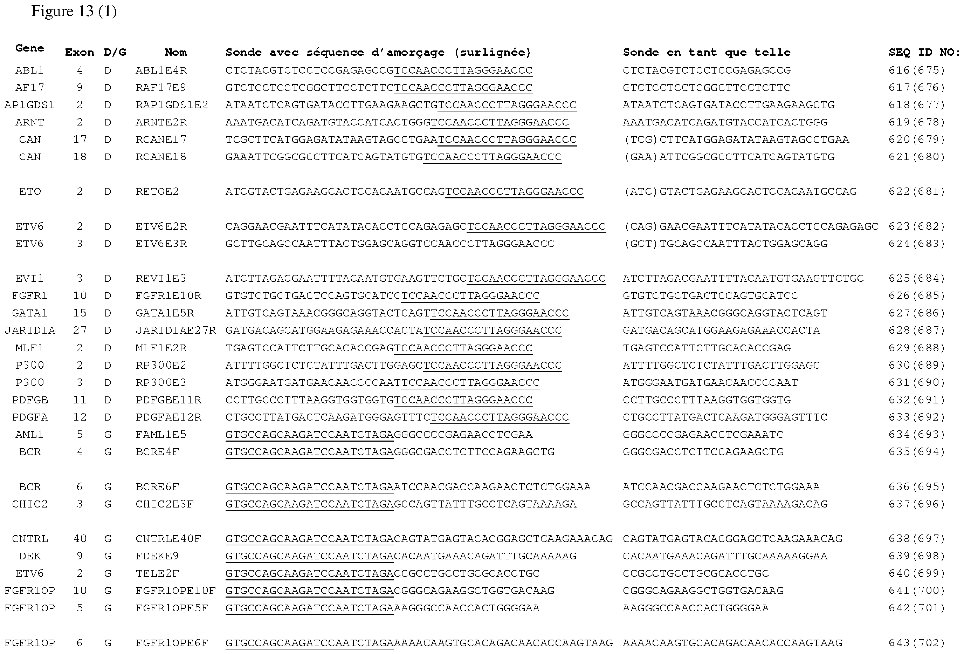

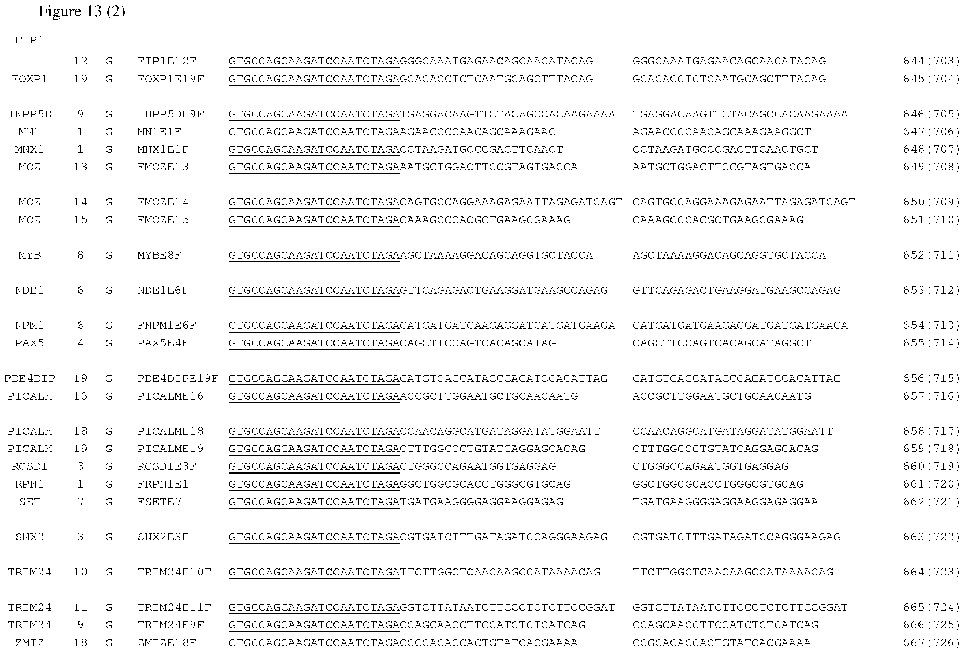

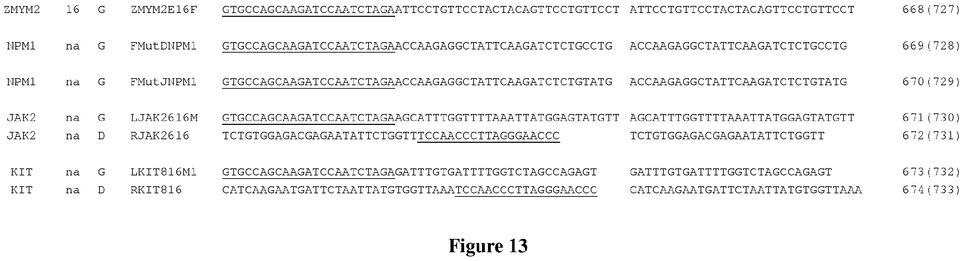

**Figure 13** : **Tableau des sondes SEQ ID NO** : **616-674 (correspondant aux sondes fusionnées à la séquence d'amorçage SEQ ID NO : 675-733).**
   Le tableau indique le gène et l'exon auxquels la sonde s'hybride. La colonne « SEQ ID NO : » indique le numéro de séquence de la sonde en tant que telle, et entre parenthèses le numéro de séquence de la sonde fusionnée à la séquence d'amorçage.
   Ces sondes sont utilisables pour le diagnostic des leucémies notamment.

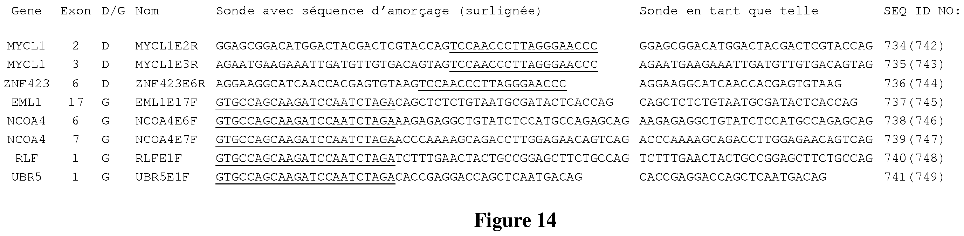

**Figure 14** : **Tableau des sondes SEQ ID NO** : **734-741 (correspondant aux sondes fusionnées à la séquence d'amorçage SEQ ID NO : 742-749).**
   Le tableau indique le gène et l'exon auxquels la sonde s'hybride. La colonne « SEQ ID NO : » indique le numéro de séquence de la sonde en tant que telle, et entre parenthèses le numéro de séquence de la sonde fusionnée à la séquence d'amorçage.
   Ces sondes sont utilisables pour le diagnostic des carcinomes notamment.

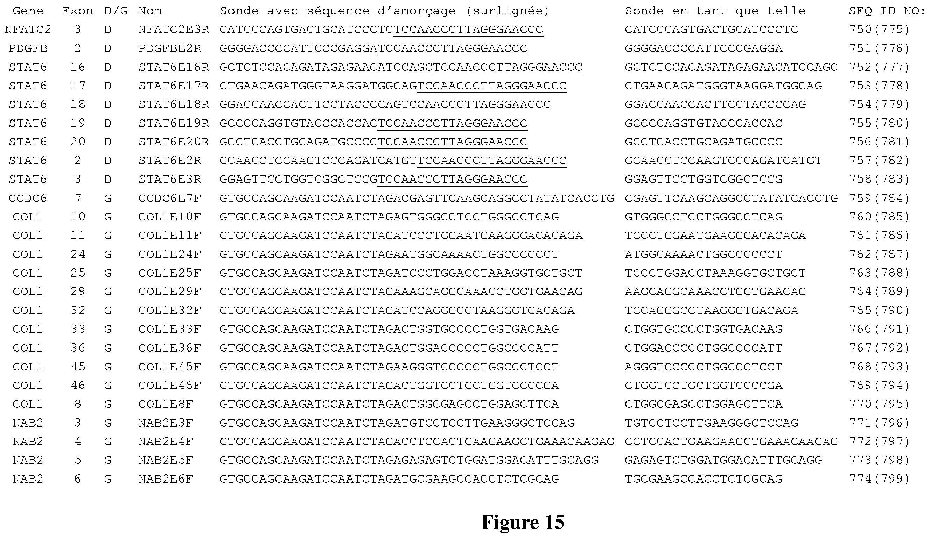

**Figure 15** : **Tableau des sondes SEQ ID NO** : **750-774 (correspondant aux sondes fusionnées à la séquence d'amorçage SEQ ID NO** : **775-799).**
   Le tableau indique le gène et l'exon auxquels la sonde s'hybride. La colonne « SEQ ID NO : » indique le numéro de séquence de la sonde en tant que telle, et entre parenthèses le numéro de séquence de la sonde fusionnée à la séquence d'amorçage.
   Ces sondes sont utilisables pour le diagnostic des carcinomes notamment.
F**igure 16 : Identification des gènes de fusion ciblés dans l'exemple 2.**
   Les transcrits de fusion s'hybrident avec les sondes SEQ ID NO : 1 à 31, 66 à 167, 169 à 182, 628, 657 à 659 et 662.

### EXEMPLE 1

### Matériel & Méthodes

### Etape a) : extraction d'ARN :

Les cellules obtenues à partir de sang ou de moelle osseuse ou de fragments de biopsie sont conservés à -80°C dans 1 mL de trizol (Life Technologies, Carlsbad, CA, USA).

200 µL de chloroforme (Merck, Darmstadt, Allemagne) sont ajoutés à chaque échantillon préalablement décongelé.

Le mélange est homogénéisé avant d'être incubé environ 5 min dans la glace puis centrifugé 15 min à 12000 rpm et à 4°C.

500 µL d'isopropanol 100% (Sigma Aldrich, St Quentin Fallavier, France) sont ajoutés à la phase aqueuse préalablement isolée.

Le mélange est homogénéisé avant d'être incubé environ 10 min dans la glace et centrifugé 10 min à 12000 rpm et à 4 °C.

Après décantation, les culots sont lavés par environ 1 mL d'éthanol à 70% et centrifugés 5 min à 7500 rpm et à 4°C.

Après 2 autres lavages les culots sont décantés et séchés avant d'être repris dans environ 100 µL d'eau.

Les ARN sont alors incubés au bain marie à environ 55°C pendant 5 à 10 min.

La concentration des ARN obtenus est ajustée entre 50 et 250 ng/µL

### Etape b) : transcription inverse:

4 µL ARN (200-1000ng) sont incubés avec 2,5 µL Tampon 5x (Thermo Fisher Scientific, Waltham, MA, USA), 1 µl DTT 100mM (Thermo Fisher Scientific, Waltham, MA, USA) 2 µl dNTPs 10mM (Thermo Fisher Scientific, Waltham, MA, USA) et 2 µl d'hexamères à 100 pmol/µl (Thermo Fisher Scientific, Waltham, MA, USA), pendant 2 minutes à 80°C, puis 5 minutes à 37°C, puis les échantillons sont conservés à 4°C.

La transcriptase inverse (RT) est alors ajoutée (1 µL RT (30u) (M-MLV, Thermo Fisher Scientific, Waltham, MA, USA)), et le mélange résultant est incubé 15 minutes à 37°C, puis 2 minutes à 98°C, puis conservé à 4°C.

### Etape c) : incubation et hybridation de l'ADNc avec les sondes (MLPA) :

5 µl du mélange précédent (∼ la moitié de la réaction initiale) obtenu à l'étape b) est incubé avec 1,5 µL MLPA Buffer (Old Salsa MLPA Buffer. MRC Holland, Amsterdam, Netherlands) et 1,5 µL du mélange de sondes décrit ci-après, pendant 2 Minutes à 95°C, puis au moins 1 heure à 60°C.

### Mélange de sondes pour MLPA (3 fmol/1,5µl final):

**Première étape :** Reprise de chaque sonde à 100 µM (H2O)
**Deuxième étape :** Dilution de chaque sonde à 10 µM (H2O)
**Troisième étape :** Mélange de sondes : 2 µl de chaque sonde à 10µM
Volume Final : + volume idem TE20:2 pour Mix en TE10 :1 final
**Quatrième étape :** Dilution finale : (0.2µl du mélange de sondes F et R de l'étape 3 x n sondes) + TE10 :1 qsp 1ml
(exemple : mélange de sondes F avec 10 sondes différentes : 0.2x10=2µl mélange F ; mélange R avec 10 sondes: 0.x10=4µl mélange R ; Mélange Final = 2µl mélange F + 4µl mélange R +994µl TE10 :1)

### Etape d) : Ligation :

La ligase 65 (n tubes+10%) (Salsa Ligase-65. MRC-Holland, Amsterdam, Netherlands) est préparée avec le mélange suivant :
3 µL Ligase Buffer A (Ligase Buffer A, MRC-Holland, Amsterdam, Netherlands), 3 µL Ligase Buffer B (Ligase Buffer B, MRC-Holland, Amsterdam, Netherlands), 25 µL H2O Vortex
1µL mix ligase 65 (Salsa Ligase-65. MRC-Holland, Amsterdam, Netherlands)
   Vortex.

Le mélange de sondes de l'étape c) est mélangé à 54 °C avec 32 µL de ce mix ligase, puis incubé 15 minutes à 54°C, et 5 minutes à 98°C, puis conservé à 4°C.

### Etape e) : PCR :

La PCR (n tubes + 10%) est effectuée dans le mélange suivant:
20 µl Mix Eurogentec Red'y'Start Mix (Eurogentec, Angers, France)
1 µl d'amorce SEQ ID NO:32 (Biot)
1 µl d'amorce SEQ ID NO:33
13 µl H2O

5 µl du mélange de l'étape d) sont mélangés à 35 µl du mélange PCR ci-dessus, puis soumis au programme de PCR suivant :

| | | |
|---|---|---|
| | 94°C | 6min |
| | | |
| | 94°C | 30" |
| 35x | 58°C | 30" |
| | 72°C | 30" |
| | | |
| | 72°C | 4min |
| | 4°C | |

### Etape f) : Pyroséquençage :

Contrôle (optionnel) : gel d'acrylamide 8% (Acrykrrride/Bis-Acrylamide 29:1, 40%, Biosolve B.V., Valkenswaard, Netherlands)
20µl du produit amplifié par PCR est analysé par pyroséquenceur.

### Résultats

Les résultats obtenus sont présentés en Figures 2, 3 et 6A pour les cas de leucémies, et en Figure 6B pour les tumeurs solides.

A l'issue de la PCR, si la translocation recherchée est détectée, alors l'échantillon est positif, et le diagnostic de la maladie est immédiat.

### EXEMPLE 2

Dans la leucémie aiguë, les translocations chromosomiques récurrentes qui conduisent à la fusion de deux gènes sont fréquentes. Certains de ces marqueurs ont un impact pronostique et thérapeutique bien établi et sont systématiquement contrôlés au moment du diagnostic par cytogénétique et RT-PCR. Pourtant, en raison des limites de ces méthodes, seulement quelques réarrangements connus parmi tous ceux existants sont systématiquement testés. De nombreuses anomalies qui pourraient fournir des informations cliniques importantes restent ainsi ignorées, principalement en raison de l'impossibilité d'exécuter un dépistage multi-cible rentable, rapide et fiable. Le test proposé ici est simple et permet une détection fiable des douzaines de gènes de fusion en seulement quelques heures.
L'essai a été conçu pour détecter simultanément plus de 50 translocations impliquant 70 gènes récurrents dans les leucémies aiguës myéloblastiques (LAM), les leucémies aiguës lymphoïdes (LAL) et les leucémies myéloïdes chroniques (LMC).

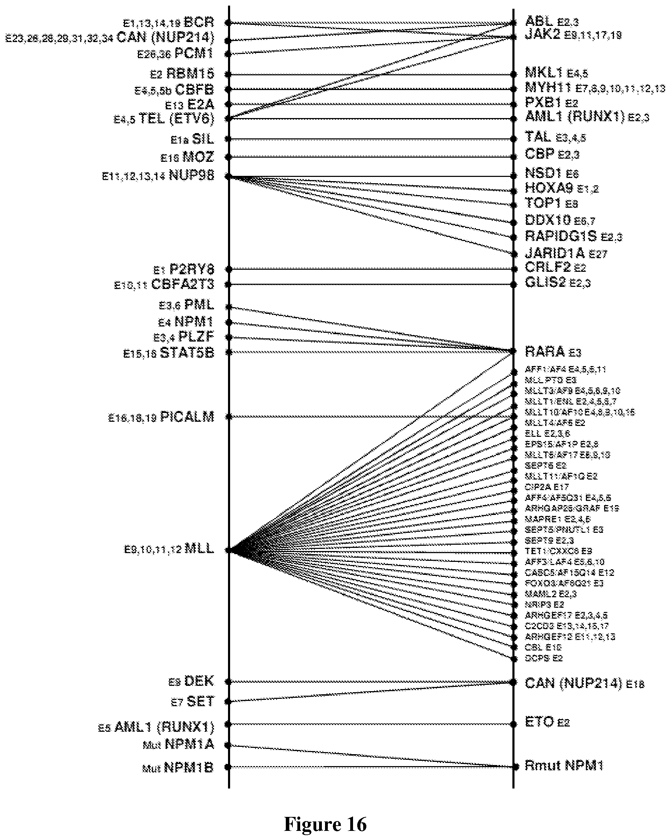

Les échantillons d'ADNc obtenus à partir de cellules leucémiques sont d'abord incubés avec un mélange de sondes oligonucléotidiques qui sont complémentaires des extrémités des exons, aux jonctions anormales sur les ARNm de fusion. Ces sondes correspondent aux transcrits de fusion listés en Figure 16, et ont pour séquence SEQ ID NO : 1 à 31, 66 à 167, 169 à 182, 628, 657 à 659 et 662. Pour la plupart des gènes, différentes sondes ont été conçues pour détecter différentes transcrits résultant de recombinaisons génomiques alternatives (par exemple sur les exons 1, 13, 14 et 19 pour le gène *BCR*, et sur les exons 2 et 3 pour le gène *ABL*). Le mélange regroupe ainsi plus de 150 sondes et vise plus de 400 transcrits de fusion différents. Toutes les sondes de gauche ont une queue commune (S_{A}) à leur extrémité 5', toutes les sondes de droite ont une queue commune (S_{B}) à leur extrémité 3' (cf Figures 2A à E). Des sondes supplémentaires ont également été incluses pour détecter les mutations les plus fréquentes du gène *NPM1* (A, B, D). Si une translocation est présente dans l'échantillon, les deux sondes s'hybrident côte à côte sur l'ADNc de fusion. Une ADN ligase est ensuite utilisée pour créer une liaison covalente entre ces sondes, ce qui permet leur amplification par PCR avec les amorces S_{A} et S_{B}. Si un produit de PCR est amplifié, les deux partenaires sont identifiés par analyse de séquence.

Cette méthode a été appliquée à une série rétrospective de 430 patients (252 LAM et 178 enfants LAL). Dans les LAL-B (147 cas), les 33 réarrangements *ETV6-RUNX1*, les 6 réarrangements *BCR-ABL* et les 5 réarrangements *TCF3-PBX1*, ainsi que les 6 réarrangements *MLL* (3 *AF4;* 1 *ENL*, 1 *AF9* et 1 *AFF4)* identifiés au moment du diagnostic par des méthodes conventionnelles, ont été détectés, ainsi que 5 jonctions *P2RY8-CRLF2* préalablement inconnues.
Dans les LAL-T (31 cas), 6 réarrangements connus (4 *SIL-TAL*, 2 *CALM-AF710*) et 5 fusions non détectées auparavant (2 *NUP214-ABL*, 1 *MLL-ENL*, 1 *ETV6-ABL*, et une nouvelle jonction *PLZF-ABL*) ont été détectés.
Dans les LAM, 86 fusions ont été détectées: 23 *PML-RARA*, *2 PLZF-RARA*, 18 *CBFB-MYH11*, 12 *RUNX1-RUNX1T1*, 4 *NUP98-NSD1*, 2 *BCR-ABL*, 1 *DEK-NUP214*, 1 *CALM-AF10*, 1 *MOZ-CBP*, 22 réarrangements *MLL* (13 *PTD*, 3 *AF9*, 2 *AF6*, 1 *AF10*, 1 *ENL*, 1 *AF1Q* et 1 *MAPRE*) et 44 mutations de *NPM1*. Surtout, 20 translocations de cette série, dont 14 fusions du gène *MLL* et une anomalie cytogénétique cryptique t(8, 21) n'avaient pas été identifiés lors du diagnostic.
En outre, toutes ces nouvelles anomalies (dans les LAM et LAL) ont pu être confirmées par RT-PCR classique et séquençage, démontrant la spécificité de la méthode.
Dans toute la cohorte de 430 patients, les trois méthodes ont détecté ainsi 157 fusions. 85 fusions (54,1%) et 112 fusions (71,3%) ont été détectées respectivement au moment du diagnostic par cytogénétique ou par RT-PCR, et 152 fusions (96,8%) ont été détectées par la présente méthode.

En conclusion, la méthode selon l'invention est un test multiplexe simple qui peut révéler un très grand nombre de fusions de gènes récurrents dans la leucémie. Son court temps de rotation (jusqu'à 40 patients peuvent être testés en parallèle et les résultats peuvent être obtenus en moins d'une journée) et son faible coût (seulement un appareil à PCR, un pyroséquenceur et des réactifs basiques de biologie moléculaire sont nécessaires) la rendent particulièrement adaptée à la pratique quotidienne. Sa capacité à détecter de nombreuses anomalies qui ne sont presque jamais testées dans la pratique quotidienne pourrait fournir beaucoup de diagnostics et de pronostics, et permettre la stratification des patients dans les essais cliniques prospectifs.

## Revendications

1. Méthode de diagnostic d'un cancer associé à la formation de gènes de fusion chez un sujet, choisi parmi une leucémie, un sarcome ou un carcinome, ladite méthode comprenant une étape de RT-MLPA sur un échantillon biologique obtenu à partir dudit sujet à l'aide au moins des couples de sondes choisis parmi :
- les sondes SEQ ID NO : 1 à 25, 30, 31 et 113 à 120 pour le diagnostic d'une leucémie, et/ou
- les sondes SEQ ID NO : 374 à 405 pour le diagnostic d'un sarcome, et/ou
- les sondes SEQ ID NO : 524 à 559 pour le diagnostic d'un carcinome,
chacune des sondes étant fusionnée, à au moins une extrémité, avec une séquence d'amorçage ; ledit couple de sondes étant constitué de deux sondes s'hybridant côte à côte pendant l'étape de RT-MLPA.

2. Méthode selon la revendication 1, **caractérisée en ce que** la séquence d'amorçage est choisie parmi les séquences SEQ ID NO : 33 et SEQ ID NO : 34.

3. Méthode selon l'une des revendications 1 à 2, **caractérisée en ce que** ledit échantillon biologique est choisi parmi le sang total, la moelle osseuse et une biopsie dudit sujet.

4. Méthode selon l'une quelconque des revendications précédentes, **caractérisée en ce que** les sondes SEQ ID NO : 26 à 29, 66 à 112 et 121 à 219 et/ou les sondes SEQ ID NO : 616 à 674 sont également utilisées pour l'étape de RT-MLPA, chacune des sondes étant fusionnée, à au moins une extrémité, avec une séquence d'amorçage.

5. Méthode selon l'une quelconque des revendications précédentes, **caractérisée en ce que** les sondes SEQ ID NO : 750 à 774 sont également utilisées pour l'étape de RT-MLPA, chacune des sondes étant fusionnée, à au moins une extrémité, avec une séquence d'amorçage.

6. Méthode selon l'une quelconque des revendications précédentes, **caractérisée en ce que** les sondes SEQ ID NO : 734 à 741 sont également utilisées pour l'étape de RT-MLPA, chacune des sondes étant fusionnée, à au moins une extrémité, avec une séquence d'amorçage.

7. Méthode selon l'une quelconque des revendications 1 à 6, **caractérisée en ce que** l'étape de RT-MLPA comprend au moins les étapes suivantes :
a) extraction de l'ARN de l'échantillon biologique du sujet ;
b) conversion de l'ARN extrait en a) en ADNc par transcription inverse ;
c) incubation de l'ADNc obtenu en b) avec les couples de sondes choisis parmi :
- les sondes SEQ ID NO : 1 à 25, 30, 31 et 113 à 120 pour le diagnostic d'une leucémie, et/ou,
- les sondes SEQ ID NO : 374 à 405 pour le diagnostic d'un sarcome, et/ou,
- les sondes SEQ ID NO : 524 à 559 pour le diagnostic d'un carcinome,
chacune des sondes étant fusionnée, à au moins une extrémité, avec une séquence d'amorçage ;
d) addition d'une ADN ligase dans le mélange obtenu en c), afin d'établir une liaison covalente entre deux sondes contiguës ;
e) amplification par PCR des sondes contiguës liées de manière covalente obtenues en d).

8. Méthode selon la revendication 7, **caractérisée en ce qu'**elle comprend une étape f) d'analyse des résultats de la PCR de l'étape e), de préférence par pyroséquençage.

9. Méthode selon la revendication 8, **caractérisée en ce que**, dans l'étape f), si, pour un échantillon biologique d'un sujet, une amplification par PCR est obtenue à l'étape e) suite à l'hybridation avec un couple de sondes, alors le sujet est porteur du cancer lié au réarrangement chromosomique correspondant au couple de sondes identifiées.

10. Méthode selon l'une quelconque des revendications 7 à 9, **caractérisée en ce que** l'amplification par PCR de l'étape e) se fait à l'aide des amorces SEQ ID NO : 32 et 33.

11. Méthode selon l'une quelconque des revendications 1 à 10, **caractérisée en ce qu'**elle est une méthode de diagnostic d'une leucémie chez un sujet, comprenant une étape de RT-MLPA sur un échantillon biologique obtenu à partir dudit sujet avec au moins les sondes SEQ ID NO : 1 à 25, 30, 31 et 113 à 120, chacune des sondes étant fusionnée, à au moins une extrémité, avec une séquence d'amorçage, lesdites sondes correspondant aux sondes SEQ ID NO : 35 à 59, 64, 65 et 267 à 274.

12. Méthode selon la revendication 11, **caractérisée en ce que** l'étape de RT-MLPA sur un échantillon biologique obtenu à partir du sujet est effectuée avec en outre au moins les sondes SEQ ID NO : 60 à 63, 220 à 266 et 275 à 373, et/ou les sondes SEQ ID NO : 675 à 733.

13. Méthode selon l'une quelconque des revendications 1 à 10, **caractérisée en ce qu'**elle est une méthode de diagnostic d'un sarcome chez un sujet, comprenant une étape de RT-MLPA sur un échantillon biologique obtenu à partir dudit sujet avec au moins les sondes SEQ ID NO : 374 à 405, chacune des sondes étant fusionnée, à au moins une extrémité, avec une séquence d'amorçage, lesdites sondes correspondant aux sondes SEQ ID NO : 406 à 437.

14. Méthode selon la revendication 13, **caractérisée en ce que** l'étape de RT-MLPA sur un échantillon biologique obtenu à partir du sujet est effectuée avec en outre au moins les sondes SEQ ID NO : 481 à 523 et/ou les sondes SEQ ID NO : 775 à 799.

15. Méthode selon l'une quelconque des revendications 1 à 10, **caractérisée en ce qu'**elle est une méthode de diagnostic d'un carcinome chez un sujet, comprenant une étape de RT-MLPA sur un échantillon biologique obtenu à partir dudit sujet avec au moins les sondes SEQ ID NO : 524 à 559, chacune des sondes étant fusionnée, à au moins une extrémité, avec une séquence d'amorçage, lesdites sondes correspondant aux sondes SEQ ID NO : 560 à 595.

16. Méthode selon la revendication 15, **caractérisée en ce que** l'étape de RT-MLPA sur un échantillon biologique obtenu à partir du sujet est effectuée avec en outre au moins les sondes SEQ ID NO : 481 à 523 et/ou les sondes SEQ ID NO : 742 à 749.

17. Kit comprenant au moins les sondes SEQ ID NO : 1 à 25, 30, 31 et 113 à 120, et/ou les sondes SEQ ID NO : 374 à 405, et/ou les sondes SEQ ID NO : 524 à 559, de préférence comprenant en outre les sondes SEQ ID NO : 26 à 29, 66 à 112 et 121 à 219, et/ou les sondes SEQ ID NO :616 à 674, et/ou les sondes SEQ ID NO : 438 à 480, et/ou les sondes SEQ ID NO : 750 à 774, et/ou les sondes SEQ ID NO : 734 à 741, chacune des sondes étant fusionnée, à au moins une extrémité, avec une séquence d'amorçage.

18. Kit comprenant au moins les sondes SEQ ID NO : 35 à 59, 64, 65 et 267 à 274, et/ou les sondes SEQ ID NO : 406 à 437, et/ou les sondes SEQ ID NO : 560 à 595, de préférence comprenant en outre les sondes SEQ ID NO : 60 à 63, 220 à 266 et 275 à 373, et/ou les sondes SEQ ID NO : 675 à 733, et/ou les sondes SEQ ID NO : 775 à 799, et/ou les sondes SEQ ID NO : 742 à 749.

## Patentansprüche

1. Verfahren zur Diagnose einer Krebserkrankung, die mit der Bildung von fusionierten Genen bei einem Patienten assoziiert ist, gewählt aus Leukämie, Sarkom oder Karzinom, wobei das Verfahren einen RT-MLPA-Schritt an einer von diesem Patienten mittels wenigstens eines Paars von Sonden gewonnenen biologischen Probe umfasst, wobei die Sonden gewählt sind aus:
- den Sonden SEQ ID NO: 1 bis 25, 30, 31 und 113 bis 120 zur Diagnose einer Leukämie, und/oder
- den Sonden SEQ ID NO: 374 bis 405 zur Diagnose eines Sarkoms, und/oder
- den Sonden SEQ ID NO: 524 bis 559 zur Diagnose eines Karzinoms,
wobei jede der Sonden an wenigstens einem Ende mit einer Initiierungssequenz fusioniert ist; wobei das Sondenpaar zwei Sonden umfasst, die während des RT-MLPA-Schritts nebeneinander hybridisieren.

2. Verfahren nach Anspruch 1, **dadurch gekennzeichnet, dass** die Initiierungssequenz gewählt ist aus den Sequenzen SEQ ID NO: 33 und SEQ ID NO: 34.

3. Verfahren nach einem der Ansprüche 1 bis 2, **dadurch gekennzeichnet, dass** die biologische Probe gewählt ist aus Vollblut, Knochenmark und einer Biopsie des Patienten.

4. Verfahren nach einem der vorhergehenden Ansprüche, **dadurch gekennzeichnet, dass** die Sonden SEQ ID NO: 26 bis 29, 66 bis 112 und 121 bis 219 und/oder die Sonden SEQ ID NO: 616 bis 674 auch für den RT-MLPA-Schritt verwendet werden, wobei jede der Sonden an wenigstens einem Ende mit einer Initiierungssequenz fusioniert ist.

5. Verfahren nach einem der vorhergehenden Ansprüche, **dadurch gekennzeichnet, dass** die Sonden SEQ ID NO: 750 bis 774 auch für den RT-MLPA-Schritt verwendet werden, wobei jede der Sonden an wenigstens einem Ende mit einer Initiierungssequenz fusioniert ist.

6. Verfahren nach einem der vorhergehenden Ansprüche, **dadurch gekennzeichnet, dass** die Sonden SEQ ID NO: 734 bis 741 auch für den RT-MLPA-Schritt verwendet werden, wobei jede der Sonden an wenigstens einem Ende mit einer Initiierungssequenz fusioniert ist.

7. Verfahren nach einem der Ansprüche 1 bis 6, **dadurch gekennzeichnet, dass** der RT-MLPA-Schritt wenigstens folgende Schritte umfasst:
a) Extraktion der RNS aus der biologischen Probe des Patienten;
b) Umwandlung der extrahierten RNS aus a) in cDNA durch reverse Transkription;
c) Inkubieren der in b) erhaltenen cDNA mit den Sondenpaaren, gewählt aus
- den Sonden SEQ ID NO: 1 bis 25, 30, 31 und 113 bis 120 zur Diagnose einer Leukämie, und/oder
- den Sonden SEQ ID NO: 374 bis 405 zur Diagnose eines Sarkoms, und/oder
- den Sonden SEQ ID NO: 524 bis 559 zur Diagnose eines Karzinoms,
wobei jede der Sonden an wenigstens einem Ende mit einer Initiierungssequenz fusioniert ist;
d) Hinzufügen einer DNA-Ligase zu der in c) erhaltenen Mischung zur Herstellung einer kovalenten Bindung zwischen zwei benachbarten Sonden;
e) Verstärkung der in d) erhaltenen kovalent gebundenen benachbarten Sonden durch PCR.

8. Verfahren nach Anspruch 7, **dadurch gekennzeichnet, dass** es einen Schritt f) der Analyse der Ergebnisse des PCR in Schritt e), bevorzugt durch Pyrosequenzierung, umfasst.

9. Verfahren nach Anspruch 8, **dadurch gekennzeichnet, dass**, wenn in Schritt e) für eine biologische Probe eines Patienten eine Amplifikation durch PCR nach der Hybridisierung mit einem Sonden-Paar erhalten wird, in Schritt f) der Patient Träger der Krebserkrankung ist, die mit der Chromosomenneuanordnung verbunden ist, welche dem Paar identifizierter Sonden entspricht.

10. Verfahren nach einem der Ansprüche 7 bis 9, **dadurch gekennzeichnet, dass** die Amplifikation durch PCR in Schritt e) mittels der Primer SEQ ID NO: 32 und 33 erfolgt.

11. Verfahren nach einem der Ansprüche 1 bis 10, **dadurch gekennzeichnet, dass** es ein Verfahren zur Diagnose einer Leukämie bei einem Patienten ist, welches einen RT-MLPA-Schritt an einer von diesem Patienten gewonnenen biologischen Probe umfasst, wenigstens mit den Sonden SEQ ID NO: 1 bis 25, 30, 31 und 113 bis 120, wobei jede der Sonden an wenigstens einem Ende mit einer Initiierungssequenz fusioniert ist, wobei die Sonden den Sonden SEQ ID NO: 35 bis 59, 64, 65 und 267 bis 274 entsprechen.

12. Verfahren nach Anspruch 11, **dadurch gekennzeichnet, dass** der RT-MLPA-Schritt an einer von dem Patienten gewonnenen biologischen Probe ferner wenigstens mit den Sonden SEQ ID NO: 60 bis 63, 220 bis 266 und 275 bis 373 und/oder mit den Sonden SEQ ID NO: 675 bis 733 durchgeführt wird.

13. Verfahren nach einem der Ansprüche 1 bis 10, **dadurch gekennzeichnet, dass** es ein Verfahren zur Diagnose eines Sarkoms bei einem Patienten ist, welches einen RT-MLPA-Schritt an einer von diesem Patienten gewonnenen biologischen Probe umfasst, wenigstens mit den Sonden SEQ ID NO: 374 bis 405, wobei jede der Sonden an wenigstens einem Ende mit einer Initiierungssequenz fusioniert ist, wobei die Sonden den Sonden SEQ ID NO: 406 bis 437 entsprechen.

14. Verfahren nach Anspruch 13, **dadurch gekennzeichnet, dass** der RT-MLPA-Schritt an einer von dem Patienten gewonnenen biologischen Probe ferner wenigstens mit den Sonden SEQ ID NO: 481 bis 523 und/oder mit den Sonden SEQ ID NO: 775 bis 799 durchgeführt wird.

15. Verfahren nach einem der Ansprüche 1 bis 10, **dadurch gekennzeichnet, dass** es ein Verfahren zur Diagnose eines Karzinoms bei einem Patienten ist, welches einen RT-MLPA-Schritt an einer von diesem Patienten gewonnenen biologischen Probe umfasst, wenigstens mit den Sonden SEQ ID NO: 524 bis 559, wobei jede der Sonden an wenigstens einem Ende mit einer Initiierungssequenz fusioniert ist, wobei die Sonden den Sonden SEQ ID NO: 560 bis 595 entsprechen.

16. Verfahren nach Anspruch 15, **dadurch gekennzeichnet, dass** der RT-MLPA-Schritt an einer von dem Patienten gewonnenen biologischen Probe ferner wenigstens mit den Sonden SEQ ID NO: 481 bis 523 und/oder mit den Sonden SEQ ID NO: 742 bis 749 durchgeführt wird.

17. Kit, welches wenigstens die Sonden SEQ ID NO: 1 bis 25, 30, 31 und 113 bis 120 und/oder die Sonden SEQ ID NO: 374 bis 405 und/oder die Sonden SEQ ID NO: 524 bis 559, bevorzugt ferner umfassend die Sonden SEQ ID NO: 26 bis 29, 66 bis 112 und 121 bis 219 und/oder die Sonden SEQ ID NO: 616 bis 674 und/oder die Sonden SEQ ID NO: 438 bis 480 und/oder die Sonden SEQ ID NO: 750 bis 774 und/oder die Sonden SEQ ID NO: 734 bis 741 umfasst, wobei jede der Sonden an wenigstens einem Ende mit einer Initiierungssequenz fusioniert ist.

18. Kit, welches wenigstens die Sonden SEQ ID NO: 35 bis 59, 64, 65 und 267 bis 274 und/oder die Sonden SEQ ID NO: 406 bis 437 und/oder die Sonden SEQ ID NO: 560 bis 595 umfasst und welches bevorzugt ferner die Sonden SEQ ID NO: 60 bis 63, 220 bis 266 und 275 bis 373 umfasst und/oder die Sonden SEQ ID NO: 675 bis 733 und/oder die Sonden SEQ ID NO: 775 bis 799 und/oder die Sonden SEQ ID NO: 742 bis 749.

## Claims

1. Method for diagnosing cancer associated with the formation of fusion genes in a subject, chosen from leukaemia, sarcoma or carcinoma, said method comprising an RT-MLPA step on a biological sample obtained from said subject using at least probe pairs chosen from:
- the probes SEQ ID NO: 1 to 25, 30, 31 and 113 to 120 for diagnosing leukaemia, and/or
- the probes SEQ ID NO: 374 to 405 for diagnosing sarcoma, and/or
- the probes SEQ ID NO: 524 to 559 for diagnosing carcinoma,
each of the probes being fused, at at least one end, with a priming sequence; said probe pair consisting of two probes hybridising side by side during the RT-MLPA step.

2. Method according to claim 1, **characterised in that** the priming sequence is chosen from the sequences SEQ ID NO: 33 and SEQ ID NO: 34.

3. Method according to one of claims 1 to 2, **characterised in that** said biological sample is chosen from whole blood, bone marrow and a biopsy from said subject.

4. Method according to any one of the preceding claims, **characterised in that** the probes SEQ ID NO: 26 to 29, 66 to 112 and 121 to 219 and/or the probes SEQ ID NO: 616 to 674 are also used for the RT-MLPA step, each of the probes being fused, at at least one end, with a priming sequence.

5. Method according to any one of the preceding claims, **characterised in that** the probes SEQ ID NO: 750 to 774 are also used for the RT-MLPA step, each of the probes being fused, at at least one end, with a priming sequence.

6. Method according to any one of the preceding claims, **characterised in that** the probes SEQ ID NO: 734 to 741 are also used for the RT-MLPA step, each of the probes being fused, at at least one end, with a priming sequence.

7. Method according to any one of claims 1 to 6, **characterised in that** the RT-MLPA step comprises at least the following steps:
a) extracting RNA from the subject's biological sample;
b) converting the RNA extracted in a) to cDNA by reverse transcription;
c) incubating the cDNA obtained in b) with probe pairs chosen from:
- the probes SEQ ID NO: 1 to 25, 30, 31 and 113 to 120 for diagnosing leukaemia, and/or
- the probes SEQ ID NO: 374 to 405 for diagnosing sarcoma, and/or,
- the probes SEQ ID NO: 524 to 559 for diagnosing carcinoma,
each of the probes being fused, at at least one end, with a priming sequence;
d) adding a DNA ligase into the mixture obtained in c), in order to establish a covalent bond between two contiguous probes;
e) PCR amplification of the covalently bonded contiguous probes obtained in d).

8. Method according to claim 7, **characterised in that** it comprises a step f) of analysing the PCR results from step e), preferably by pyrosequencing.

9. Method according to claim 8, **characterised in that**, in step f), if, for a subject's biological sample, a PCR amplification is obtained in step e) following hybridisation with a probe pair, then the subject is a carrier of cancer associated with the chromosomal rearrangement corresponding to the identified probe pair.

10. Method according to any one of claims 7 to 9, **characterised in that** the PCR amplification of step e) is carried out using the primers SEQ ID No: 32 and 33.

11. Method according to any one of claims 1 to 10, **characterised in that** it is a method for diagnosing leukaemia in a subject, comprising an RT-MLPA step on a biological sample obtained from said subject with at least the probes SEQ ID NO: 1 to 25, 30, 31 and 113 to 120, each of the probes being fused, at at least one end, with a priming sequence, said probes corresponding to the probes SEQ ID NO: 35 to 59, 64, 65 and 267 to 274.

12. Method according to claim 11, **characterised in that** the RT-MLPA step on a biological sample obtained from the subject is carried out with further at least the probes SEQ ID NO: 60 to 63, 220 to 266 and 275 to 373, and/or the probes SEQ ID NO: 675 to 733.

13. Method according to any of claims 1 to 10, **characterised in that** it is a method for diagnosing sarcoma in a subject, comprising an RT-MLPA step on a biological sample obtained from said subject with at least the probes SEQ ID NO: 374 to 405, each of the probes being fused, at at least one end, with a priming sequence, said probes corresponding to the probes SEQ ID NO: 406 to 437.

14. Method according to claim 13, **characterised in that** the RT-MLPA step on a biological sample obtained from the subject is carried out with further at least the probes SEQ ID NO: 481 to 523, and/or the probes SEQ ID NO: 775 to 799.

15. Method according to any of claims 1 to 10, **characterised in that** it is a method for diagnosing carcinoma in a subject, comprising an RT-MLPA step on a biological sample obtained from said subject with at least the probes SEQ ID NO: 524 to 559, each of the probes being fused, at at least one end, with a priming sequence, said probes corresponding to the probes SEQ ID NO: 560 to 595.

16. Method according to claim 15, **characterised in that** the RT-MLPA step on a biological sample obtained from the subject is carried out with further at least the probes SEQ ID NO: 481 to 523, and/or the probes SEQ ID NO: 742 to 749.

17. Kit comprising at least the probes SEQ ID NO: 1 to 25, 30, 31 and 113 to 120, and/or the probes SEQ ID NO: 374 to 405, and/or the probes SEQ ID NO: 524 to 559, preferably further comprising the probes SEQ ID NO: 26 to 29, 66 to 112 and 121 to 219, and/or the probes SEQ ID NO: 616 to 674, and/or the probes SEQ ID NO: 438 to 480, and/or the probes SEQ ID NO: 750 to 774, and/or the probes SEQ ID NO: 734 to 741, each of the probes being fused, at at least one end, with a priming sequence.

18. Kit comprising at least the probes SEQ ID NO: 35 to 59, 64, 65 and 267 to 274, and/or the probes SEQ ID NO: 406 to 437, and/or the probes SEQ ID NO: 560 to 595, preferably further comprising the probes SEQ ID NO: 60 to 63, 220 to 266 and 275 to 373, and/or the probes SEQ ID NO: 675 to 733, and/or the probes SEQ ID NO: 775 to 799, and/or the probes SEQ ID NO: 742 to 749.
